(19) Europäisches Patentamt
European Patent Office
Office européen des brevets

(11) EP 3 426 634 B1

(12) **EUROPEAN PATENT SPECIFICATION**

(45) Date of publication and mention of the grant of the patent:
**22.01.2020 Bulletin 2020/04**

(21) Application number: **17771757.6**

(22) Date of filing: **22.09.2017**

(51) Int Cl.:
*C07D 211/14* (2006.01) *C07D 211/18* (2006.01)
*C07D 211/22* (2006.01) *C07D 211/34* (2006.01)
*A61K 31/445* (2006.01) *A61P 25/16* (2006.01)
*A61P 25/28* (2006.01)

(86) International application number:
**PCT/EP2017/074067**

(87) International publication number:
**WO 2018/055096 (29.03.2018 Gazette 2018/13)**

(54) **DISUBTITUTED AZETIDINES, PYRROLIDINES, PIPERIDINES AND AZEPANES AS INHIBITORS OF MONOAMINE OXIDASE B FOR THE TREATMENT OF NEURODEGENERATIVE DISEASES**

DISUBSTITUIERTE AZETIDINE, PYRROLIDINE, PIPERIDINE UND AZEPANE ALS INHIBITOREN DER MONOAMINOXIDASE B ZUR BEHANDLUNG VON NEURODEGENERATIVEN ERKRANKUNGEN

AZÉTIDINES, PYRROLIDINES, PIPÉRIDINES ET AZÉPANES DI-SUBSTITUÉS EN TANT QU'INHIBITEURS DE MONOAMINE OXYDASE B POUR LE TRAITEMENT DE MALADIES NEURODÉGÉNÉRATIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.09.2016 SI 201600237**

(43) Date of publication of application:
**16.01.2019 Bulletin 2019/03**

(73) Proprietor: **University of Ljubljana
1000 Ljubljana (SI)**

(72) Inventors:
- **KNEZ, Damijan
2367 Vuzenica (SI)**
- **SOVA, Matej
4207 Cerklje na Gorenjskem (SI)**
- **GOBEC, Stanislav
1000 Ljubljana (SI)**

(74) Representative: **Zacco GmbH
Bayerstrasse 83
80335 München (DE)**

(56) References cited:
**EP-A1- 0 435 387 US-A1- 2003 105 133**

- **WRIGHT J L ET AL: "Subtype-selective N-methyl-D-aspartate receptor antagonists: synthesis and biological evaluation of 1-(heteroarylalkynyl)-4-benzylpiperidines" , JOURNAL OF MEDICINAL CHEMISTRY, AMERICAN CHEMICAL SOCIETY, vol. 43, no. 18, 7 September 2000 (2000-09-07), pages 3408-3419, XP002731396, ISSN: 0022-2623, DOI: 10.1021/JM000023O [retrieved on 2000-08-17]**
- **WRIGHT J L ET AL: "Subtype-selective N-Methyl-D-aspartate receptor Antagonists: Synthesis and Biological Evaluation of 1-(Arylalkynyl)-4-benzylpiperidines", JOURNAL OF MEDICINAL CHEMISTRY, AMERICAN CHEMICAL SOCIETY, vol. 42, 1 January 1999 (1999-01-01), pages 2469-2477, XP002205398, ISSN: 0022-2623, DOI: 10.1021/JM990148X**
- **WRIGHT J L ET AL: "Discovery of subtype-selective NMDA receptor ligands: 4-benzyl-1-piperidinylalkynylpyrroles, pyrazoles and imidazoles as NR1A/2B antagonists", BIOORGANIC & MEDICINAL CHEMISTRY LET, PERGAMON, AMSTERDAM, NL, vol. 9, no. 19, 4 October 1999 (1999-10-04), pages 2815-2818, XP004179169, ISSN: 0960-894X, DOI: 10.1016/S0960-894X(99)00482-5**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- GUZIKOWSKI A P ET AL: "Synthesis of N-Substituted 4-(4-Hydroxyphenyl)piperidines, 4-(4-Hydroxybenzyl)piperidines, and (.+-.)-3-(4-Hydroxyphenyl)pyrrolidines: Selective Antagonists at the 1A/2B NMDA Receptor Subtype", JOURNAL OF MEDICINAL CHEMISTRY, AMERICAN CHEMICAL SOCIETY, vol. 43, no. 5, 15 February 2000 (2000-02-15), pages 984-994, XP002373148, ISSN: 0022-2623, DOI: 10.1021/JM990428C
- L. PISANI ET AL: "Targeting Monoamine Oxidases with Multipotent Ligands: An Emerging Strategy in the Search of New Drugs Against Neurodegenerative Diseases", CURRENT MEDICINAL CHEMISTRY, vol. 18, no. 30, 1 October 2011 (2011-10-01), pages 4568-4587, XP055116034, ISSN: 0929-8673, DOI: 10.2174/092986711797379302

## Description

### Technical Field

The present invention belongs to the field of pharmaceutical industry and relates to novel inhibitors of monoamine oxidase B (MAO-B) for the treatment of acute and chronic diseases such as Parkinson's disease, Alzheimer's disease, Huntington's disease, amyotrophic lateral sclerosis, to the preparation of these compounds and pharmaceutical products that contain these compounds. The characteristic of these compounds is that they bind into the active site through specific noncovalent (polar and hydrophobic interactions) and/or covalent interactions.

### Technical Problem

There is a need for novel drugs for the treatment of neurodegenerative diseases due to the lack of efficacy and safety reasons of the current therapy. One of the validated and promising targets for the development of new drugs for the aforementioned therapeutic indications represents the enzyme monoamine oxidase B (MAO-B). The field of present invention is the discovery of new drugs with azetidine, pyrrolidine, piperidine and azepane scaffold that are active as selective reversible or irreversible inhibitors of MAO-B.

### Background Art

Amine oxidases are the enzymes, which catalyze the oxidative deamination of numerous biologically important amines to aldehyde, hydrogen peroxide and ammonia (Mondovi B and Finazzi A, 1985). According to the nature of the prosthetic group, which takes part in the catalytic cycle, we subdivide them into flavine-dependent and copper-dependent amine oxidases. To the flavine-dependent amine oxidases, which possess covalenty bound flavin adenine dinucleotide (FAD), belong monoamine oxidases (MAO, EC 1.4.3.4) that are located in the outer mitochondrial membrane (Mondovi B and Finazzi A, 1985).

MAO belong to the family of oxidoreductases and have an important role in regulation and metabolism of neurotransmitters that contain amine group (serotonin, noradrenaline, dopamine, histamine) in peripheral tissues and the central nervous system. Two isoforms of MAO are present in humans (MAO-A and MAO-B), which differs in amino acid sequences, three-dimensional structures, distributions in tissue and organs, inhibitor sensitivities and substrate specificity (Cai Z, Mol Med Rep, 2014, 9, 1533-1541). Both isozymes are found in and outside of the central nervous system (CNS). MAO-A preferentially catalyzes the oxidative deamination of serotonin, adrenaline, and noradrenaline, whereas MAO-B deaminates preferentially β-phenethylamine and benzylamine (Carradori S, J Med Chem, 2015, 58, 6717-6732). The unique location of MAO in CNS and peripheral tissues enables modulation of the functions of different neurotransmitters in association with various conditions, including mood disorders, anxiety and depression, schizophrenia, attention deficit hyperactivity disorder, migraine, sexual maturation and neurodegenerative diseases (Cai Z, Mol Med Rep, 2014, 9, 1533-1541).

MAO-B comprises about 80% of the total MAO activity in the human brain and is the predominant isozyme in the striatum (Riederer P, NeuroToxicology, 2004, 25, 271-277). In contrast to MAO-A, which is in CNS the most abundant in catecholaminergic neurons, MAO-B is predominantly found in serotonergic and histaminergic neurons and glial cells (Shih JC, Annu Rev Neurosci 1999, 22, 197-217), and in trombocytes in peripheral tissues. The activity of MAO-B in human body increases with aging, which might be related to the proliferation of glia cells (Shih JC, Annu Rev Neurosci 1999, 22, 197-217). The active site consists of two separate cavities, an entrance and a substrate cavity, that are separated by the side chain of Ile-199; however, in the presence of larger ligands, the Ile-199 adopts an alternate conformation, which enables the fusion of both cavities (Singh R, Chem Biol Lett, 2014, 1, 33-39). MAO-B preferentially oxidizes phenethylamine, and also uses dopamine and tyramine as substrates irrespective of their concentration. Several studies have shown the connection between MAO-B enzyme and neuropsychiatric/neurodegenerative disorders, personality traits, type II alcoholism, borderline personality disorders, aggressiveness and violence, obsessive-compulsive disorder, depression, suicides, schizophrenia, anorexia, migraine, dementia, Alzheimer's disease and Parkinson's disease, therefore, MAO-B represents an attractive target for the treatment of numerous diseases. (Carradori S, J Med Chem, 2015, 58, 6717-6732). Selegiline and rasagiline are selective and irreversible inhibitors of MAO-B. Moreover, the enzymatic activity is also inhibited by several other compounds - chalcones, pyrazole derivatives, chromones, coumarins, xanthines, thiazolidindiones, isatines, (thiazol-2-yl)hydrazones and their analogs, hydrazones, hydrazothiazoles, phthalimides, and derivatives of 8-phenoxymethylcaffeine (Carradori S, J Med Chem, 2015, 58, 6717-6732; Kumar B, RSC Adv, 2016, 6, 42660-42683). Among reversible inhibitors it is worth to mention lazabemide as antiparkinsonic, and safinamide for the treatment of CNS disorders, particularly Parkinson's disease, epilepsy, Alzheimer's disease, depression, restless legs syndrome and migraine (Asthon A, Pain: New Insights for the Healthcare Professional, 2013). The main therapeutic indications of MAO-B inhibitors from the literature are: Parkinson's disease (Riederer Z, Exp Neurobiol,

2011, 20, 1-17), Alzheimer's disease (Cai Z, Mol Med Rep. 2014, 9, 1533-1541), Huntington's disease (Ooi J, Mol Neurobiol, 2015, 52, 1850-1861), and amyotrophic lateral sclerosis.

The main structural feature of irreversible MAO-B inhibitors is *N*-propargylamine group, which is present in therapeutic drugs and potential drugs in clinical studies, namely rasagiline, selegiline, ladostigil, pargyline (Kumar B et al. RSC Adv., 2016, 6, 42660-42683).

US2003/0105133 discloses 4-subsituted piperidine analogs and their synthesis.

Pisani et al. (Curr Med Chem. 2011;18(30):4568-87) reviewed the discovery strategy, the mechanism of action and the biopharmacological evaluation of monoamine oxidase inhibitors exhibiting additional activities such as acetylcholinesterase inhibition, iron chelation/antioxidant-radical scavenging/anti-inflamatory/A2A receptor agonist and APP processing modulating activities.

**Solution to Problem**

The present invention relates to compound of Formula (I)

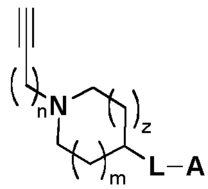

**(I)**

wherein:

**n:** 1-5;

**m:** 0, 1, or 2;

**z:** 0 or 1;

**L:** $=CH_2-$, $=CH_2CH_2-$ or

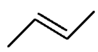

trans ;

and

**A:**

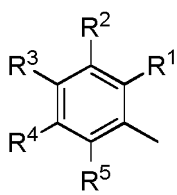

,

wherein

**R¹:** H, OH, Me, Et, i-Pr, t-Bu, i-Bu, CN, F, Cl, Br, $CF_3$, $CH_2CF_2$, $CONH_2$, cyclopropyl, cyclobutyl, cyclopentyl, $SR^6$, $OR^6$ or $NR^6R^7$

**$R^6$:** H, Me, Et, n-Pr, i-Pr, t-Bu or i-Bu,

**$R^7$:** H, Me, Et, n-Pr, i-Pr, t-Bu or i-Bu;

**$R^2$:** H, OH, Me, Et, i-Pr, t-Bu, i-Bu, CN, F, Cl, Br, $CF_3$, $CH_2CF_2$, $CONH_2$, cyclopropyl, cyclobutyl, cyclopentyl, $SR^6$, $OR^6$ or $NR^6R^7$

**$R^6$:** H, Me, Et, n-Pr, i-Pr, t-Bu or i-Bu,

**$R^7$:** H, Me, Et, n-Pr, i-Pr, t-Bu or i-Bu;

**$R^3$:** H, OH, Me, Et, i-Pr, t-Bu, i-Bu, CN, F, Cl, Br, $CF_3$, $CH_2CF_2$, $CONH_2$, cyclopropyl, cyclobutyl, cyclopentyl, $SR^6$, $OR^6$ or $NR^6R^7$

**$R^6$:** H, Me, Et, n-Pr, i-Pr, t-Bu or i-Bu,

**$R^7$:** H, Me, Et, n-Pr, i-Pr, t-Bu or i-Bu;

**$R^4$:** H, OH, Me, Et, i-Pr, t-Bu, i-Bu, CN, F, Cl, Br, $CF_3$, $CH_2CF_2$, $CONH_2$, cyclopropyl, cyclobutyl, cyclopentyl, $SR^6$, $OR^6$ or $NR^6R^7$

**$R^6$:** H, Me, Et, n-Pr, i-Pr, t-Bu or i-Bu,

**$R^7$:** H, Me, Et, n-Pr, i-Pr, t-Bu, or i-Bu; and

**$R^5$:** H, OH, Me, Et, i-Pr, t-Bu, i-Bu, CN, F, Cl, Br, $CF_3$, $CH_2CF_2$, $CONH_2$, cyclopropyl, cyclobutyl, cyclopentyl, $SR^6$, $OR^6$ or $NR^6R^7$

**$R^6$:** H, Me, Et, n-Pr, i-Pr, t-Bu or i-Bu,

**$R^7$:** H, Me, Et, n-Pr, i-Pr, t-Bu or i-Bu.

According to certain embodiments, A is

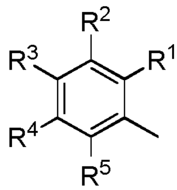

,

wherein

**$R^1$:** H, OH, Me, Et, i-Pr, t-Bu, i-Bu, CN, F, Cl, Br, $CF_3$, $CH_2CF_2$, $CONH_2$, cyclopropyl, cyclobutyl, cyclopentyl or $OCH_{2;}$

**$R^2$:** H, OH, Me, Et, i-Pr, t-Bu, i-Bu, CN, F, Cl, Br, $CF_3$, $CH_2CF_2$, $CONH_2$, cyclopropyl, cyclobutyl, cyclopentyl or $OCH_{2;}$

**$R^3$:** H, OH, Me, Et, i-Pr, t-Bu, i-Bu, CN, F, Cl, Br, $CF_3$, $CH_2CF_2$, $CONH_2$, cyclopropyl, cyclobutyl, cyclopentyl or $OCH_{2;}$

**$R^4$:** H, OH, Me, Et, i-Pr, t-Bu, i-Bu, CN, F, Cl, Br, $CF_3$, $CH_2CF_2$, $CONH_2$, cyclopropyl, cyclobutyl, cyclopentyl or $OCH_{2;}$ and

**$R^5$:** H, OH, Me, Et, i-Pr, t-Bu, i-Bu, CN, F, Cl, Br, $CF_3$, $CH_2CF_2$, $CONH_2$, cyclopropyl, cyclobutyl, cyclopentyl or $OCH_2$.

According to some embodiments, $R^1$ is H. According to some embodiments, $R^1$ is OH.

According to some embodiments, $R^1$ is Me. According to some embodiments, $R^1$ is Et. According to some embodiments, $R^1$ is i-Pr. According to some embodiments, $R^1$ is t-Bu. According to some embodiments, $R^1$ is i-Bu. According to some embodiments, $R^1$ is CN. According to some embodiments, $R^1$ is F. According to some embodiments, $R^1$ is Cl. According to some embodiments, $R^1$ is Br. According to some embodiments, $R^1$ is CF3. According to some embodiments, $R^1$ is $CH_2CF_2$. According to some embodiments, $R^1$ is $CONH_2$. According to some embodiments, $R^1$ is cyclopropyl.

According to some embodiments, $R^1$ is cyclobutyl. According to some embodiments, $R^1$ is cyclopentyl. According to some embodiments, $R^1$ is $OCH_2$.

According to some embodiments, $R^2$ is H. According to some embodiments, $R^2$ is OH. According to some embodiments, $R^2$ is Me. According to some embodiments, $R^2$ is Et. According to some embodiments, $R^2$ is i-Pr. According to some embodiments, $R^2$ is t-Bu. According to some embodiments, $R^2$ is i-Bu. According to some embodiments, $R^2$ is CN. According to some embodiments, $R^2$ is F. According to some embodiments, $R^2$ is Cl. According to some embodiments, $R^2$ is Br. According to some embodiments, $R^2$ is CF3. According to some embodiments, $R^2$ is $CH_2CF_2$. According to some embodiments, $R^2$ is $CONH_2$. According to some embodiments, $R^2$ is cyclopropyl. According to some embodiments, $R^2$ is cyclobutyl. According to some embodiments, $R^2$ is cyclopentyl. According to some embodiments, $R^2$ is $OCH_2$.

According to some embodiments, $R^3$ is H. According to some embodiments, $R^3$ is OH. According to some embodiments, $R^3$ is Me. According to some embodiments, $R^3$ is Et. According to some embodiments, $R^3$ is i-Pr. According to some embodiments, $R^3$ is t-Bu. According to some embodiments, $R^3$ is i-Bu. According to some embodiments, $R^3$ is CN. According to some embodiments, $R^3$ is F. According to some embodiments, $R^3$ is Cl. According to some embodiments, $R^3$ is Br. According to some embodiments, $R^3$ is $CF_3$. According to some embodiments, $R^3$ is $CH_2CF_2$. According to some embodiments, $R^3$ is $CONH_2$. According to some embodiments, $R^3$ is cyclopropyl. According to some embodiments, $R^3$ is cyclobutyl. According to some embodiments, $R^3$ is cyclopentyl. According to some embodiments, $R^3$ is $OCH_2$

According to some embodiments, $R^4$ is H. According to some embodiments, $R^4$ is OH. According to some embodiments, $R^4$ is Me. According to some embodiments, $R^4$ is Et. According to some embodiments, $R^4$ is i-Pr. According to some embodiments, $R^4$ is t-Bu. According to some embodiments, $R^4$ is i-Bu. According to some embodiments, $R^4$ is CN. According to some embodiments, $R^4$ is F. According to some embodiments, $R^4$ is Cl. According to some embodiments, $R^4$ is Br. According to some embodiments, $R^4$ is CF3. According to some embodiments, $R^4$ is $CH_2CF_2$. According to some embodiments, $R^4$ is $CONH_2$. According to some embodiments, $R^4$ is cyclopropyl.

According to some embodiments, $R^4$ is cyclobutyl. According to some embodiments, $R^4$ is cyclopentyl. According to some embodiments, $R^4$ is $OCH_2$

According to some embodiments, $R^5$ is H. According to some embodiments, $R^5$ is OH. According to some embodiments, $R^5$ is Me. According to some embodiments, $R^5$ is Et. According to some embodiments, $R^5$ is i-Pr. According to some embodiments, $R^5$ is t-Bu. According to some embodiments, $R^5$ is i-Bu. According to some embodiments, $R^5$ is CN. According to some embodiments, $R^5$ is F. According to some embodiments, $R^5$ is Cl. According to some embodiments, $R^5$ is Br. According to some embodiments, $R^5$ is $CF_3$. According to some embodiments, $R^5$ is $CH_2CF_2$. According to some embodiments, $R^5$ is $CONH_2$. According to some embodiments, $R^5$ is cyclopropyl. According to some embodiments, $R^5$ is cyclobutyl. According to some embodiments, $R^5$ is cyclopentyl. According to some embodiments, $R^5$ is $OCH_2$.

According to certain embodiments, A is mono-substituted, such as at position $R^2$ or $R^3$ (the remaining positions are each H).

According to certain embodiments, A is di-subsituted, such as at positions $R^1$ and $R^3$ (the remaining positions are each H).

According to certain embodiments, A is tri-subsituted, such as at positions $R^1$, $R^3$ and $R^4$ (the remaining positions are each H).

According to some embodiments, $R^1$, $R^2$, $R^4$ and $R^5$ are each H, and $R^3$ is Me, Et, i-Pr, t-Bu, i-Bu, CN, F, Cl, Br, $CF_3$, cyclopropyl, cyclobutyl, cyclopentyl or $OCH_2$. According to some embodiments, $R^1$, $R^2$, $R^4$ and $R^5$ are each H, and $R^3$ is Me, i-Pr, CN, F, Cl, Br, $CF_3$ or cyclopropyl.

According to some embodiments, n is 1-5. According to some embodiments, n is 1-4. According to some embodiments n is 1-3. According to some embodiments n is 1 or 2. According to some embodiments, n is 1. According to some embdoments, n is 2. According to some embodiments, n is 3. According to some embodiments, n is 4. According to some embodiments, n is 5.

According to some embodiments, m is 0. According to some embodiments, m is 1. According to some embodiments, m is 2.

According to some embodiments z is 0. According to some embodiments z is 1.

According to some embodiments, m is 1 and z is 1. According to some embodiments, n is 1, m is 1 and z is 1. According to some embodiments, n is 2, m is 1 and z is 1.

According to some embodiments, L is $=CH_2$-. According to some embodiments, L is $=CH_2CH_2$-. According to some embodiments, L is

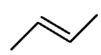

trans .

A compound according to the present invention may be a compound selected from the group consisting of:

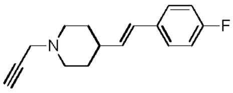, 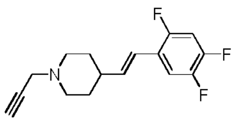, 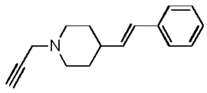, 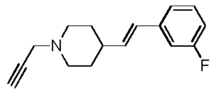,

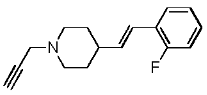, 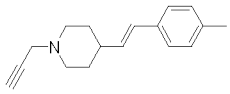, 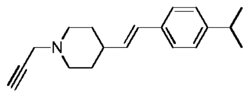, 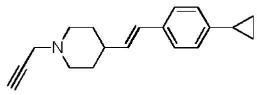,

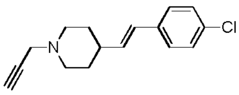, 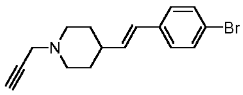, 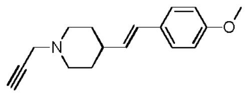, 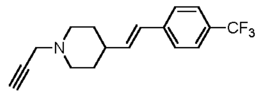,

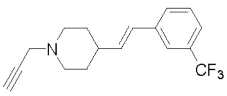, 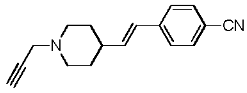, 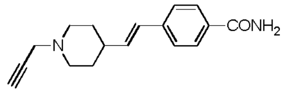, 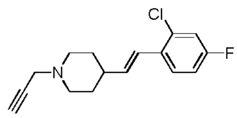,

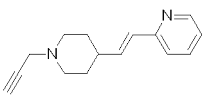, 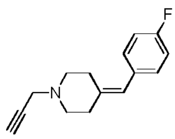, 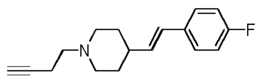, 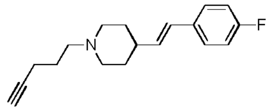,

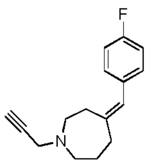, 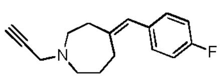, 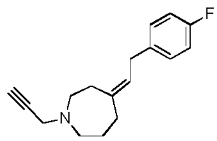, 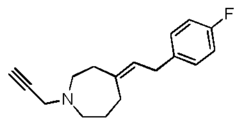

and

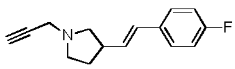.

According to one embodiment, the compound of the present invention is selected from the group consisting of:

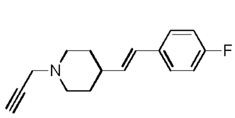, 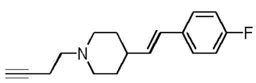, 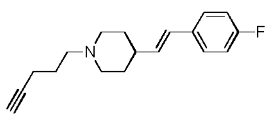, 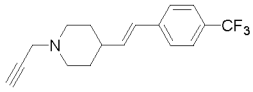,

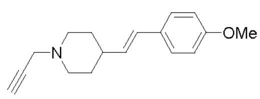, and 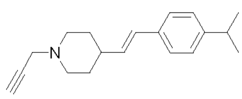.

According to one embodiment, the compound of the present invention is (has the following structure)

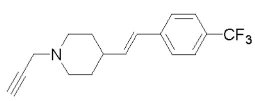.

According to one embodiment, the compound of the present invention is (has the following structure)

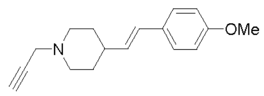

.

According to one embodiment, the compound of the present invention is (has the following structure)

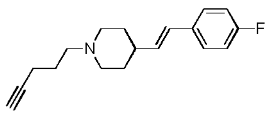

.

Some compounds from this invention possess stereogenic center with an absolute configuration of R or S, where the compounds can appear in a racemic form, in a form of conglomerate or in a form of pure enantiomers.

The invention further relates to the pharmaceutically acceptable salts, hydrates and solvates of the compounds with the general Formula (I).

The invention further relates to the use of the compounds with the general Formula (I) as active pharmaceutical ingredients for the preparation of medicaments. Compounds with the general Formula (I) are inhibitors of MAO-B enzyme and are used for the diagnostics, prevention, alleviation of symptoms and treatment of acute and chronic neurological disorders, cognitive and neurodegenerative diseases such as Parkinson's disease, Alzheimer's disease, Huntington's disease and dementia.

The invention is related to parenteral, oral, or other pharmaceutically acceptable forms containing the compounds with the general Formula (I). Beside active pharmaceutical ingredient, the pharmaceutical composition can contain excipients suitable for the intended route of administration. Pharmaceutical compositions are prepared using standard procedures. Pharmaceutical compositions can be prepared in the way that ensures the sustained and prolonged release of the active pharmaceutical ingredient. The dose, frequency, and way of use depend on several factors, which further depend on the active pharmaceutical ingredient used, its pharmacokinetic and pharmacodynamic properties and the condition of the patient.

According to certain embodiments, a compound of the present invention is used in the preparation of a medicament.

According to certain embodiments, a compound of the present invention is for use as a medicament.

According to some embodiments, a compound of the present invention is for use in the prevention, alleviation of symptoms or treatment of a medical condition selected from the group consisting of acute and chronic neurological disorders, cognitive and neurodegenerative diseases.

According to some embodiments, a compound of the present invention is for use in the prevention, alleviation of symptoms or treatment of a neurodegenerative disease, such as Parkinson's disease, Alzheimer's disease, Huntington's disease or dementia.

According to some embodiments, a compound of the present invention is for use in the prevention, alleviation of symptoms or treatment of Parkinson's disease, Alzheimer's disease, Huntington's disease or dementia.

According to some embodiments, a compound of the present invention is for use in the prevention, alleviation of symptoms or treatment of Parkinson's disease.

According to some embodiments, a compound of the present invention is for use in the prevention, alleviation of symptoms or treatment of Alzheimer's disease
According to some embodiments, a compound of the present invention is for use in the prevention, alleviation of symptoms or treatment of Huntington's disease.

According to some embodiments, a compound of the present invention is for use in the prevention, alleviation of symptoms or treatment of dementia.

Compounds with the general Formula (I) are prepared using the procedures, described in the section Synthesis of the compounds with the general Formula (I).

**Examples**

The present invention is further exemplified, but not limited by, the following examples that illustrate the preparation of compounds of Formula (I).

**Synthesis of the compounds with the general Formula (I)**

Reaction scheme 1: Synthesis of (*E*) vinyl/alkenyl and reduced derivatives

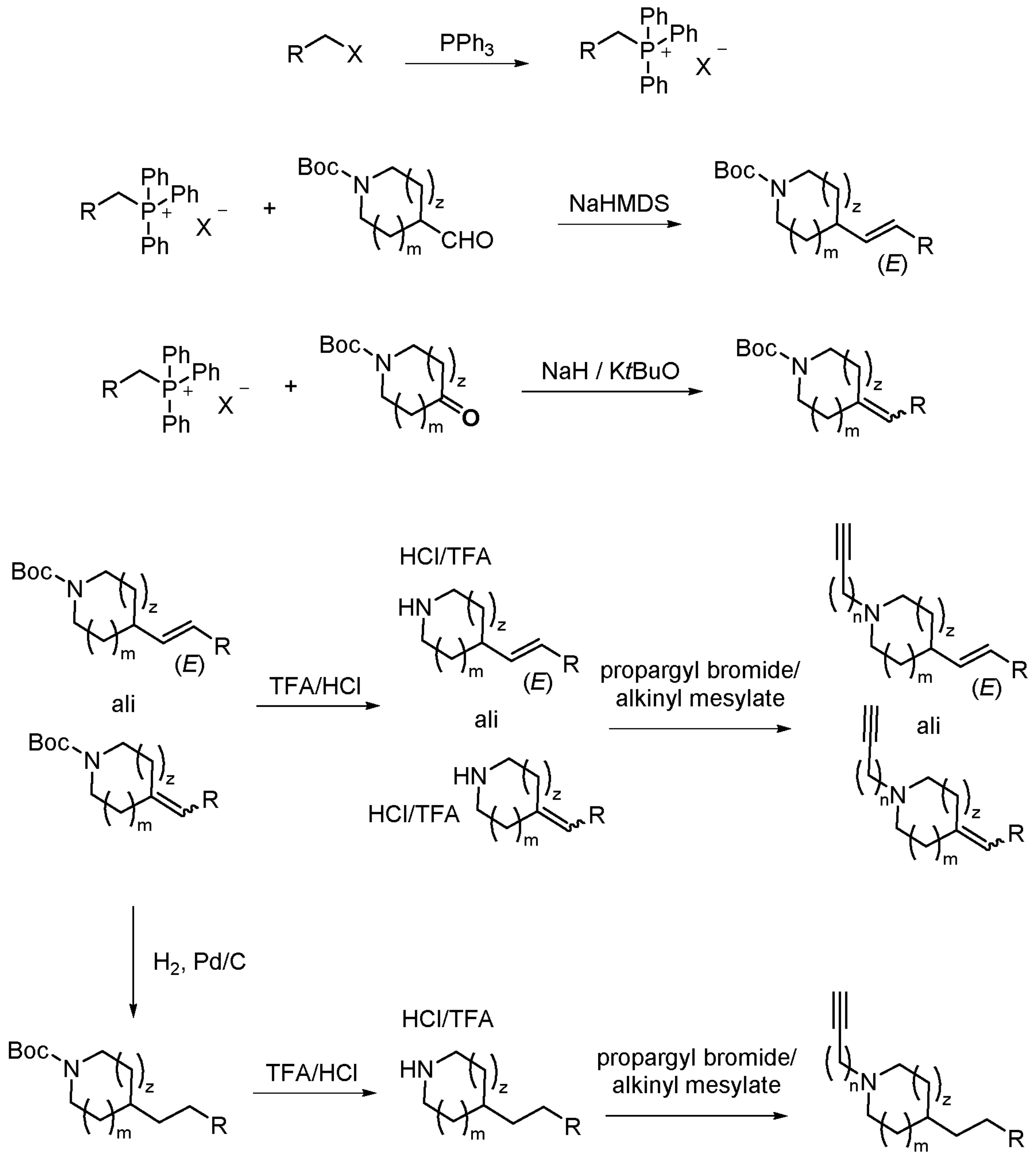


**General synthetic procedures**

**General procedure A: Synthesis of Wittig reagents**

Appropriate benzyl or phenethyl halide (25.0 mmol, 1.0 equiv.) was dissolved in MeCN (25 mL), and triphenylphosphine (6.56 g, 25.0 mmol, 1.0 equiv.) was added at room temperature. The reaction mixture was stirred at 85 °C for 16-24 h. The solvent was evaporated and $CH_2Cl_2$ (10 mL) and $Et_2O$ (50 mL) were added to the residue. The precipitated solid was filtered off, washed with $Et_2O$ (20 mL), and dried overnight at room temperature. The crude product

was used in the following reaction step without further purification.

**General procedure B: Wittig reaction**

Appropriate Witting reagent synthesized according to the general procedure A (1.1 equiv.) was dissolved in anhydrous THF (30 mL) under argon atmosphere. Sodium bis(trimethylsilyl)amide-NaHMDS (2N solution in THF, 1.2 equiv.) was added to the resulting suspension at room temperature. The orange-red colored reaction mixture was then stirred for 30 min under argon, followed by a drop-wise addition of *N*-Boc protected 4- formylpiperidine (1.0 equiv.) solution in anhydrous THF (10 mL). The reaction mixture was stirred at room temperature overnight (16-24 h), and then quenched by adding saturated aqueous $NaHCO_3$ solution (10-15 mL). The solvent was evaporated and the white residue was resuspended in EtOAc (50 mL) and saturated aqueous $NaHCO_3$ solution (50 mL), and transferred into a separating funnel. The phases were separated and the aqueous phase was additionally extracted with EtOAc (2 × 50 mL). Combined organic phases were washed with saturated brine (100 mL), dried over anhydrous $Na_2SO_4$, and evaporated under reduced pressure. The crude product was purified by flash column chromatography to yield pure *trans* (*Z*) isomer and a mixture of both isomers that was used for reduction of the double bond.

**General procedure C: reduction of the double bond**

Alkene derivative (Wittig reaction product-mixture of *cis* and *trans* isomer, 1.0 equiv.) was dissolved in EtOH or MeOH (20-50 mL) and purged under a stream of argon for 10 min. Catalytic amount of Pd/C (10% load on carbon, 10-20% [w/w] calculated to the starting material) was added, and the resulting suspension mixture was stirred under $H_2$(g) atmosphere at room temperature for 16-24 h. The catalyst was removed by filtration through a pad of Celite® and evaporated to obtain crude product that was used in the next reaction step.

**General procedure D: Boc protection removal with HCl or TFA**

To the solution of Boc protected derivative (1.0 equiv.) in EtOH or 1,4-dioxane (30-50 mL), or $CH_2Cl_2$, concentrated HCl (10.0 equiv., method with HCl) or TFA (40.0 equiv., method with TFA) was added drop-wise. After stirring for 1-2 h at 85 °C (method with HCl) or 4-20 h at room temperature (method with TFA), the reaction mixture was evaporated. The crude material was used in the next reaction step without further purification.

**General procedure E: *N*-alkylation**

Intermediate in the form of the salt with HCl from the previous reaction step (procedure D) was dissolved in MeCN (20-30 mL) or DMF (5-10 mL) at 0 °C under argon atmosphere. $K_2CO_3$ or $CS_2CO_3$ (3.0 equiv.) was added, followed by a drop-wise addition of propargyl bromide (80 % solution in toluene, 1.2 equiv.). The resulting suspension was stirred at room temperature (unless specified otherwise) for 16-72 h. The solvent was evaporated, the residue dissolved in a mixture of $CH_2Cl_2$ (50 mL) and saturated aqueous $NaHCO_3$ solution (50 mL), and transferred into a separating funnel. The organic phase was further washed with saturated brine solution (50 mL), dried over anhydrous $Na_2SO_4$, and evaporated under reduced pressure. The crude product was purified by flash column chromatography.

**Procedure F1: Synthesis of *tert*-butyl 4-formylpiperidine-1-carboxylate**

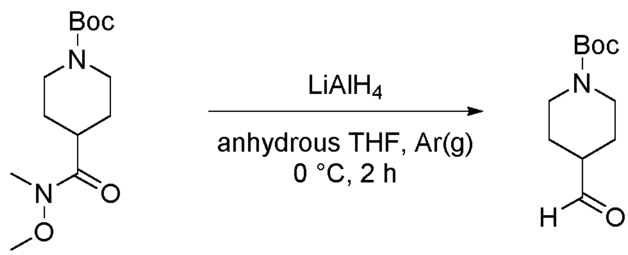


*Tert*-butyl 4-formylpiperidine-1-carboxylate was synthesized following the published procedure (Caroon JM, PCT Int. Appl. 9943657, Sep 02, 1999) starting from *tert*-butyl 4-(methoxy(methyl)carmaboyl)piperidine-1-carboxylate. The crude product was used in the Wittig reaction without further purification.

**Procedure F2: Synthesis of tert-butyl 4-formylpyrrolidine-1-carboxylate**

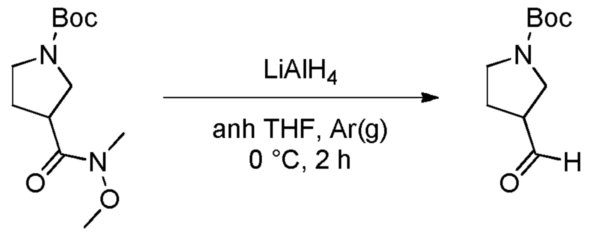


*Tert*-butyl 4-formylpyrrolidine-1-carboxylate was synthesized following the published procedure (Caroon JM, PCT Int. Appl. 9943657, Sep 02, 1999) starting from *tert*-butyl 4-(methoxy(methyl)carmaboyl)pyrrolidine-1-carboxylate. The crude product was used in the Wittig reaction without further purification.

**Procedure G: Synthesis of *tert*-butyl 4-oxoazepane-1-carboxylate**

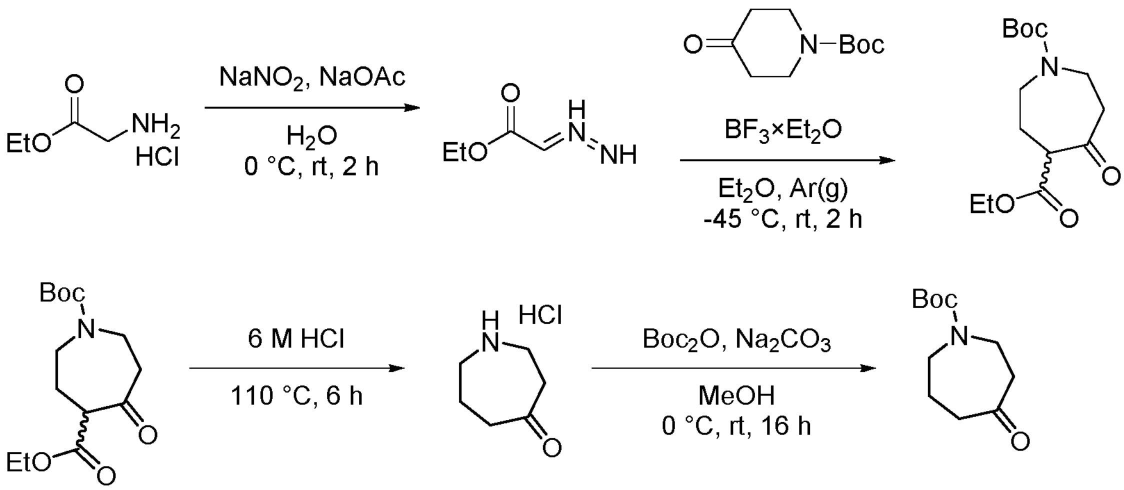


*Tert*-butyl 4-oxoazepane-1-carboxylate was synthesized following the published procedure (Huang YS, Ind End Chem Res, 2010, 49, 12164-12167) starting from glycine ethyl ester (salt with HCl) and *N*-Boc-4-piperidone (9,19 g, 46,12 mmol, 1,0 ekviv.).The crude product was used in the Wittig reaction without further purification. $R_f$ = 0.39 (EtOAc/n-hex = 1/1, v/v); colorles oil; $^1$H NMR (400 MHz, $CDCl_3$): δ 1.42 (s, 9H), 1.72-1.78 (m, 2H), 2.60-2.63 (m, 4H), 3.53-3.58 (m, 4H).

**Procedure H: Synthesis of activated alcohols with mesyl chloride**

The primary alcohol (1.0 equiv.) was dissolved in $CH_2Cl_2$ (20 mL), solution purged under a stream of argon for 10 min, and cooled to 0 °C. $Et_3N$ (1.05 equiv.) and methansulfonyl chloride (1.05 equiv.) were added consecutively. The reaction mixture was stirred at room temperature for 3 h. The resulting suspension was transferred into a separating funnel and washed with $H_2O$ (2 × 20 mL), saturated brine solution (30 mL), dried over anhydrous $Na_2SO_4$, and evaporated under reduced pressure. Crude intermediate (mesylate) was used in the next reaction step without purification.

EXAMPLE 1

**Synthesis of (*E*)-4-(4-fluorostyryl)-1-(prop-2-yn-1-yl)piperidine**

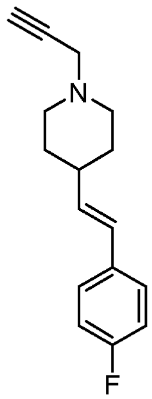

*Step 1:* ***Tert*-butyl (*E*)-4-(4-fluorostyryl)piperidine-1-carboxylate**

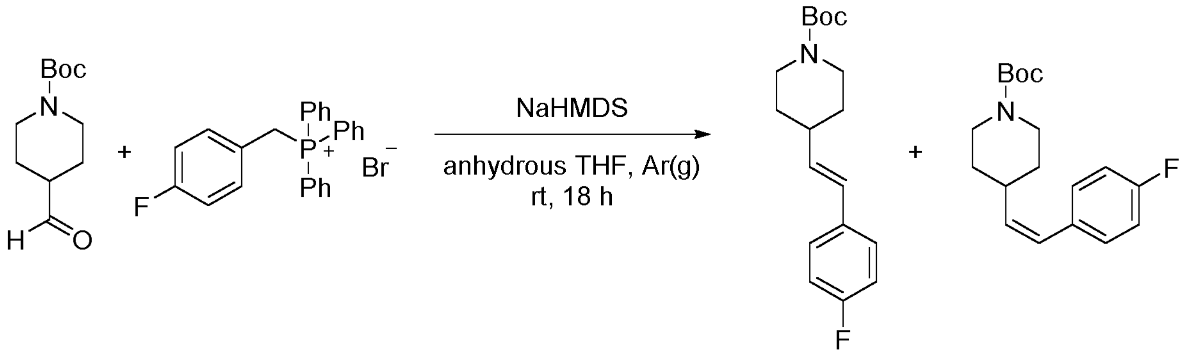


Synthesized from *tert*-butyl 4-formylpiperidine-1-carboxylate (2.00 g, 9.38 mmol, 1.0 equiv.) and 4-fluorobenzyltriphenylphosphonium bromide (4.66 g, 10.32 mmol, 1.1 equiv., prepared via general procedure A) via general procedure B. The compound was purified by flash column chromatography using petroleum ether/$Et_2O$ = 10/1 (v/v) as eluent. Mass: 155 mg; $R_f$ = 0.79 (EtOAc/n-hex = 1/2, v/v); yellow transparent oil; $^1$H NMR (400 MHz, $CDCl_3$): δ 1.30-1.42 (m, 2H), 1.46 (s, 9H), 1.51-1.57 (m, 1H), 1.71-1.77 (m, 2H), 2.77 (dt, *J* = 13.2, 2.7 Hz, 2H), 4.13 (d, *J* = 13.0 Hz, 2H), 6.05 (dd, *J* = 15.9, 6.9 Hz, 1H), 6.34 (d, *J* = 16.2 Hz, 1H), 6.95-7.01 (m, 2H), 7.27-7.32 (m, 2H).

*Step 2:* **(*E*)-4-(4-Fluorostyryl)-1-(prop-2-yn-1-yl)piperidine**

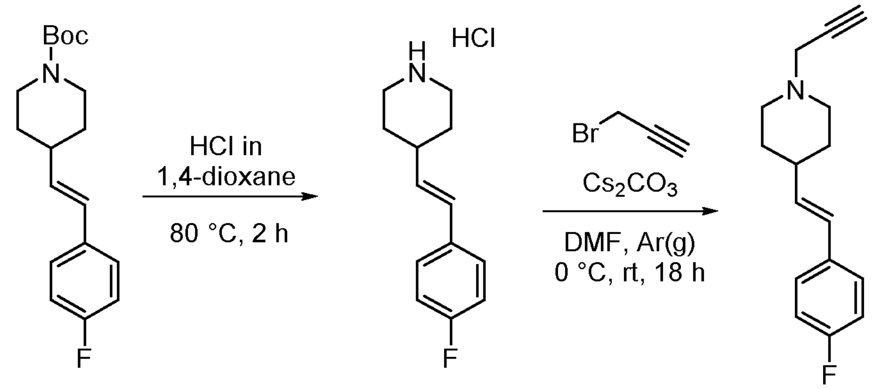


Synthesized from *tert*-butyl (*E*)-4-(4-fluorostyryl)piperidine-1-carboxylate (157 mg, 0.514 mmol, 1.0 ekviv.) via general procedures D and E. The compound was purified by flash column chromatography using EtOAc/n-hex = 1/1

(v/v) as eluent. Yield: 26% (33 mg); $R_f$ = 0.17 (EtOAc/n-hex = 1/1, v/v); yellow oil; $^1$H NMR (400 MHz, $CDCl_3$): δ 1.57 (dt, *J* = 12.3, 3.4 Hz, 1H), 1.60 (dt, *J* = 12.0, 3.7 Hz, 1H), 1.77-1.83 (m, 2H), 2.08-2.18 (m, 1H), 2.27 (t, *J* = 2.4 Hz, 1H), 2.30 (dd, *J* = 11.7, 2.0 Hz, 2H), 2.95 (td, *J* = 11.1, 2.8 Hz, 2H), 3.34 (d, *J* = 2.4 Hz, 2H), 6.07 (dd, *J* = 16.0, 7.0 Hz, 1H), 6.34 (d, *J* = 16.0 Hz, 1H), 6.95-7.00 (m, 2H), 7.28-7.33 (m, 2H); $^{13}$C NMR (100 MHz, $CDCl_3$): δ 31.88, 38.70, 47.19, 52.18, 73.26, 78.74, 115.32 (d, $J_{C,F}$ = 21.4 Hz), 127.15, 127.41 (d, $J_{C,F}$ = 7.7 Hz), 133.71 (d, $J_{C,F}$ = 3.3 Hz), 134.50, 161.95 (d, $J_{C,F}$ = 245.9 Hz); HRMS (ESI+): *m*/*z* calcd for $C_{16}H_{19}FN$ $[M+H]^+$ 244.1502; found 244.1508; HPLC purity, 96.2%.

REFERENCE EXAMPLE 1

**Synthesis of 4-(4-fluorophenethyl)-1-(prop-2-yn-1-yl)piperidine**

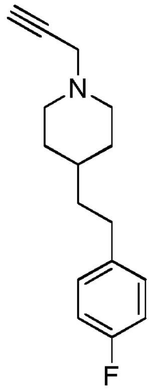

*Step 1:* ***Tert*-butyl 4-(4-fluorophenethyl)piperidine-1-carboxylate**

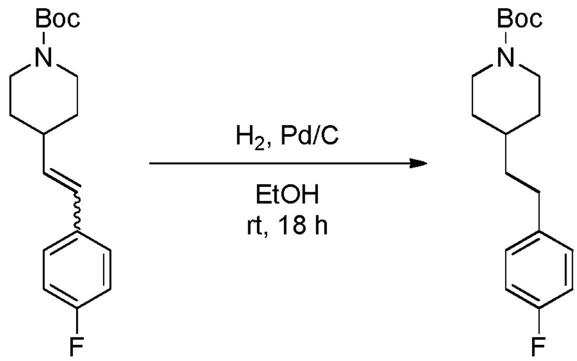


Synthesized from *tert*-butyl (*E*/*Z*)-4-(4-fluorostyryl)piperidine-1-carboxylate (0.152 g, 0.50 mmol, 1.0 equiv.) via general procedure C. The product was used without further purification. Yield: quantitative (0.155 g); Rf = 0.86 (EtOAc/n-hex = 1/2, v/v); yellow transparent oil; 1H NMR (400 MHz, $CDCl_3$): δ 1.11 (dt, J = 12.4, 4.2 Hz, 1H), 1.14 (dt, J = 12.5, 4.2 Hz, 1H), 1.35-1.44 (m, 1H), 1.45 (s, 9H), 1.51-1.56 (m, 2H), 1.60-1.62 (m, 1H), 1.65-1.72 (m, 2H), 2.57-2.62 (m, 1H), 2.66 (t, J = 11.5 Hz, 2H), 4.07 (bs, 2H), 6.93-6.99 (m, 2H), 7.09-7.14 (m, 2H); HRMS (ESI+): m/z calcd for $C_{18}H_{26}FNO_2Na$ $[M+Na]^+$ 330.1845; found 330.1848.

*Step 2:* **4-(4-Fluorophenethyl)-1-(prop-2-yn-1-yl)piperidine**

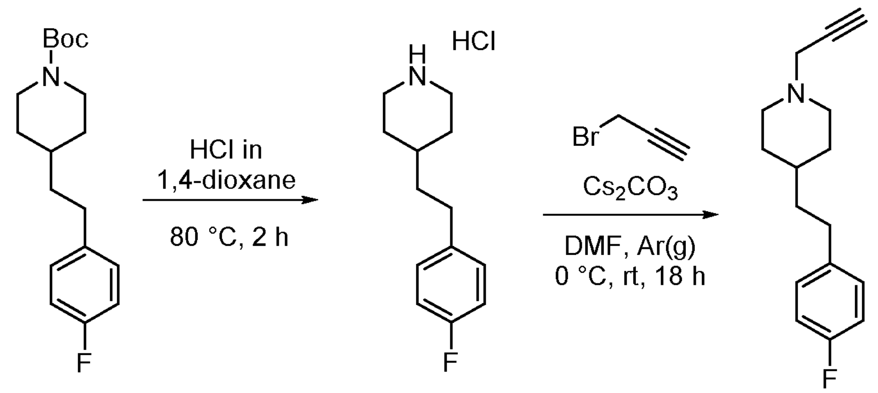


Synthesized from *tert*-butyl 4-(4-fluorophenethyl)piperidine-1-carboxylate (0.152 g, 0.45 mmol, 1.0 equiv.) via general procedures D and E. The compound was purified by flash column chromatography using EtOAc/n-hex = 1/1 (v/v) as eluent. Yield: 85% (88 mg); $R_f$ = 0.17 (EtOAc/n-hex = 1/1, v/v); colorless oil; $^1$H NMR (400 MHz, $CDCl_3$): δ 1.21-1.40 (m, 3H), 1.52-1.57 (m, 2H), 1.74-1.78 (m, 2H), 2.18-2.23 (m, 2H), 2.25 (t, $J$ = 2.4 Hz, 1H), 2.57-2.61 (m, 2H), 2.89-2.94 (m, 2H), 3.33 (d, $J$ = 2.4 Hz, 2H), 6.82-6.98 (m, 2H), 7.09-7.14 (m, 2H); $^{13}$C NMR (100 MHz, $CDCl_3$): δ 32.07, 32.21, 34.60, 38.35, 47.15, 52.47, 73.09, 78.90, 114.99 (d, $J_{C,F}$ = 21.1 Hz), 129.54 (d, $J_{C,F}$ = 7.9 Hz), 138.16 (d, $J_{C,F}$ = 3.1 Hz), 161.11 (d, $J_{C,F}$ = 243.2 Hz); HRMS (ESI+): *m/z* calcd for $C_{16}H_{21}FN$ [M+H]$^+$ 246.1658; found 246.1657; HPLC purity, 97.5%.

EXAMPLE 2

**Synthesis of (*E*)-4-(2,4,5-trifluorostyryl)-1-(prop-2-yn-1-yl)piperidine**

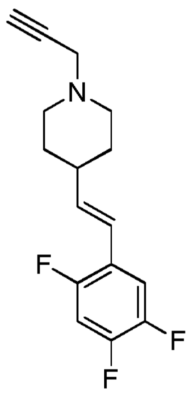

*Step 1:* ***Tert*-butyl (*E*)-4-(2,4,5-trifluorostyryl)piperidine-1-carboxylate**

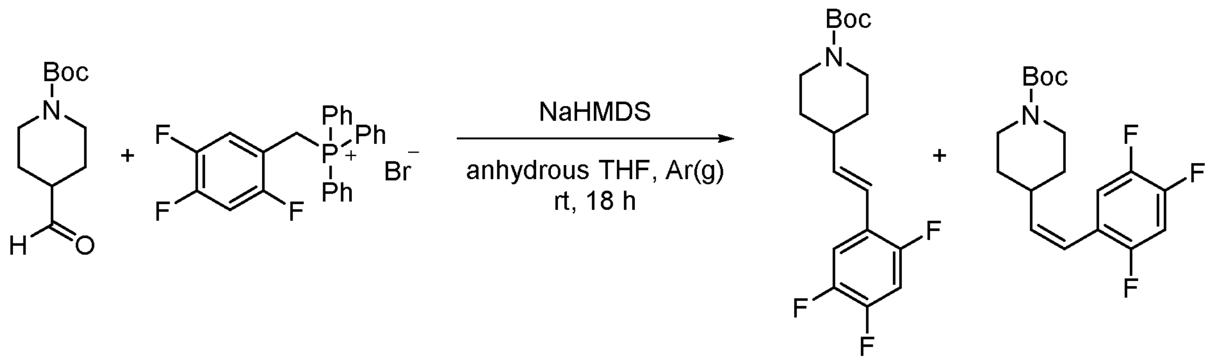


Synthesized from *tert*-butyl 4-formylpiperidine-1-carboxylate (0.575 g, 2.70 mmol, 1.0 equiv.) and 2,4,5-trifluorobenzyltriphenylphosphonium bromide (1.59 g, 3.25 mmol, 1.1 equiv., prepared via general procedure A) via general procedure B. The compound was purified by flash column chromatography using petroleum ether/$Et_2O$ = 10/1 (v/v) as eluent. Mass: 135 mg; $R_f$ = 0.78 (EtOAc/n-hex = 1/2, v/v); pale yellow amorphous solid, mp 60-63 °C; $^1H$ NMR (400 MHz, $CDCl_3$): δ 1.36 (dt, *J* = 12.4, 4.2 Hz, 1H), 1.39 (dt, *J* = 12.3, 4.3 Hz, 1H), 1.47 (s, 9H), 1.71-1.79 (m, 2H), 2.25-2.36 (m, 1H), 2.78 (t, *J* = 12.0 Hz, 2H), 4.14 (bs, 2H), 6.13 (dd, *J* = 16.1, 6.9 Hz, 1H), 6.44 (d, *J* = 16.1 Hz, 1H), 6.88 (dt, *J* = 9.9, 6.6 Hz, 1H), 7.01 (ddd, *J* = 11.1, 8.8, 7.0 Hz, 1H); HRMS (ESI+): *m/z* calcd for $C_{18}H_{22}F_3NO_2Na$ $[M+Na]^+$ 364.1500; found 364.1497.

*Step 2:* **(*E*)-4-(2,4,5-Trifluorostyryl)-1-(prop-2-yn-1-yl)piperidine**

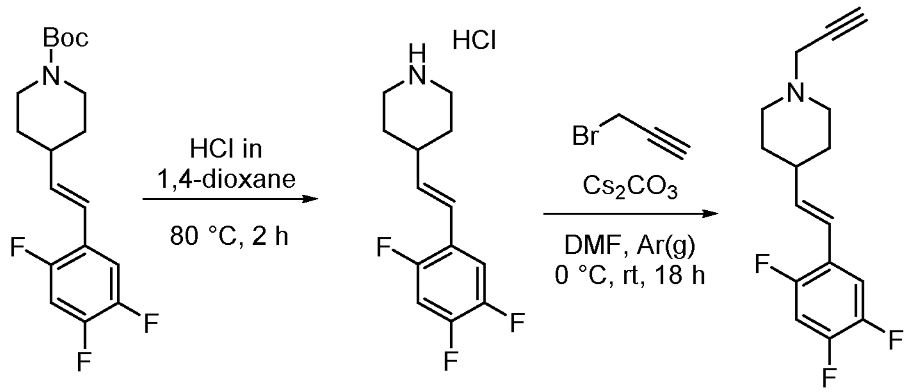


Synthesized from *tert*-butyl (*E*)-4-(2,4,5-trifluorostyryl)piperidine-1-carboxylate (135 mg, 0.395 mmol, 1.0 ekviv.) via general procedures D and E. The compound was purified by flash column chromatography using EtOAc/n-hex = 1/1 (v/v) as eluent. Yield: 74% (82 mg); $R_f$ = 0.26 (EtOAc/n-hex = 1/1, v/v); yellow oil; $^1H$ NMR (400 MHz, $CDCl_3$): δ 1.56 (dt, *J* = 12.4, 3.4 Hz, 1H), 1.59 (dt, *J* = 12.0, 3.7 Hz, 1H), 1.79-1.84 (m, 2H), 2.11-2.20 (m, 1H), 2.26 (t, *J* = 2.4 Hz, 1H), 2.30 (dt, *J* = 11.7, 2.3 Hz, 2H), 2.95 (dt, *J* = 11.6, 2.8 Hz, 2H), 3.34 (d, *J* = 2.4 Hz, 2H), 6.13 (dd, *J* = 16.1, 7.0 Hz, 1H), 6.43 (d, *J* = 16.1 Hz, 1H), 6.87 (dt, *J* = 9.9, 6.7 Hz, 1H), 7.23 (ddd, *J* = 11.1, 8.8, 7.0 Hz, 1H); $^{13}C$ NMR (100 MHz, $CDCl_3$): δ 31.69, 39.02, 47.17, 52.07, 73.23, 78.73, 105.51 (dd, $J_{C,F}$ = 28.5, 20.9 Hz), 114.06 (dd, $J_{C,F}$ = 19.6, 5.2 Hz), 118.96-119.03 (m), 121.95 (ddd, $J_{C,F}$ = 14.8, 5.8, 4.4 Hz), 138.10-138.17 (m), 146.93 (ddd, $J_{C,F}$ = 243.5, 12.9, 3.4 Hz), 148.76 (ddd, $J_{C,F}$ = 251.1, 14.9, 12.6 Hz); 154.73 (ddd, $J_{C,F}$ = 247.4, 9.1, 2.3 Hz); HRMS (ESI+): *m/z* calcd for $C_{16}H_{17}F_3N$ $[M+H]^+$ 280,1313; found 280,1318; HPLC purity, 100%.

REFERENCE EXAMPLE 2

**Synthesis of 4-(2,4,5-trifluorophenethyl)-1-(prop-2-yn-1-yl)piperidine**

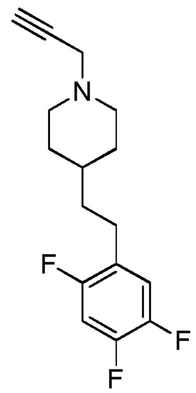

*Step 1:* ***Tert*-butyl 4-(2,4,5-trifluorophenethyl)piperidine-1-carboxylate**

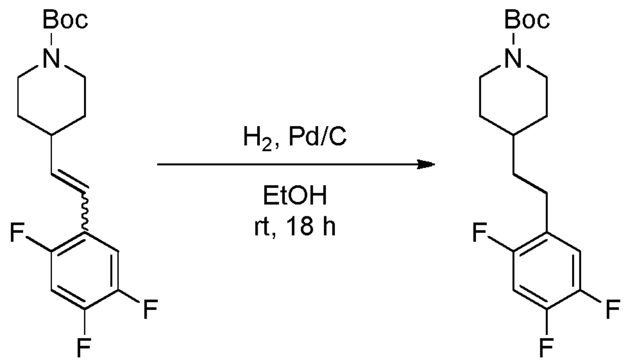


Synthesized from *tert*-butyl (*E*/*Z*)-4-(2,4,5-trifluorostyryl)piperidine-1-carboxylate (0.152 g, 0.45 mmol, 1.0 equiv.) via general procedure C. The product was used without further purification. Yield: quantitative (0.155 g); $R_f$ = 0.86 (EtOAc/n-hex = 1/2, v/v); yellow transparent oil; $^1H$ NMR (400 MHz, $CDCl_3$): δ 1.11 (dt, *J* = 12.4, 4.3 Hz, 1H), 1.14 (dt, *J* = 12.7, 4.2 Hz, 1H), 1.35-1.44 (m, 1H), 1.45 (s, 9H), 1.49-1.54 (m, 2H), 1.66-1.73 (m, 2H), 2.59 (t, *J* = 8.0 Hz, 2H), 2.66 (dt, *J* = 13.2, 2.5 Hz, 2H), 4.09 (d, *J* = 13.2 Hz, 2H), 6.87 (ddd, *J* = 10.0, 9.4, 6.7 Hz, 1H), 6.97 (ddd, *J* = 10.6, 8.8, 7.1 Hz, 1H); HRMS (ESI+): *m*/*z* calcd for $C_{18}H_{24}F_3NO_2Na$ $[M+Na]^+$ 366.1657; found 366.1652.

*Step 2:* **4-(2,4,5-Trifluorophenethyl)-1-(prop-2-yn-1-yl)piperidine**

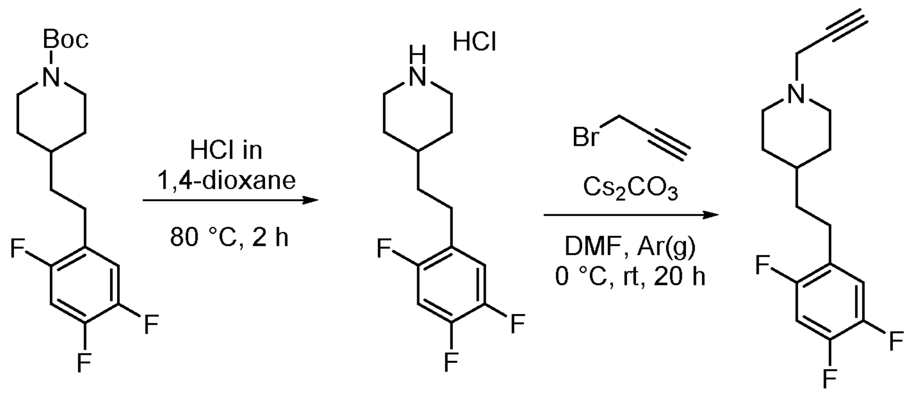


Synthesized from *tert*-butyl 4-(2,4,5-trifluorophenethyl)piperidine-1-carboxylate (0.175 g, 0.509 mmol, 1.0 equiv.) via general procedures D and E. The compound was purified by flash column chromatography using EtOAc/n-hex = 1/1 (v/v) as eluent. Yield: 32% (45 mg); $R_f$ = 0.16 (EtOAc/n-hex = 1/1, v/v); yellow transparent oil; $^1$H NMR (400 MHz, $CDCl_3$): δ 1.22-1.39 (m, 3H), 1.49-1.55 (m, 2H), 1.72-1.78 (m, 2H), 2.16-2.22 (m, 2H), 2.23-2.25 (m, 1H), 2.59 (t, *J* = 7.8 Hz, 2H), 2.88-2.93 (m, 2H), 3.30-3.32 (m, 2H), 6.86 (ddd, *J* = 10.0, 9.5, 6.7 Hz, 1H), 6.97 (ddd, *J* = 10.7, 8.8, 6.5 Hz, 1H); $^{13}$C NMR (100 MHz, $CDCl_3$): δ 25.55, 31.94, 34.68, 36.55, 47.13, 52.40, 73.16, 78.81, 105.18 (dd, $J_{C,F}$ = 28.7, 20.6 Hz), 117.66 (dd, $J_{C,F}$ = 19.1, 6.5 Hz), 125.50-125.82 (m), 146.57 (ddd, $J_{C,F}$ = 243.6, 12.6, 3.2 Hz), 148.09 (ddd, $J_{C,F}$ = 249.2, 13.4, 11.5 Hz); 155.72 (ddd, $J_{C,F}$ = 243.4, 9.3, 2.7 Hz); HRMS (ESI+): *m/z* calcd for $C_{16}H_{19}F_3N$ $[M+H]^+$ 282.1470; found 282.1471; HPLC purity, 100%.

EXAMPLE 3

**Synthesis of (*E*)-4-(3-fluorostyryl)-1-(prop-2-yn-1-yl)piperidine**

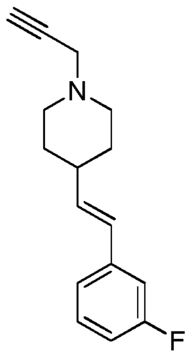

*Step 1:* ***Tert*-butyl (*E*)-4-(3-fluorostyryl)piperidine-1-carboxylate**

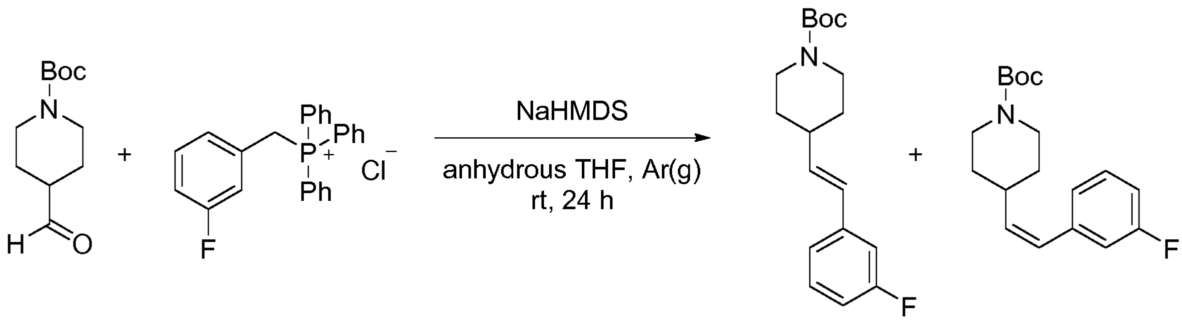


Synthesized from *tert*-butyl 4-formylpiperidine-1-carboxylate (2.00 g, 9.38 mmol, 1.0 equiv.) and 3-fluorobenzyltriphenylphosphonium chloride (4.20 g, 10.32 mmol, 1.1 equiv., prepared via general procedure A) via general procedure B. The compound was purified by flash column chromatography using petroleum ether/$Et_2O$ = 10/1 (v/v) as eluent. Mass: 355 mg; $R_f$ = 0.67 (EtOAc/n-hex = 1/2, v/v); white solid, mp 45-46 °C; $^1$H NMR (400 MHz, $CDCl_3$): δ 1.31-1.44 (m, 2H), 1.47 (s, 9H), 1.71-1.78 (m, 2H), 2.23-2.33 (m, 1H), 2.78 (t, *J* = 12.2 Hz, 2H), 4.13 (bs, 2H), 6.15 (dd, *J* = 16.0, 6.8 Hz, 1H), 6.35 (d, *J* = 16.0 Hz, 1H), 6.89 (ddt, *J* = 8.4, 2.6, 0.9 Hz, 1H), 7.02-7.06 (m, 1H), 7.07-7.11 (m, 1H), 7.25 (td, *J* = 8.0, 5.8 Hz, 1H); MS (ESI+): *m/z* $[M+Na]^+$ 328.17; found 328.40.

*Step 2:* **(*E*)-4-(3-Fluorostyryl)-1-(prop-2-yn-1-yl)piperidine**

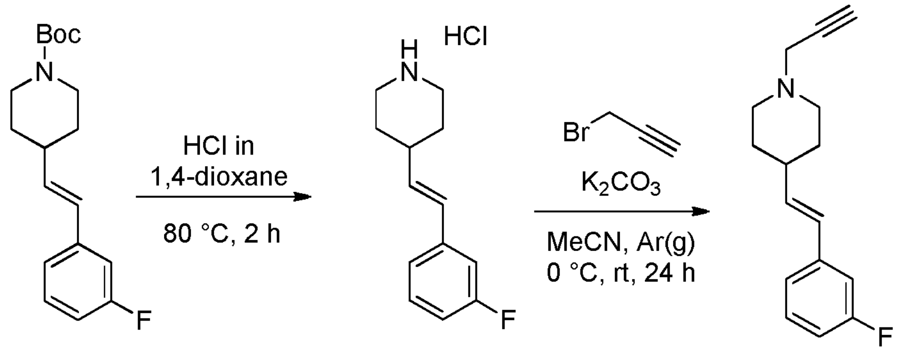


Synthesized from *tert*-butyl (*E*)-4-(3-fluorostyryl)piperidine-1-carboxylate (300 mg, 0.982 mmol, 1.0 ekviv.) via general procedures D and E. The compound was purified by flash column chromatography using EtOAc/n-hex = 1/2 (v/v) as eluent. Yield: 38% (91 mg); $R_f$ = 0.27 (EtOAc/n-hex = 1/2, v/v); pale yellow transparent oil; $^1$H NMR (400 MHz, $CDCl_3$): δ 1.57 (dt, *J* = 12.0, 3.9 Hz, 1H), 1.60 (dt, *J* = 11.9, 3.9 Hz, 1H), 1.78-1.85 (m, 2H), 2.09-2.19 (m, 1H), 2.27 (t, *J* = 2.5 Hz, 1H), 2.31 (dt, *J* = 11.7, 2.5 Hz, 2H), 2.92-2.98 (m, 2H), 3.35 (d, *J* = 2.5 Hz, 2H), 6.17 (dd, *J* = 16.0, 6.9 Hz, 1H), 6.34 (d, *J* = 16.0 Hz, 1H), 6.88 (ddt, *J* = 8.4, 2.6, 0.9 Hz, 1H), 7.02-7.06 (m, 1H), 7.07-7.11 (m, 1H), 7.21-7.27 (m, 1H); $^{13}$C NMR (100 MHz, $CDCl_3$): δ 31.71, 38.64, 47.14, 52.09, 73.32, 78.63, 112.39 (d, $J_{C,F}$ = 22.0 Hz), 113.73 (d, $J_{C,F}$ = 21.3 Hz), 121.87 (d, $J_{C,F}$ = 2.9 Hz), 127.33 (d, $J_{C,F}$ = 2.9 Hz), 129.85 (d, $J_{C,F}$ = 8.7 Hz), 136.11, 139.95 (d, $J_{C,F}$ = 7.3 Hz), 163.07 (d, $J_{C,F}$ = 244.9 Hz); HRMS (ESI+): *m/z* calcd for $C_{16}H_{19}FN$ $[M+H]^+$ 244.1502; found 244.1504; HPLC purity, 99.6%.

REFERENCE EXAMPLE 3

**Synthesis of 4-(3-fluorophenethyl)-1-(prop-2-yn-1-yl)piperidine**

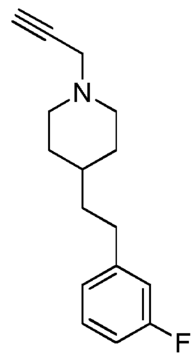

*Step 1:* ***Tert*-butyl 4-(3-fluorophenethyl)piperidine-1-carboxylate**

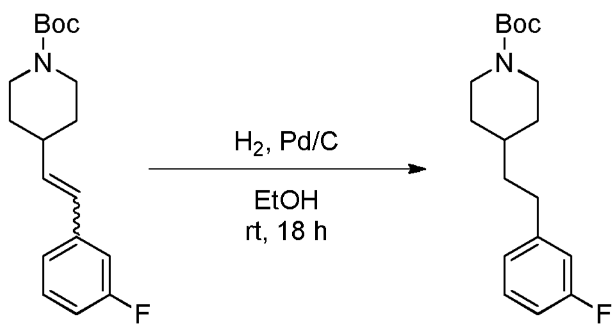

Synthesized from *tert*-butyl (*E*/*Z*)-4-(3-fluorostyryl)piperidine-1-carboxylate (0.452 g, 1.48 mmol, 1.0 equiv.) via general procedure C. The product was used without further purification. Yield: 95% (0.432 g); $R_f$ = 0.65 (EtOAc/n-hex = 1/2, v/v); pale yellow transparent oil; $^1$H NMR (400 MHz, $CDCl_3$): δ 1.11 (dt, *J* = 12.3, 4.1 Hz, 1H), 1.14 (dt, *J* = 12.5, 4.2 Hz, 1H), 1.35-1.44 (m, 1H), 1.45 (s, 9H), 1.50-1.59 (m, 2H), 1.65-1.72 (m, 2H), 2.59-2.72 (m, 4H), 4.08 (bs, 2H), 6.84-6.89 (m, 2H), 6.91-6.95 (m, 1H), 7.22 (ddd, *J* = 8.9, 7.6, 6.1 Hz, 1H); MS (ESI+): *m*/*z* $[M+Na]^+$ 330.18; found 330.42.

*Step 2:* **4-(3-Fluorophenethyl)-1-(prop-2-yn-1-yl)piperidine**

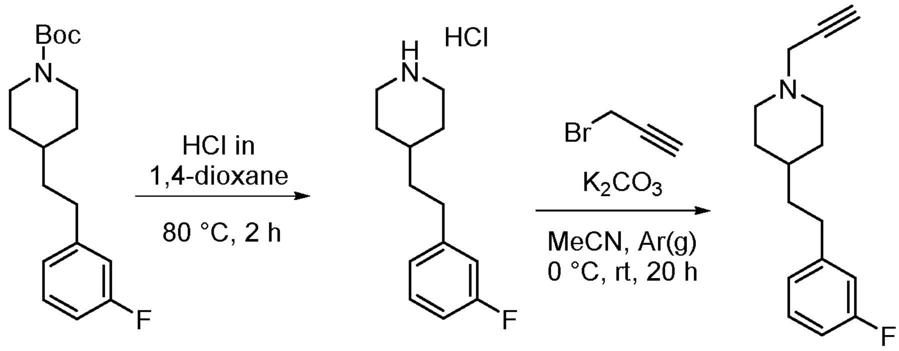


Synthesized from *tert*-butyl 4-(3-fluorophenethyl)piperidine-1-carboxylate (0.32 g, 1.04 mmol, 1.0 equiv.) via general procedures D and E. The compound was purified by flash column chromatography using EtOAc/n-hex = 1/2 (v/v) as eluent. Yield: 18% (46 mg); $R_f$ = 0.18 (EtOAc/n-hex = 1/2, v/v); yellow transparent oil; $^1$H NMR (400 MHz, $CDCl_3$): δ 1.23-1.37 (m, 3H), 1.53-1.59 (m, 2H), 1.72-1.78 (m, 2H), 2.14-2.21 (m, 2H), 2.24 (t, *J* = 2.4 Hz, 1H), 2.59-2.64 (m, 2H), 2.85-2.90 (m, 2H), 3.30 (d, *J* = 2.4 Hz, 2H), 6.83-6.89 (m, 2H), 6.92-6.95 (m, 1H), 7.19-7.25 (m, 1H); $^{13}$C NMR (100 MHz, $CDCl_3$): *δ* 32.09, 32.77, 34.64, 37.89, 47.14, 52.44, 72.92, 79.04, 112.46 (d, $J_{C,F}$ = 21.9 Hz), 115.04 (d, $J_{C,F}$ = 20.5 Hz), 123.90 (d, $J_{C,F}$ = 2.2 Hz), 129.61 (d, $J_{C,F}$ = 8.7 Hz), 145.19 (d, $J_{C,F}$ = 7.2 Hz), 162.84 (d, $J_{C,F}$ = 245.0 Hz); HRMS (ESI+): *m*/*z* calcd for $C_{16}H_{21}FN$ $[M+H]^+$ 246.1658; found 246.1659; HPLC purity, 97.6%.

EXAMPLE 4

**Synthesis of (*E*)-4-(2-fluorostyryl)-1-(prop-2-yn-1-yl)piperidine**

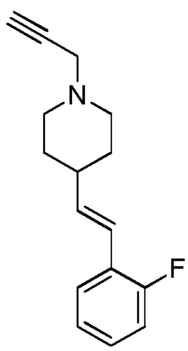

*Step 1:* ***Tert*-butyl (*E*)-4-(2-fluorostyryl)piperidine-1-carboxylate**

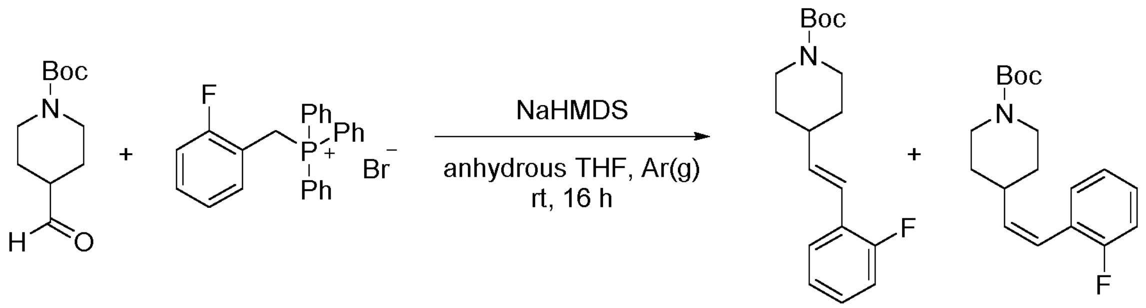


Synthesized from *tert*-butyl 4-formylpiperidine-1-carboxylate (1.50 g, 7.04 mmol, 1.0 equiv.) and (2-fluorobenzyl)triphenylphosphonium bromide (3.49 g, 7.74 mmol, 1.1 equiv., prepared via general procedure A) via general procedure B. The compound was purified by flash column chromatography using petroleum ether/$Et_2O$ = 10/1 (v/v) as eluent. Mass: 194 mg; $R_f$ = 0.14 (petroleum ether/$Et_2O$ = 10/1, v/v); colorless oil; $^1$H NMR (400 MHz, $CDCl_3$): δ 1.36 (dt, *J* = 12.3, 4.2 Hz, 1H), 1.39 (dt, *J* = 12.3, 4.2 Hz, 1H), 1.46 (s, 9H), 1.70-1.77 (m, 2H), 2.23-2.33 (m, 1H), 2.76 (t, *J* = 11.0 Hz, 2H), 4.12 (bs, 2H), 6.20 (dd, *J* = 16.1, 7.0 Hz, 1H), 6.53 (d, *J* = 16.1 Hz, 1H), 6.99 (ddd, *J* = 10.8, 8.1, 1.2 Hz, 1H), 7.05 (dt, *J* = 7.6, 1.2 Hz, 1H), 7.12-7.18 (m, 1H), 7.41 (dt, *J* = 7.7, 1.8 Hz, 1H); MS (ESI+): *m/z* $[M+Na]^+$ 328.17; found 328.42.

*Step 2:* **(*E*)-4-(2-Fluorostyryl)-1-(prop-2-yn-1-yl)piperidine**

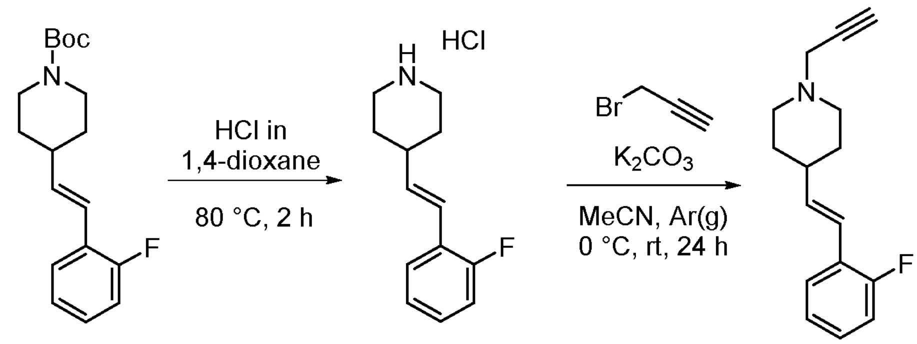


Synthesized from *tert*-butyl (*E*)-4-(2-fluorostyryl)piperidine-1-carboxylate (180 mg, 0.589 mmol, 1.0 ekviv.) via general procedures D and E. The compound was purified by flash column chromatography using EtOAc/n-hex = 1/2 (v/v) as eluent. Yield: 58% (83 mg); $R_f$ = 0.20 (EtOAc/n-hex = 1/2, v/v); pale yellow transparent oil; $^1$H NMR (400 MHz, $CDCl_3$): δ 1.56 (dt, *J* = 12.0, 3.9 Hz, 1H), 1.59 (dt, *J* = 12.0, 3.9 Hz, 1H), 1.80-1.86 (m, 2H), 2.12-2.22 (m, 1H), 2.26 (t, *J* = 2.4 Hz, 1H), 2.28 (dt, *J* = 11.7, 2.5 Hz, 2H), 2.91-2.96 (m, 2H), 3.33 (d, *J* = 2.4 Hz, 2H), 6.23 (dd, *J* = 16.1, 7.1 Hz, 1H), 6.55 (d, *J* = 16.1 Hz, 1H), 7.00 (ddd, *J* = 10.8, 8.1, 1.2 Hz, 1H), 7.07 (dt, *J* = 7.4, 1.0 Hz, 1H), 7.14-7.19 (m, 1H), 7.44 (dt, *J* = 7.7, 1.7 Hz, 1H); $^{13}$C NMR (100 MHz, $CDCl_3$): δ 31.93, 39.21, 47.24, 52.21, 72.97, 79.06, 115.58 (d, $J_{C,F}$ = 22.3 Hz), 120.60 (d, $J_{C,F}$ = 3.8 Hz), 123.96 (d, $J_{C,F}$ = 3.6 Hz), 125.32 (d, $J_{C,F}$ = 12.3 Hz), 126.88 (d, $J_{C,F}$ = 12.3 Hz), 128.15 (d, $J_{C,F}$ = 8.5 Hz), 137.41 (d, $J_{C,F}$ = 4.2 Hz), 159.95 (d, $J_{C,F}$ = 248.4 Hz); HRMS (ESI+): *m/z* calcd for $C_{16}H_{19}FN$ $[M+H]^+$ 244.1502; found 244.1500; HPLC purity, 99.5%.

REFERENCE EXAMPLE 4

**Synthesis of 4-(2-fluorophenethyl)-1-(prop-2-yn-1-yl)piperidine**

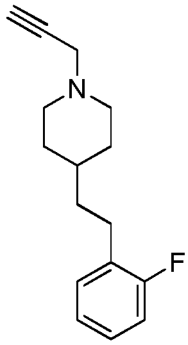

*Step 1:* ***Tert*-butyl 4-(2-fluorophenethyl)piperidine-1-carboxylate**

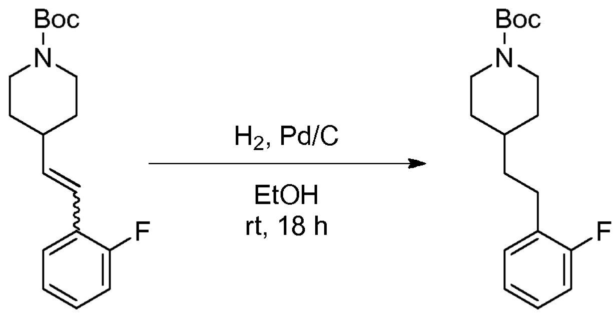


Synthesized from *tert*-butyl (*E*/*Z*)-4-(2-fluorostyryl)piperidine-1-carboxylate (0.252 g, 0.83 mmol, 1.0 equiv.) via general procedure C. The product was used without further purification. Yield: 95% (0.242 g); $R_f$ = 0.70 (EtOAc/n-hex = 1/2, v/v); pale yellow transparent oil; $^1$H NMR (400 MHz, $CDCl_3$): δ 1.12 (dt, *J* = 12.4, 4.1 Hz, 1H), 1.15 (dt, *J* = 12.4, 4.2 Hz, 1H), 1.37-1.44 (m, 1H), 1.46 (s, 9H), 1.52-1.59 (m, 2H), 1.69-1.76 (m, 2H), 2.58-2.72 (m, 4H), 4.08 (bs, 2H), 6.97-7.02 (m, 1H), 7.03-7.04 (m, 1H), 7.13-7.19 (m, 2H); MS (ESI+): *m*/*z* $[M+Na]^+$ 330.18; found 330.41.

*Step 2:* **4-(2-Fluorophenethyl)-1-(prop-2-yn-1-yl)piperidine**

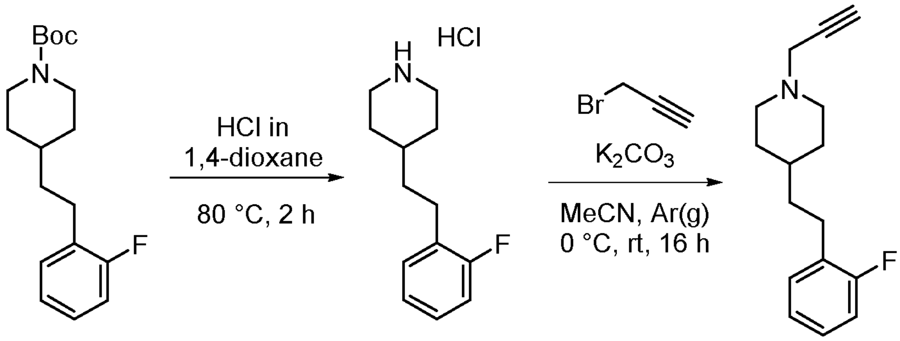


Synthesized from *tert*-butyl 4-(2-fluorophenethyl)piperidine-1-carboxylate (0.22 g, 0.716 mmol, 1.0 equiv.) via general procedures D and E. The compound was purified by flash column chromatography using EtOAc/n-hex = 1/2 (v/v) as eluent. Yield: 8% (14 mg); $R_f$ = 0.21 (EtOAc/n-hex = 1/1, v/v); yellow transparent oil; $^1$H NMR (400 MHz, $CDCl_3$): δ 1.23-1.37 (m, 3H), 1.53-1.59 (m, 2H), 1.74-1.80 (m, 2H), 2.14-2.21 (m, 2H), 2.23 (t, *J* = 2.4 Hz, 1H), 2.63-2.68 (m, 2H), 2.86-2.91 (m, 2H), 3.30 (d, *J* = 2.4 Hz, 2H), 6.97-7.02 (m, 1H), 7.03-7.07 (m, 1H), 7.12-7.20 (m, 2H); $^{13}$C NMR (100 MHz, $CDCl_3$): δ 26.16, 32.12, 34.85, 36.88, 47.19, 52.52, 72.89, 79.15, 115.15 (d, $J_{C,F}$ = 22.5 Hz), 123.88 (d, $J_{C,F}$ = 3.4 Hz), 127.32 (d, $J_{C,F}$ = 8.0 Hz), 129.41 (d, $J_{C,F}$ = 16.1 Hz), 130.41 (d, $J_{C,F}$ = 5.3 Hz), 161.09 (d, $J_{C,F}$ = 244.5 Hz); HRMS (ESI+): *m*/*z* calcd for $C_{16}H_{21}FN$ $[M+H]^+$ 246.1658; found 246.1657; HPLC purity, 95.1%.

EXAMPLE 5

**Synthesis of (*E*)-1-(Prop-2-yn-1-yl)-4-styrylpiperidine**

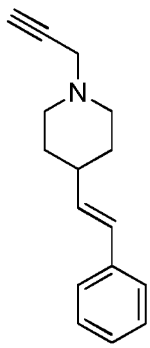

*Step 1:* ***Terc*-butyl (*E*)-4-styrylpiperidine-1-carboxylate**

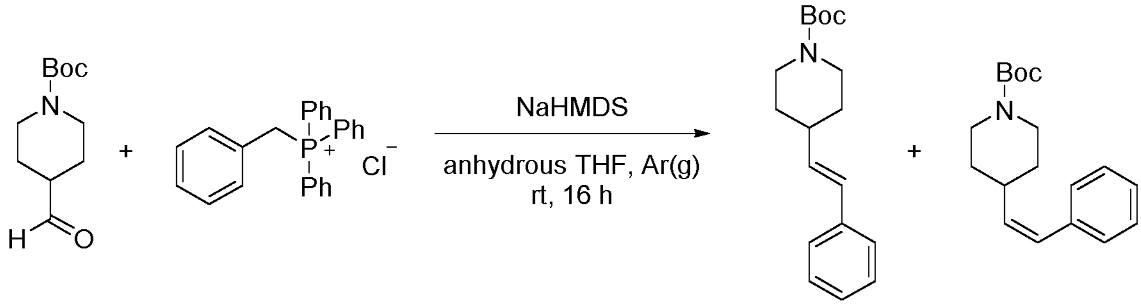


Synthesized from *tert*-butyl 4-formylpiperidine-1-carboxylate (1.50 g, 7.04 mmol, 1.0 equiv.) and benzyltriphenylphosphonium bromide (3.49 g, 7.74 mmol, 1.1 equiv., prepared via general procedure A) via general procedure B. The compound was purified by flash column chromatography using petroleum ether/$Et_2O$ = 9/1 (v/v) as eluent. Mass: 285 mg; $R_f$ = 0.28 (petroleum ether/$Et_2O$ = 9/1, v/v); colorless oil; $^1H$ NMR (400 MHz, $CDCl_3$): $\delta$ 1.36 (ddd, *J* = 16.6, 12.6, 4.5 Hz, 2H), 1.47 (s, 9H), 1.73-1.76 (m, 2H), 2.22-2.31 (m, 1H), 2.77 (t, *J* = 11.5 Hz, 2H), 4.12 (bs, 2H), 6.13 (dd, *J* = 17.0, 6.9 Hz, 1H), 6.38 (d, *J* = 16.0 Hz, 1H), 7.17-7.21 (m, 1H), 7.27-7.35 (m, 4H); MS (ESI+): *m*/*z* $[M+Na]^+$ 310.18; found 310.12.

*Step 2:* **(*E*)-1-(Prop-2-yn-1-yl)-4-styrylpiperidine**

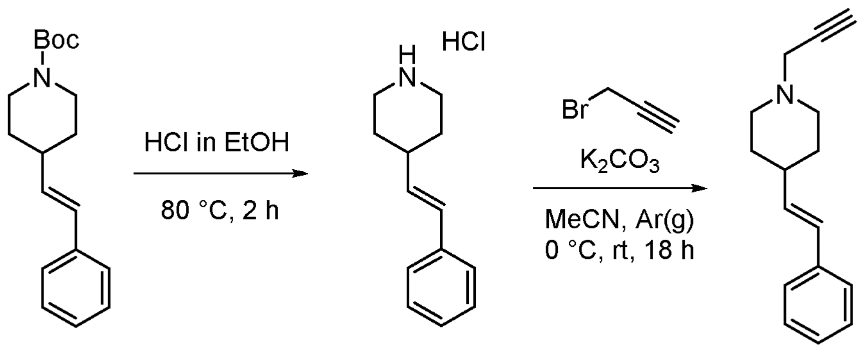

Synthesized from *tert*-butyl (*E*)-4-styrylpiperidine-1-carboxylate (196 mg, 0.682 mmol, 1.0 ekviv.) via general procedures D and E. The compound was purified by flash column chromatography using EtOAc/n-hex = 1/1 (v/v) as eluent. Yield: 89% (137 mg); $R_f$ = 0.41 (EtOAc/n-hex = 1/1, v/v); white crystals, mp 44-46 °C; $^1$H NMR (400 MHz, $CDCl_3$): δ 1.56 (dd, *J* = 11.9, 3.9 Hz, 1H), 1.59 (dd, *J* = 11.9, 3.9 Hz, 1H), 1.79-1.84 (m, 2H), 2.09-2.18 (m, 1H), 2.24-2.31 (m, 3H), 2.94 (td, *J* = 11.1, 2.4 Hz, 2H), 3.33 (d, *J* = 2.5 Hz, 2H), 6.18 (dd, *J* = 16.0, 7.0 Hz, 1H), 6.39 (d, *J* = 16.5 Hz, 1H), 7.18-7.22 (m, 1H), 7.27-7.32 (m, 2H), 7.35-7.37 (m, 2H); $^{13}$C NMR (100 MHz, $CDCl_3$): δ 31.90, 38.70, 47.13, 52.13, 72.90, 78.98, 125.87, 126.85, 128.10, 128.36, 134.77, 137.48; HRMS (ESI+): *m*/*z* calcd for $C_{16}H_{20}N$ $[M+H]^+$ 226.1596; found 226.1594; HPLC purity, 99.2%.

REFERENCE EXAMPLE 5

**Synthesis of 4-phenethyl-1-(prop-2-yn-1-yl)piperidine**

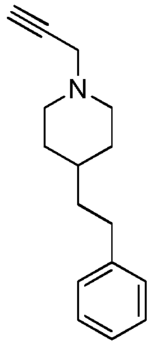

*Step 1:* ***Tert*-butyl 4-phenethylpiperidine-1-carboxylate**

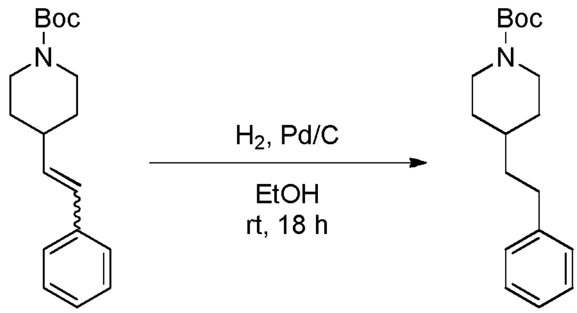


Synthesized from *tert*-butyl 4-phenethylpiperidine-1-carboxylate (1.139 g, 4.84 mmol, 1.0 equiv.) via general procedure C. The product was used without further purification. Yield: quantitative (1.39 g); $R_f$ = 0.44 (petroleum ether/$Et_2O$ = 3/1, v/v); colorless oil; $^1$H NMR (400 MHz, $CDCl_3$): δ 1.21 (ddd, *J* = 16.5, 12.5, 4.3 Hz, 2H), 1.35-1.43 (m, 1H), 1.45 (s, 9H), 1.53-1.59 (m, 2H), 1.68-1.71 (m, 2H), 2.60-2.69 (m, 4H), 4.07 (bs, 2H), 7.15-7.19 (m, 3H), 7.24-7.29 (m, 2H); MS (ESI+): *m*/*z* $[M+Na]^+$ 312.19; found 311.91.

*Step 2:* **4-Phenethyl-1-(prop-2-yn-1-yl)piperidine**

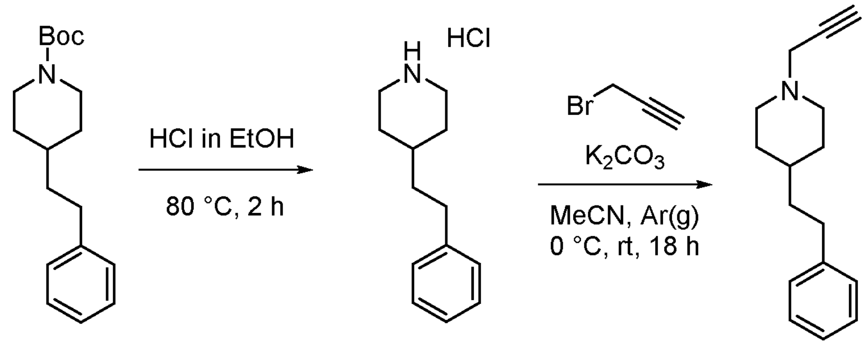


Synthesized from *tert*-butyl 4-phenethylpiperidine-1-carboxylate (1.04 g, 3.593 mmol, 1.0 equiv.) via general procedures D and E. The compound was purified by flash column chromatography using EtOAc/n-hex = 1/2 (v/v) as eluent. Yield: 56% (202 mg); $R_f$ = 0.36 (EtOAc/n-hex = 1/1, v/v); yellow transparent oil; $^1H$ NMR (400 MHz, $CDCl_3$): $\delta$ 1.20-1.35 (m, 3H), 1.52-1.58 (m, 2H), 1.73-1.76 (m, 2H), 2.12-2.18 (m, 2H), 2.21 (t, $J$ = 2.5 Hz, 1H), 2.58-2.63 (m, 2H), 2.84-2.87 (m, 2H), 3.26 (d, $J$ = 2.5 Hz, 2H), 7.13-7.17 (m, 3H), 7.23-7.28 (m, 2H); $^{13}C$ NMR (100 MHz, $CDCl_3$): $\delta$ 32.04, 32.89, 34.57, 38.14, 47.03, 52.34, 72.74, 79.02, 125.45, 128.10 (4×C), 142.44; HRMS (ESI+): $m/z$ calcd for $C_{16}H_{22}N$ $[M+H]^+$ 228.1752; found 228.1755; HPLC purity, 99.0%.

EXAMPLE 6

**Synthesis of (*E*)-4-(4-chlorostyryl)-1-(prop-2-yn-1-yl)piperidine**

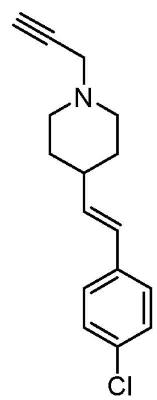

*Step 1:* ***Tert*-butyl (*E*)-4-(4-chlorostyryl)piperidine-1-carboxylate**

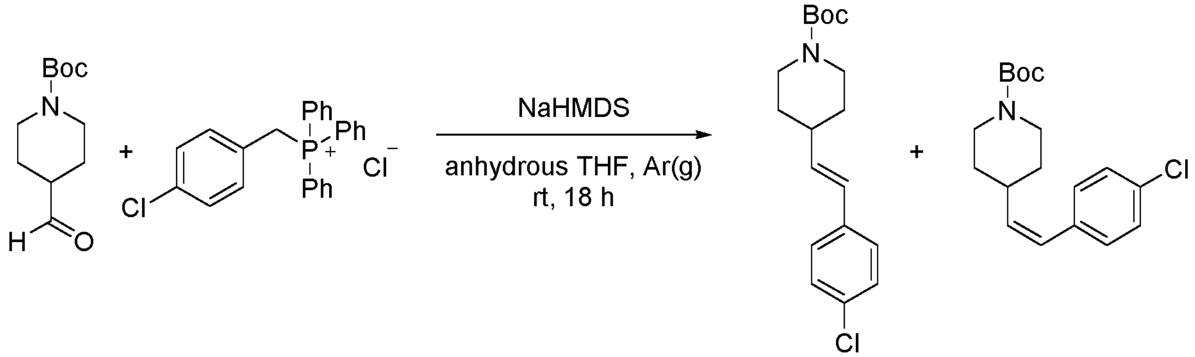

Synthesized from *tert*-butyl 4-formylpiperidine-1-carboxylate (2.00 g, 9.38 mmol, 1.0 equiv.) and 4-chlorobenzyltriphenylphosphonium chloride (4.37 g, 10.32 mmol, 1.1 equiv., prepared via general procedure A) via general procedure B. The compound was purified by flash column chromatography using petroleum ether/$Et_2O$ = 10/1 (v/v) as eluent. Mass: 221 mg; $R_f$ = 0.10 (petroleum ether/$Et_2O$ = 10/1, v/v); white solid, mp 60-62 °C; $^1H$ NMR (400 MHz, $CDCl_3$): δ 1.35 (dd, *J* = 12.4, 4.1 Hz, 1H), 1.38 (dd, *J* = 12.2, 4.1 Hz, 1H), 1.46 (s, 9H), 1.71-1.77 (m, 2H), 2.22-2.31 (m, 1H), 2.77 (t, *J* = 11.5 Hz, 2H), 4.14 (bs, 2H), 6.11 (dd, *J* = 16.0, 6.9 Hz, 1H), 6.32 (dd, *J* = 16.0, 1.2 Hz, 1H), 7.52 (s, 4H); MS (ESI+): *m*/*z* $[M+Na]^+$ 344.14; found 344.48.

*Step 2:* **(*E*)-4-(4-Chlorostyryl)-1-(prop-2-yn-1-yl)piperidine**

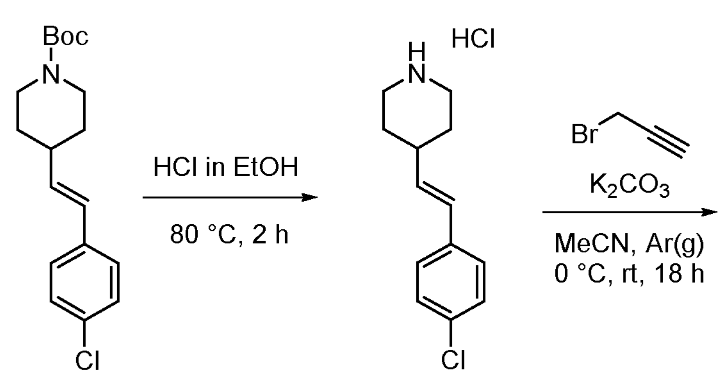


Synthesized from *tert*-butyl (*E*)-4-(4-chlorostyryl)piperidine-1-carboxylate (165 mg, 0.513 mmol, 1.0 ekviv.) via general procedures D and E. The compound was purified by flash column chromatography using EtOAc/n-hex = 1/2 (v/v) as eluent. Yield: 45% (87 mg); $R_f$ = 0.30 (EtOAc/n-hex = 1/1, v/v); white crystals, mp 83-85 °C; $^1H$ NMR (400 MHz, $CDCl_3$): δ 1.49-1.59 (m, 2H), 1.76-1.81 (m, 2H), 2.03-2.16 (m, 1H), 2.23-2.29 (m, 2H), 2.25 (t, *J* = 2.4 Hz, 1H), 2.92 (td, *J* = 11.1, 2.4 Hz, 2H), 3.31 (d, *J* = 2.5 Hz, 2H), 6.13 (dd, *J* = 16.0, 7.0 Hz, 1H), 6.31 (dd, *J* = 16.0, 1.1 Hz, 1H), 7.22-7.27 (m, 4H); $^{13}C$ NMR (100 MHz, $CDCl_3$): δ 31.88, 38.77, 47.18, 52.15, 72.94, 79.00, 127.00, 127.14, 128.52, 132.40, 135.55, 136.05; HRMS (ESI+): *m*/*z* calcd for $C_{16}H_{19}ClN$ $[M+H]^+$ 260.1206; found 260.1207; HPLC purity, 99.6%.

REFERENCE EXAMPLE 6

**Synthesis of 4-(4-chlorophenethyl)-1-(prop-2-yn-1-yl)piperidine**

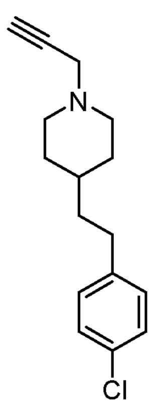

*Step 1:* ***Tert*-butyl 4-(4-chlorophenethyl)piperidine-1-carboxylate**

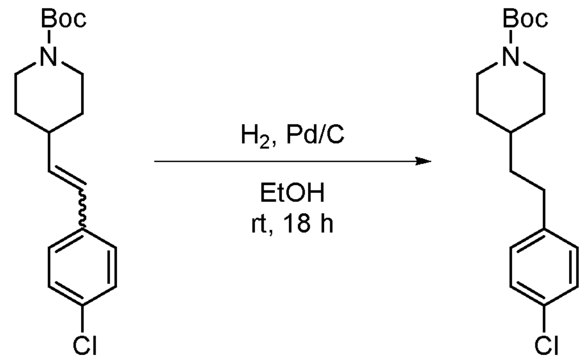


Synthesized from *tert*-butyl (*E*/*Z*)-4-(4-chlorostyryl)piperidine-1-carboxylate (0.850 g, 2.64 mmol, 1.0 equiv.) via general procedure C. The product was used without further purification. Yield: 86% (0.736 g); $R_f$ = 0.37 (petroleum ether/$Et_2O$ = 3/1, v/v); colorless oil; $^1$H NMR (400 MHz, $CDCl_3$): δ 1.08 (dd, *J* = 12.3, 4.2 Hz, 1H), 1.11 (dd, *J* = 12.8, 3.9 Hz, 1H), 1.33-1.40 (m, 1H), 1.44 (s, 9H), 1.48-1.53 (m, 2H), 1.63-1.68 (m, 2H), 2.55-2.59 (m, 2H), 2.64 (t, *J* = 11.6 Hz, 2H), 4.07 (bs, 2H), 7.05-7.08 (m, 2H), 7.18-7.22 (m, 2H); MS (ESI+): *m*/*z* $[M+Na]^+$ 346.15; found 346.55.

*Step 2:* **4-(4-Chlorophenethyl)-1-(prop-2-yn-1-yl)piperidine**

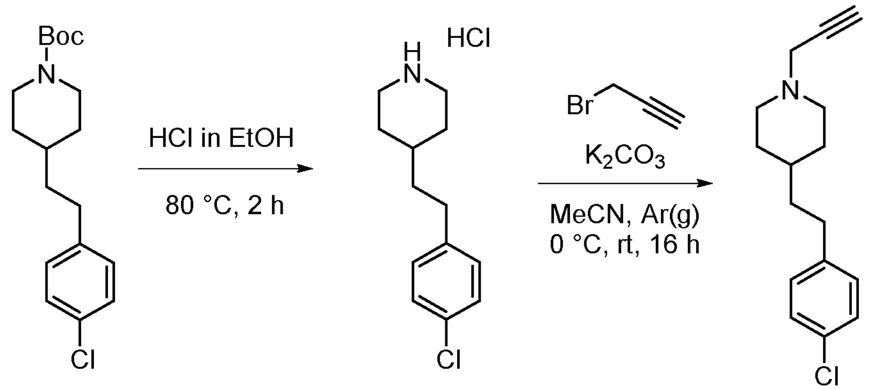


Synthesized from *tert*-butyl 4-(4-chlorophenethyl)piperidine-1-carboxylate (0.195 g, 0.602 mmol, 1.0 equiv.) via general procedures D and E. The compound was purified by flash column chromatography using EtOAc/n-hex = 1/2 (v/v) as eluent. Yield: 62% (85 mg); $R_f$ = 0.27 (EtOAc/n-hex = 1/1, v/v); white solid, mp 37-39 °C; $^1$H NMR (400 MHz, $CDCl_3$): δ 1.18-1.35 (m, 3H), 1.49-1.55 (m, 2H), 1.72-1.75 (m, 2H), 2.12-2.18 (m, 2H), 2.22 (t, *J* = 2.5 Hz, 1H), 2.55-2.59 (m, 2H), 2.84-2.88 (m, 2H), 3.27 (d, *J* = 2.5 Hz, 2H), 7.06-7.10 (m, 2H), 7.20-7.24 (m, 2H); $^{13}$C NMR (100 MHz, $CDCl_3$): δ 32.11, 32.32, 34.59, 38.08, 47.13, 52.42, 72.82, 79.10, 128.29, 129.56, 131.21, 140.98; HRMS (ESI+): *m*/*z* calcd for $C_{16}H_{21}ClN$ $[M+H]^+$ 262.1363; found 262.1362; HPLC purity, 100%.

EXAMPLE 7

**Synthesis of (*E*)-4-(4-bromostyryl)-1-(prop-2-yn-1-yl)piperidine**

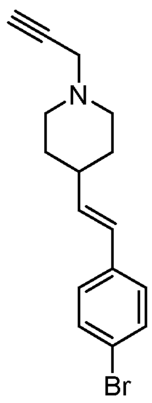

*Step 1:* ***Tert*-butyl (*E*)-4-(4-bromostyryl)piperidine-1-carboxylate**

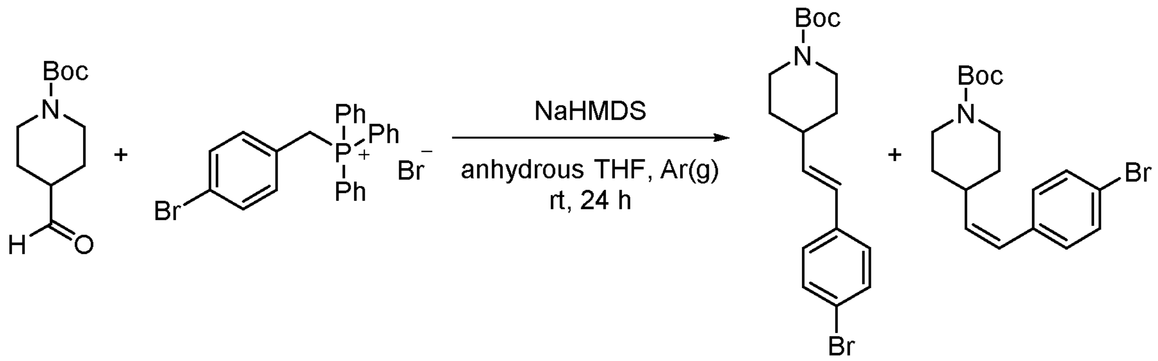


Synthesized from *tert*-butyl 4-formylpiperidine-1-carboxylate (2.00 g, 9.38 mmol, 1.0 equiv.) and 4-bromobenzyltriphenylphosphonium bromide (5.28 g, 10.31 mmol, 1.1 equiv., prepared via general procedure A) via general procedure B. The compound was purified by flash column chromatography using EtOAc/n-hex = 1/2 (v/v) as eluent. Mass: 325 mg; $R_f$ = 0.40 (EtOAc/n-hex = 1/2, v/v); white amorphous solid, mp 65-67 °C; $^1H$ NMR (400 MHz, $CDCl_3$): δ 1.36 (dt, *J* = 12.4, 4.2 Hz, 1H), 1.39 (dt, *J* = 12.4, 4.5 Hz, 1H), 1.47 (s, 9H), 1.71-1.78 (m, 2H), 2.22-2.32 (m, 1H), 2.74-2.81 (m, 2H), 4.12 (bs, 2H), 6.13 (dd, *J* = 16.0, 6.8 Hz, 1H), 6.32 (d, *J* = 16.0 Hz, 1H); 7.19-7.22 (m, 2H), 7.39-7.43 (m, 2H).

*Step 2:* **(*E*)-4-(4-Bromostyryl)-1-(prop-2-yn-1-yl)piperidine**

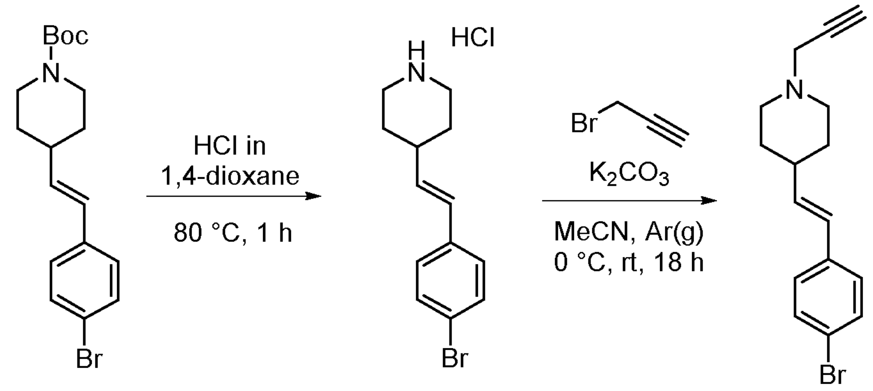


Synthesized from *tert*-butyl (*E*)-4-(4-bromostyryl)piperidine-1-carboxylate (360 mg, 0.98 mmol, 1.0 ekviv.) via general procedures D and E. The compound was purified by flash column chromatography using EtOAc/n-hex = 1/2

(v/v) as eluent. Yield: 34% (102 mg); $R_f$ = 0.34 (EtOAc/n-hex = 1/2, v/v); white crystals, mp 103-106 °C; $^1$H NMR (400 MHz, $CDCl_3$): δ 1.54 (dt, $J$ = 12.2, 3.4 Hz, 1H), 1.57 (dt, $J$ = 12.3, 3.6 Hz, 1H), 1.77-1.83 (m, 2H), 2.08-2.17 (m, 1H), 2.19-2.30 (m, 3H), 2.91-2.96 (m, 2H), 3.32 (d, $J$ = 2.2 Hz, 2H), 6.15 (dd, $J$ = 16.0, 6.9 Hz, 1H), 6.31 (d, $J$ = 16.2 Hz, 1H), 7.21 (d, $J$ = 8.4 Hz, 2H), 7.41 (d, $J$ = 8.4 Hz, 2H); $^{13}$C NMR (100 MHz, $CDCl_3$): δ 31.92, 38.85, 47.24, 52.21, 72.98, 79.04, 127.13, 127.56, 130.12, 131.53, 135.77, 136.57; HRMS (ESI+): *m/z* calcd for $C_{16}H_{19}NBr$ [M+H]$^+$ 304.0701; found 304.0706; HPLC purity, 100%.

REFERENCE EXAMPLE 7

**Synthesis of 4-(4-bromophenethyl)-1-(prop-2-yn-1-yl)piperidine**

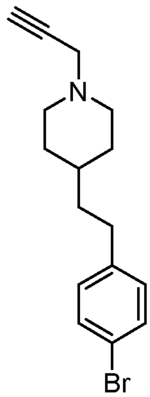

*Step 1:* ***Tert*-butyl 4-(4-bromophenethyl)piperidine-1-carboxylate**

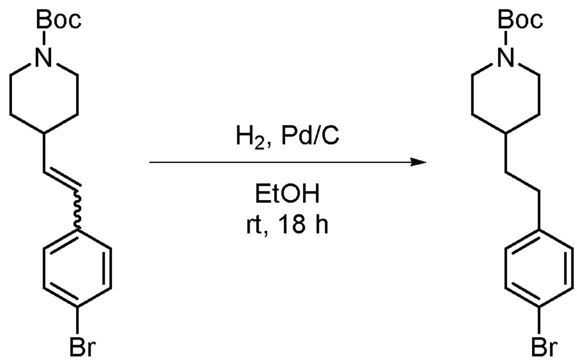


Synthesized from *tert*-butyl (*E*/*Z*)-4-(4-bromostyryl)piperidine-1-carboxylate (0.200 g, 0.55 mmol, 1.0 equiv.) via general procedure C. The product was used without further purification. Mass: 0.20 g; $R_f$ = 0.62 (EtOAc/n-hex = 1/2, v/v); pale brown transparent oil; $^1$H NMR (400 MHz, $CDCl_3$): δ 1.07-1.19 (m, 2H), 1.36-1.48 (m, 1 H), 1.45 (s, 9H), 1.53-1.60 (m, 2H), 1.65-1.73 (m, 2H), 2.60-2.73 (m, 4H), 4.09 (bs, 2H), 7.15-7.19 (m, 2H), 7.25-7.29 (m, 2H).

*Step 2:* **4-(4-Bromophenethyl)-1-(prop-2-yn-1-yl)piperidine**

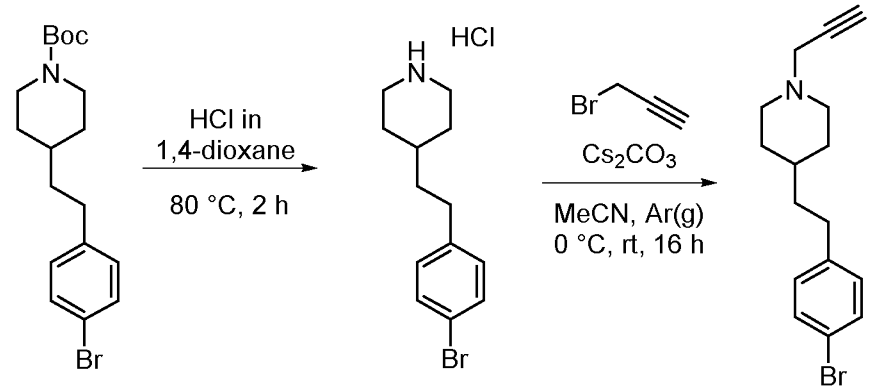


Synthesized from *tert*-butyl 4-(4-bromophenethyl)piperidine-1-carboxylate (0.200 g, 0.54 mmol, 1.0 equiv.) via general procedures D and E. The compound was purified by flash column chromatography using EtOAc/n-hex = 1/2 (v/v) as eluent. Yield: 32% (54 mg); $R_f$ = 0.34 (EtOAc/n-hex = 1/2, v/v); yellow amorphous solid, mp 139-140 °C; $^1$H NMR (400 MHz, $CDCl_3$): δ 1.26-1.37 (m, 3H), 1.55-1.60 (m, 2H), 1.76-1.79 (m, 2H), 2.14-2.21 (m, 2H), 2.23 (t, *J* = 2.4 Hz, 1H), 2.61-2.65 (m, 2H), 2.86-2.91 (m, 2H), 3.29 (d, *J* = 2.5 Hz, 2H), 7.16-7.20 (m, 3H), 7.28-7.30 (m, 1H); $^{13}$C NMR (100 MHz, $CDCl_3$): δ 32.20, 33.04, 34.73, 38.29, 47.19, 52.53, 72.82, 79.19, 125.61, 128.27, 128.28, 142.67; HPLC purity, 95.0%.

EXAMPLE 8

**Synthesis of (*E*)-4-(4-methylstyryl)-1-(prop-2-yn-1-yl)piperidine**

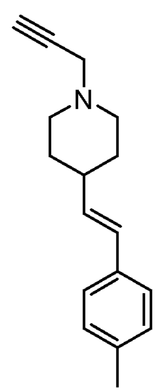

*Step 1:* ***Tert*-butyl (*E*)-4-(4-methylstyryl)piperidine-1-carboxylate**

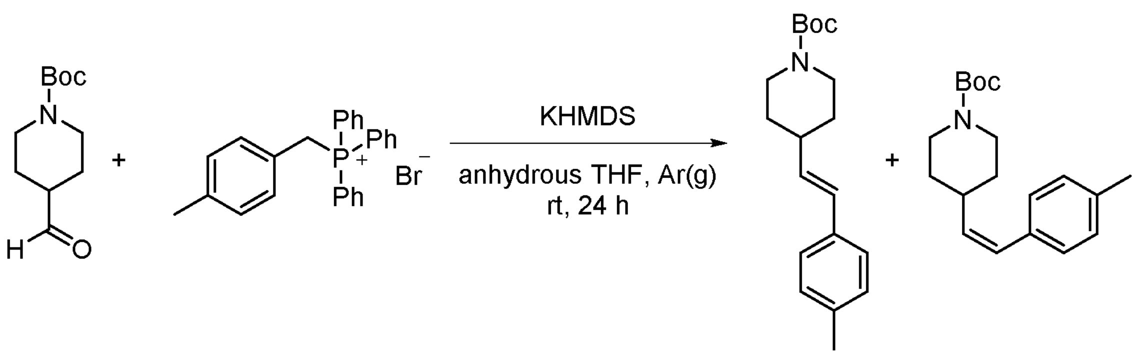

Synthesized from *tert*-butyl 4-formylpiperidine-1-carboxylate (1.49 g, 7.00 mmol, 1.0 equiv.) and 4-methylbenzyltriphenylphosphonium bromide (3.13 g, 7.00 mmol, 1.0 equiv., prepared via general procedure A) via general procedure B. The compound was purified by flash column chromatography using EtOAc/n-hex = 1/5 (v/v) as eluent. Mass: 155 mg; $R_f$ = 0.72 (EtOAc/n-hex = 1/5, v/v); colorless oil; $^1H$ NMR (400 MHz, $CDCl_3$): δ 1.36 (dt, *J* = 12.2, 4.2 Hz, 1H), 1.39 (dt, *J* = 12.4, 4.3 Hz, 1H), 1.47 (s, 9H), 1.71-1.78 (m, 2H), 2.22-2.31 (m, 1H), 2.32 (s, 3H), 2.77 (t, *J* = 12,6 Hz, 2H), 4.12 (bs, 2H), 6.09 (dd, *J* = 16.0, 6.9 Hz, 1H), 6.35 (d, *J* = 15.8 Hz, 1H), 7.09-7.12 (m, 2H), 7.22-7.25 (m, 2H); MS (ESI+): *m*/*z* $[M+H]^+$ 302.21; found 302.60.

*Step 2:* **(*E*)-4-(4-Methylstyryl)-1-(prop-2-yn-1-yl)piperidine**

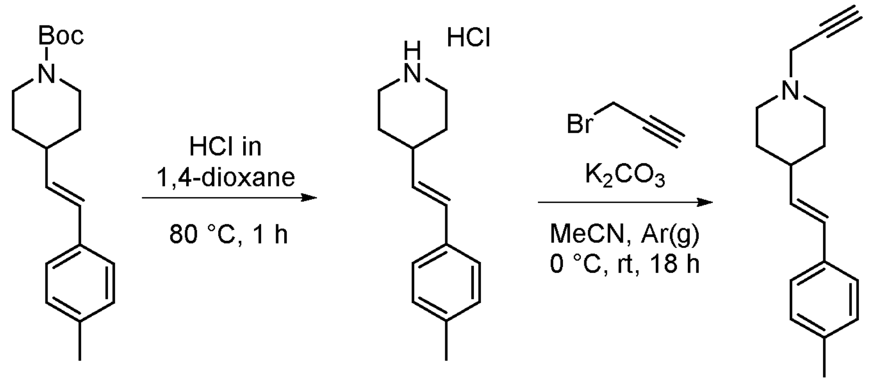


Synthesized from *tert*-butyl (*E*)-4-(4-methylstyryl)piperidine-1-carboxylate (420 mg, 1.39 mmol, 1.0 ekviv.) via general procedures D and E. The compound was purified by flash column chromatography using EtOAc/n-hex = 1/3 (v/v) as eluent. Yield: 36% (121 mg); $R_f$ = 0.25 (EtOAc/n-hex = 1/3, v/v); orange amorphous solid, mp 65-67 °C; $^1H$ NMR (400 MHz, $CDCl_3$): δ 1.65 (dt, *J* = 12.0, 3.9 Hz, 1H), 1.68 (dt, *J* = 12.0, 3.9 Hz, 1H), 1.88-1.95 (m, 2H), 2.18-2.28 (m, 1H), 2.35-2.41 (m, 3H), 2.43 (s, 3H), 3.02-3.07 (m, 2H), 3.43 (d, *J* = 2.5 Hz, 2H), 6.22 (dd, *J* = 16.0, 7.0 Hz, 1H), 6.46 (d, *J* = 15.9 Hz, 1H), 7.21 (d, *J* = 7.9 Hz, 2H), 7.36 (d, *J* = 8.3 Hz, 2H); $^{13}C$ NMR (100 MHz, $CDCl_3$): δ 21.09, 32.01, 38.77, 47.21, 52.25, 72.92, 79.07, 125.83, 127.93, 129.13, 133.84, 134.78, 136.62; HRMS (ESI+): *m*/*z* calcd for $C_{17}H_{22}N$ $[M+H]^+$ 240.1752; found 240.1756; HPLC purity, 97.7%.

REFERENCE EXAMPLE 8

**Synthesis of 4-(4-methylphenethyl)-1-(prop-2-yn-1-yl)piperidine**

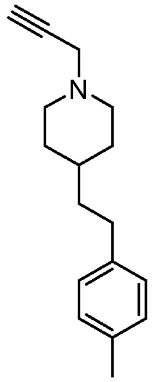

*Step 1:* ***Tert*-butyl 4-(4-methylphenethyl)piperidine-1-carboxylate**

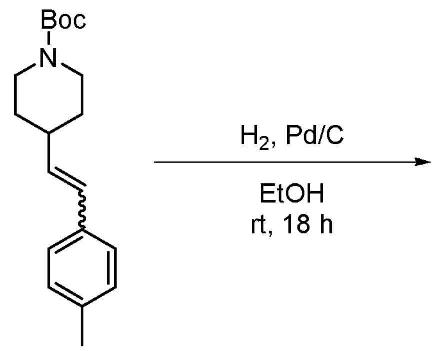


Synthesized from *tert*-butyl (*E*/*Z*)-4-(4-methylstyryl)piperidine-1-carboxylate (0.280 g, 0.93mmol, 1.0 equiv.) via general procedure C. The product was used without further purification. Mass: quantitative (200 mg); $R_f$ = 0.63 (EtOAc/n-hex = 1/2, v/v); yellow transparent oil; $^1$H NMR (400 MHz, $CDCl_3$): δ 1.11 (dt, *J* = 12.3, 4.1 Hz, 1H), 1.14 (dt, *J* = 12.4, 4.1 Hz, 1H), 1.36-1.44 (m, 1H), 1.45 (s, 9H), 1.51-1.57 (m, 2H), 1.65-1.73 (m, 2H), 2.32 (s, 3H), 2.56-2.61 (m, 2H), 2.63-2.70 (m, 2H), 4.07 (bs, 2H), 7.04-7.10 (m, 4H); MS (ESI+): *m*/*z* $[M+Na]^+$ 326.21; found 326.73.

*Step 2:* **4-(4-Methylphenethyl)-1-(prop-2-yn-1-yl)piperidine**

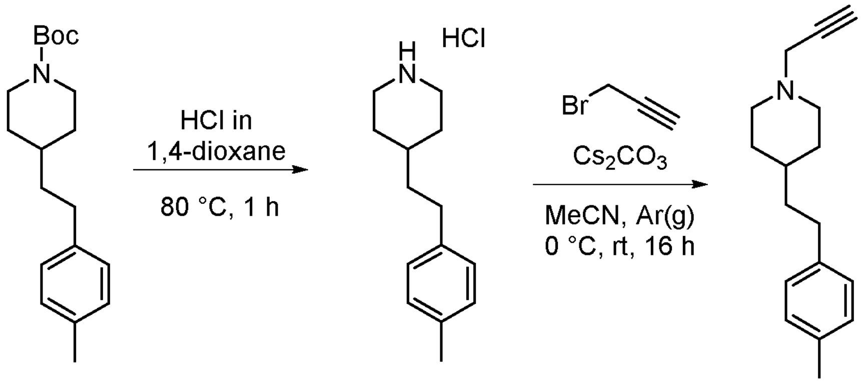


Synthesized from *tert*-butyl 4-(4-methylphenethyl)piperidine-1-carboxylate (0.280 g, 0.92 mmol, 1.0 equiv.) via general procedures D and E. The compound was purified by flash column chromatography using EtOAc/n-hex = 1/3 (v/v) as eluent. Yield: 36% (79 mg); $R_f$ = 0.26 (EtOAc/n-hex = 1/3, v/v); yellow amorphous solid; $^1$H NMR (400 MHz, $CDCl_3$): δ 1.21-1.36 (m, 3H), 1.52-1.57 (m, 2H), 1.74-1.79 (m, 2H), 2.13-2.20 (m, 2H), 2.23 (t, *J* = 2.4 Hz, 1H), 2.31 (s, 3H), 2.56-2.60 (m, 2H), 2.86-2.90 (m, 2H), 3.29 (d, *J* = 2.4 Hz, 2H), 7.05-7.10 (m, 4H); $^{13}$C NMR (100 MHz, $CDCl_3$): δ 20.97, 32.21, 32.57, 34.68, 38.43, 47.21, 52.55, 72.83, 79.06, 128.17, 128.97, 135.05, 139.58; HRMS (ESI+): *m*/*z* calcd for $C_{17}H_{24}N$ $[M+H]^+$ 242.1909; found 242.1904; HPLC purity, 97.4%.

EXAMPLE 9

**Synthesis of (*E*)-4-(4-cyclopropylstyryl)-1-(prop-2-yn-1-yl)piperidine**

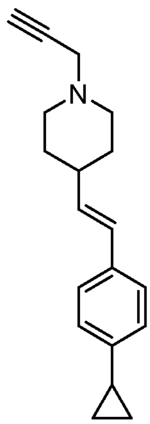

*Step 1:* ***Tert*-butyl (*E*)-4-(4-cyclopropylstyryl)piperidine-1-carboxylate**

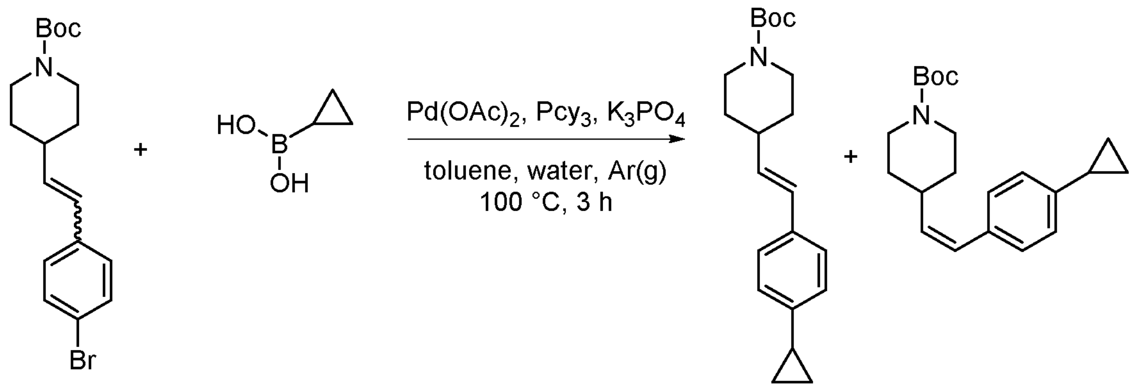


To the solution of *tert*-butyl (*E*/*Z*)-4-(4-bromostyryl)piperidine-1-carboxylate (0.733 g, 2.0 mmol, 1.0 equiv.), cyclopropylboronic acid (0.224 g, 2.60 mmol, 1.3 equiv.), $K_3PO_4$ (1.48 g, 7.00 mmol, 3.5 equiv.) and tricyclohexylphosphine (20% solution in toluene, 0.316 mL, 0.20 mmol, 0.1 equiv.) in toluene (10 mL) and water (0.4 mL), $Pd(OAc)_2$ (23 mg, 0.1 mmol, 0.05 equiv.) was added under argon atmosphere. The reaction mixture was stirred at 100 °C for 3 h, and then allowed to cool down to room temperature. Water (20 mL) was added to the mixture that was then transferred into a separating funnel, and extracted with EtOAc (2 × 50 mL). Combined organic layers were washed with saturated brine (20 mL), dried over anhydrous $Na_2SO_4$, and evaporated under reduced pressure. Crude product was purified by flash column chromatography using petroleum ether/$Et_2O$ = 10/1 (v/v) as eluent. Mass: 210 mg; $R_f$ = 0.22 (petroleum ether/$Et_2O$ = 10/1, v/v); colorless oil; $^1H$ NMR (400 MHz, $CDCl_3$): δ 0.66-0.70 (m, 2H), 0.92-0.97 (m, 2H), 1.36 (dt, *J* = 12.7, 4.4 Hz, 1H), 1.39 (dt, *J* = 12.3, 4.1 Hz, 1H), 1.48 (s, 9H), 1.70-1.78 (m, 2H), 1.83-1.90 (m, 1H), 2.21-2.31 (m, 1H), 2.78 (t, *J* = 11.6 Hz, 2H), 4.13 (bs, 2H), 6.08 (dd, *J* = 16.0, 6.9 Hz, 1H), 6.34 (d, *J* = 16.0 Hz, 1H), 6.98-7.02 (m, 2H), 7.22-7.25 (m, 2H).

*Step 2:* **(*E*)-4-(4-Cyclopropylstyryl)-1-(prop-2-yn-1-yl)piperidine**

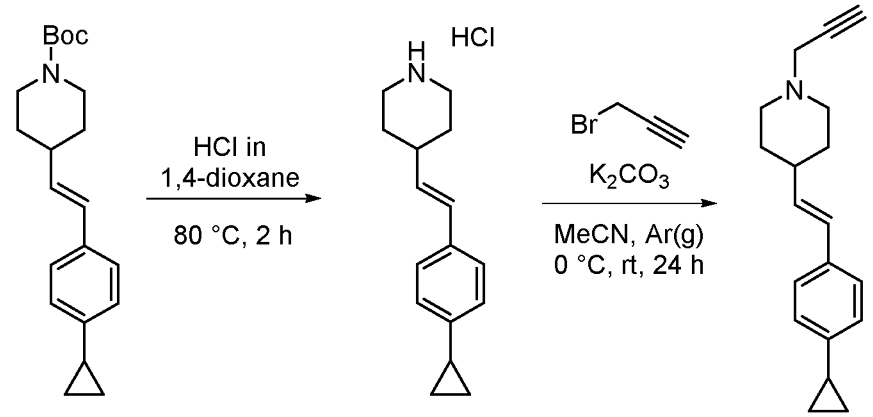


Synthesized from *tert*-butyl (*E*)-4-(4-cyclopropylstyryl)piperidine-1-carboxylate (200 mg, 0.611 mmol, 1.0 ekviv.) via general procedures D and E. The compound was purified by flash column chromatography using EtOAc/n-hex = 1/2 (v/v) as eluent. Yield: 40% (65 mg); $R_f$ = 0.28 (EtOAc/n-hex = 1/2, v/v); pale yellow crystals, mp 66-68 °C; $^1$H NMR (400 MHz, $CDCl_3$): $\delta$ 0.68-0.72 (m, 2H), 0.95-0.99 (m, 2H), 1.57 (dt, *J* = 12.0, 3.9 Hz, 1H), 1.60 (dt, *J* = 11.9, 3.9 Hz, 1H), 1.79-1.85 (m, 2H), 1.86-1.93 (m, 1H), 2.09-2.20 (m, 1H), 2.28 (t, *J* = 2.4 Hz, 1H), 2.30 (dt, *J* = 11.7, 2.5 Hz, 2H), 2.92-2.98 (m, 2H), 3.35 (d, *J* = 2.4 Hz, 2H), 6.13 (dd, *J* = 15.9, 7.0 Hz, 1H), 6.37 (d, *J* = 15.9 Hz, 1H), 7.00-7.04 (m, 2H), 7.25-7.29 (m, 2H); $^{13}$C NMR (100 MHz, $CDCl_3$): $\delta$ 9.16, 15.12, 32.03, 38.75, 47.20, 52.23, 72.91, 79.06, 125.67, 125.84, 127.90, 133.78, 134.82, 142.79; HRMS (ESI+): *m*/*z* calcd for $C_{19}H_{26}N$ $[M+H]^+$ 266.1909; found 266.1908; HPLC purity, 98.1%.

REFERENCE EXAMPLE 9

**Synthesis of 4-(4-cyclopropylphenethyl)-1-(prop-2-yn-1-yl)piperidine**

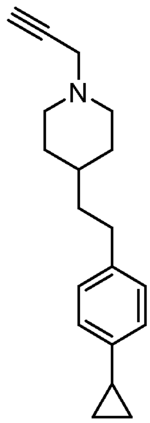

*Step 1:* ***Tert*-butyl 4-(4-cyclopropylphenethyl)piperidine-1-carboxylate**

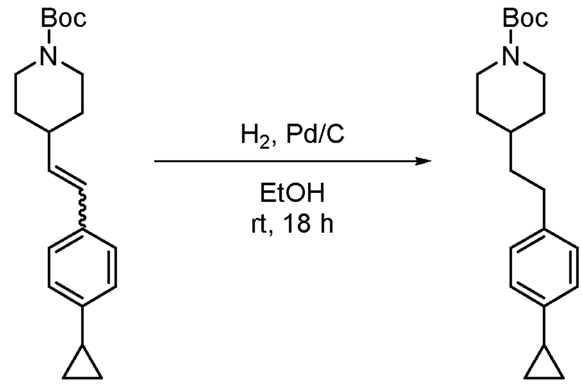


Synthesized from *tert*-butyl (*E*/*Z*)-4-(4-cyclopropylstyryl)piperidine-1-carboxylate (0.216 g, 0.66 mmol, 1.0 equiv.) via general procedure C. The product was used without further purification. Mass: 0.200 g; $R_f$ = 0.67 (EtOAc/n-hex = 1/2, v/v); colorless oil; $^1$H NMR (400 MHz, $CDCl_3$): δ 0.95 (t, *J* = 7.4 Hz, 2H), 1.11 (dt, *J* = 12.4, 4.1 Hz, 1H), 1.14 (dt, *J* = 12.4, 4.2 Hz, 1H), 1.37-1.47 (m, 1H), 1.45 (s, 9H), 1.52-1.58 (m, 2H), 1.59-1.65 (m, 3H), 1.16-1.73 (m, 2H), 2.52-2.70 (m, 4H), 4.07 (bs, 2H), 7.06-7.11 (m, 4H).

*Step 2:* **4-(4-Cyclopropylphenethyl)-1-(prop-2-yn-1-yl)piperidine**

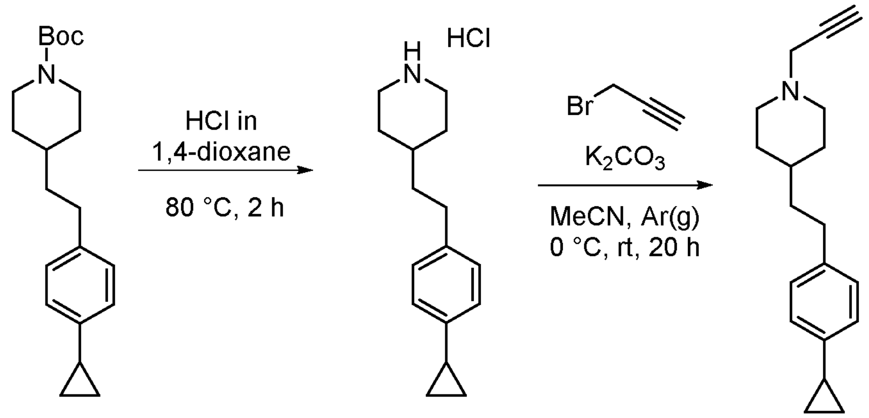


Synthesized from *tert*-butyl 4-(4-cyclopropylphenethyl)piperidine-1-carboxylate (0.185 g, 0.561 mmol, 1.0 equiv.) via general procedures D and E. The compound was purified by flash column chromatography using EtOAc/n-hex = 1/2 (v/v) as eluent. Yield: 45% (68 mg); $R_f$ = 0.32 (EtOAc/n-hex = 1/2, v/v); pale yellow transparent oil; $^1$H NMR (400 MHz, $CDCl_3$): δ 0.95 (t, *J* = 7.4 Hz, 2H), 1.26-1.37 (m, 2H), 1.53-1.59 (m, 2H), 1.60-1.68 (m, 2H), 1.74-1.79 (m, 1H), 2.14-2.21 (m, 2H), 2.23(t, *J* = 2.4 Hz, 1H), 2.53-2.62 (m, 4H), 2.86-2.91 (m, 3H), 3.30 (d, *J* = 2.4 Hz, 2H), 7.07-7.11 (m, 4H); $^{13}$C NMR (100 MHz, $CDCl_3$): δ 13.86, 24.59, 32.20, 32.60, 34.75, 37.63, 38.37, 47.19, 52.28, 72.80, 79.20, 128.08, 128.33, 132.38, 139.89; HPLC purity, 97.1%.

EXAMPLE 10

**Synthesis of (*E*)-4-(4-isopropylstyryl)-1-(prop-2-yn-1-yl)piperidine**

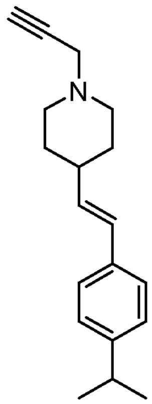

*Step 1:* ***Tert*-butyl (*E*)-4-(4-isopropylstyryl)piperidine-1-carboxylate**

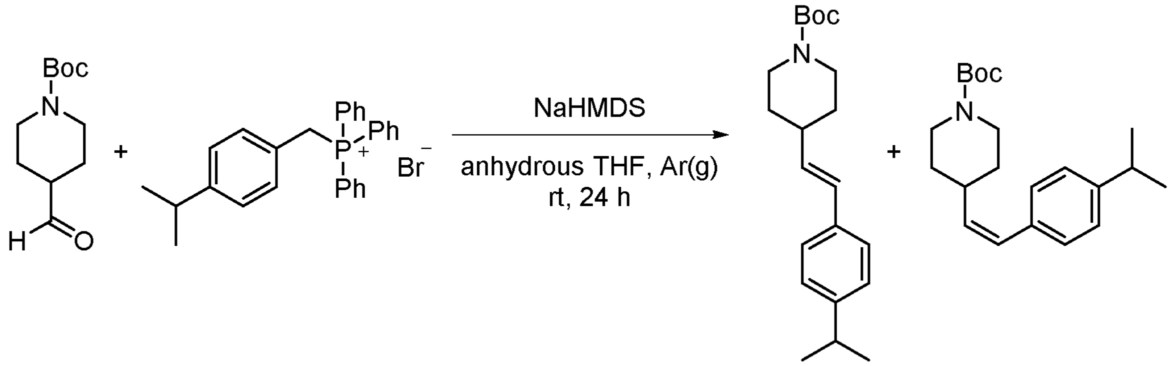


Synthesized from *tert*-butyl 4-formylpiperidine-1-carboxylate (1.30 g, 6.10 mmol, 1.0 equiv.) and 4-isopropylbenzyltriphenylphosphonium bromide (3.19 g, 6.71 mmol, 1.1 equiv., prepared via general procedure A) via general procedure B. The compound was purified by flash column chromatography using petroleum ether/$Et_2O$ = 9/1 (v/v) as eluent. Mass: 235 mg; $R_f$ = 0.24 (petroleum ether/$Et_2O$ = 10/1, v/v); colorless oil; $^1H$ NMR (400 MHz, $CDCl_3$): δ 1.25 (d, *J* = 7.0 Hz, 6H), 1.37 (dd, *J* = 12.8, 4.1 Hz, 1H), 1.40 (dd, *J* = 12.1, 3.9 Hz, 1H), 1.49 (s, 9H), 1.71-1.79 (m, 2H), 2.22-2.30 (m, 1H), 2.78 (t, *J* = 11.4 Hz, 2H), 2.89 (sept, *J* = 7.0 Hz, 1H), 4.14 (bs, 2H), 6.11 (dd, *J* = 16.0, 6.9 Hz, 1H), 6.38 (d, *J* = 15.9 Hz, 1H), 7.16-7.18 (m, 2H), 7.28-7.30 (m, 2H); MS (ESI+): *m/z* $[M+Na]^+$ 352.22; found 352.01.

*Step 2:* **(*E*)-4-(4-Isopropylstyryl)-1-(prop-2-yn-1-yl)piperidine**

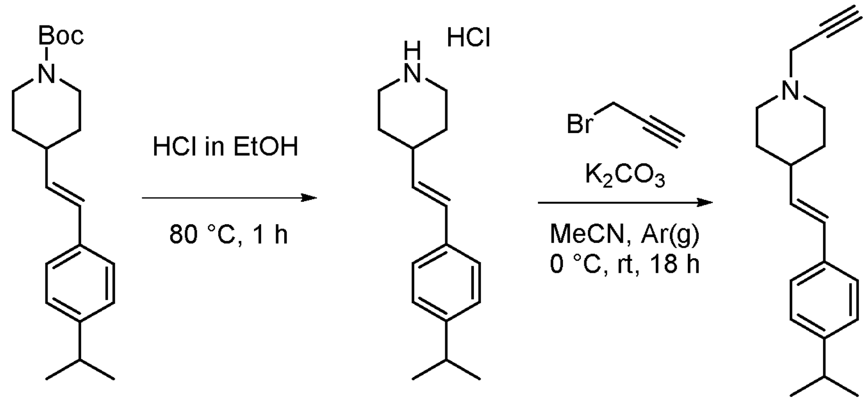


Synthesized from *tert*-butyl (*E*)-4-(4-isopropylstyryl)piperidine-1-carboxylate (0.207 g, 0.628 mmol, 1.0 equiv.)

via general procedures D and E. The compound was purified by flash column chromatography using EtOAc/n-hex = 1/1 (v/v) as eluent. Yield: 45% (76 mg); $R_f$ = 0.43 (EtOAc/n-hex = 1/1, v/v); pale yellow solid, mp 60-64 °C; $^1$H NMR (400 MHz, $CDCl_3$): δ 1.24 (d, *J* = 6.9 Hz, 6H), 1.52-1.61 (m, 2H), 1.79-1.83 (m, 2H), 2.06-2.19 (m, 1H), 2.24-2.31 (m, 2H), 2.26 (t, *J* = 2.5 Hz, 1H), 2.83-2.95 (m, 3H), 3.32 (d, *J* = 2.5 Hz, 2H), 6.13 (dd, *J* = 16.0, 7.0 Hz, 1H), 6.37 (d, *J* = 16.6 Hz, 1H), 7.15-7.18 (m, 2H), 7.27-7.31 (m, 2H); $^{13}$C NMR (100 MHz, $CDCl_3$): δ 23.93, 32.05, 33.76, 38.75, 47.22, 52.25, 72.90, 79.08, 125.90, 126.50, 127.96, 134.01, 135.21, 147.74; HRMS (ESI+): *m/z* calcd for $C_{19}H_{26}N$ $[M+H]^+$ 268.2065; found 268.2062; HPLC purity, 98.5%.

REFERENCE EXAMPLE 10

**Synthesis of 4-(4-isopropylphenethyl)-1-(prop-2-yn-1-yl)piperidine**

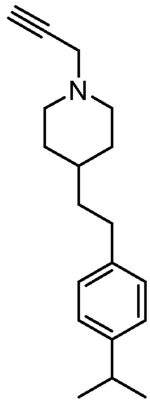

*Step 1:* ***Tert*-butyl 4-(4-isopropylphenethyl)piperidine-1-carboxylate**

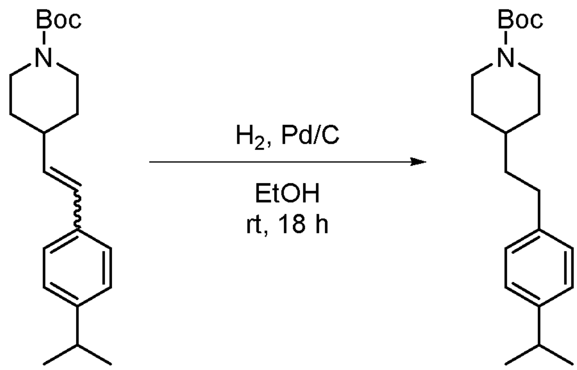


Synthesized from *tert*-butyl (*E*/*Z*)-4-(4-isopropylstyryl)piperidine-1-carboxylate (0.87 g, 2.64 mmol, 1.0 equiv.) via general procedure C. The product was used without further purification. Mass: 0.727 g; $R_f$ = 0.44 (petroleum ether/$Et_2O$ = 3/1, v/v); colorless oil; $^1$H NMR (400 MHz, $CDCl_3$): δ 1.16 (ddd, *J* = 16.9, 12.9, 5.5 Hz, 2H), 1.27 (d, *J* = 7.0 Hz, 6H), 1.40-1.47 (m, 1H), 1.49 (s, 9H), 1.54-1.61 (m, 2H), 1.70-1.76 (m, 2H), 2.61-2.64 (m, 2H), 2.70 (t, *J* = 11.5 Hz, 2H), 2.91 (sept. *J* = 6.9 Hz, 1H), 4.10 (bs, 2H), 7.11-7.13 (m, 2H), 7.16-7.18 (m, 2H MS (ESI+): *m/z* $[M+Na]^+$ 354.24; found 354.05.

*Step 2:* **4-(4-Isopropylphenethyl)-1-(prop-2-yn-1-yl)piperidine**

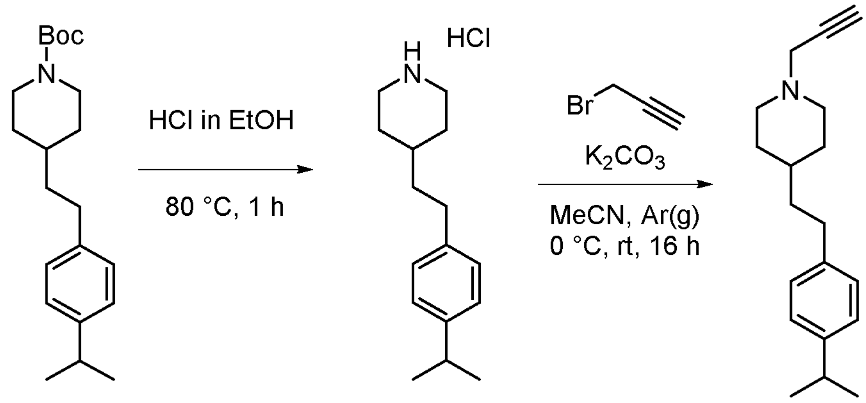


Synthesized from *tert*-butyl 4-(4-isopropylphenethyl)piperidine-1-carboxylate (0.290 g, 0.875 mmol, 1.0 equiv.) via general procedures D and E. The compound was purified by flash column chromatography using EtOAc/n-hex = 1/2 (v/v) as eluent. Yield: 55% (112 mg); $R_f$ = 0.35 (EtOAc/n-hex = 1/1, v/v); yellow transparent oil; $^1$H NMR (400 MHz, $CDCl_3$): δ 1.26 (d, *J* = 7.0 Hz, 6H), 1.29-1.39 (m, 3H), 1.55-1.61 (m, 2H), 1.75-1.81 (m, 2H), 2.16-2.22 (m, 2H), 2.24 (t, *J* = 2.4 Hz, 1H), 2.59-2.63 (m, 2H), 2.86-2.93 (m, 3H), 3.31 (d, *J* = 2.5 Hz, 2H), 7.11-7.13 (m, 2H), 7.15-7.17 (m, 2H); $^{13}$C NMR (100 MHz, $CDCl_3$): δ 24.01, 32.18, 32.56, 33.59, 34.75, 38.34, 47.16, 52.50, 72.78, 79.17, 126.24, 128.11, 139.91, 146.03; HRMS (ESI+): *m/z* calcd for $C_{19}H_{28}N$ $[M+H]^+$ 270.2222; found 270.2226; HPLC purity, 99.8%.

EXAMPLE 11

**Synthesis of (*E*)-4-(4-methoxystyryl)-1-(prop-2-yn-1-yl)piperidine**

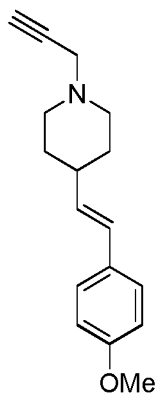

*Step 1:* ***Tert*-butyl (*E*/*Z*)-4-(4-methoxystyryl)piperidine-1-carboxylate**

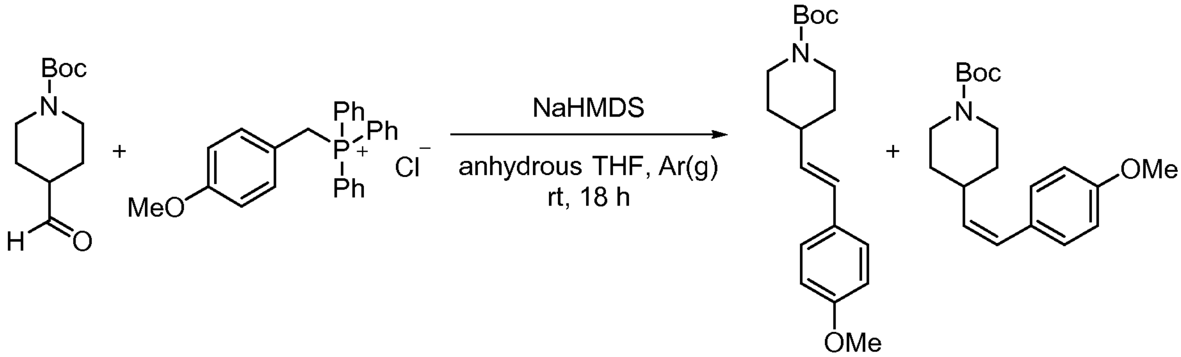

Synthesized from *tert*-butyl 4-formylpiperidine-1-carboxylate (1.87 g, 8.77 mmol, 1.0 equiv.) and 4-methoxybenzyltriphenylphosphonium chloride (4.04 g, 9.65 mmol, 1.1 equiv., prepared via general procedure A) via general procedure B. The compound was purified by flash column chromatography using EtOAc/n-hex = 1/9 (v/v) as eluent. Mass: 1120 mg (mixture of *cis*/*trans* isomers (ratio 20/80, estimated from $^1$H NMR)); $R_f$ = 0.13 (EtOAc/n-hex = 1/9, v/v); colorless oil; $^1$H NMR (400 MHz, $CDCl_3$): δ 1.31-1.41 (m, 2.6H, *cis* + *trans*), 1.46-1.47 (m, 11.5H, *cis* + *trans*), 1.63-1.75 (m, 2.6H, *cis* + *trans*), 2.20-2.29 (m, 1H, *trans*), 2.69-2.82 (m, 2.6H, *cis* + *trans*), 3.78 (s, 3H, *trans*), 3.78 (s, 0.7H, *cis*), 4.12 (bs, 2.6H, *cis* + *trans*), 5.36 (dd, *J* = 11.6, 10.0 Hz, 0.2H, *cis*), 5.99 (dd, *J* = 16.0, 6.9 Hz, 1H, *trans*), 6.30-6.34 (m, 1.2H, *cis* + *trans*), 6.81-6.85 (m, 2H, *trans*), 6.85-6.89 (m, 0.5H, *cis*), 7.16-7.19 (m, 0.5H, *cis*), 7.24-7.29 (m, 2H, *trans*); MS (ESI+): *m/z* $[M+Na]^+$ 340.19; found 340.04.

*Step 2:* **(*E*)-4-(4-Methoxystyryl)-1-(prop-2-yn-1-yl)piperidine**

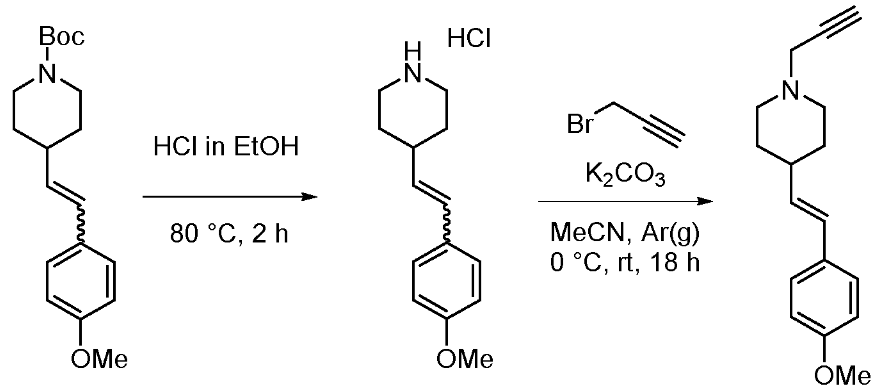


Synthesized from *tert*-butyl (*E*/*Z*)-4-(4-methoxystyryl)piperidine-1-carboxylate (0.810 g, 2.552 mmol, 1.0 equiv.) via general procedures D and E. The compound was purified by flash column chromatography using EtOAc/n-hex = 1/1 (v/v) as eluent. Only *trans* derivative was isolated. Yield: 32% (192 mg); $R_f$ = 0.29 (EtOAc/n-hex = 1/1, v/v); white solid, mp 59-61 °C; $^1$H NMR (400 MHz, $CDCl_3$): δ 1.50-1.60 (m, 2H), 1.75-1.82 (m, 2H), 2.06-2.15 (m, 1H), 2.23-2.30 (m, 2H), 2.25 (t, *J* = 2.4 Hz, 1H), 2.88-2.94 (m, 2H), 3.32 (d, *J* = 2.4 Hz, 2H), 3.79 (s, 3H), 6.02 (dd, *J* = 15.9, 7.0 Hz, 1H), 6.32 (d, *J* = 15.9 Hz, 1H), 6.81-6.85 (m, 2H), 7.26-7.30 (m, 2H); $^{13}$C NMR (100 MHz, $CDCl_3$): δ 32.13, 38.77, 47.23, 52.28, 55.23, 72.90, 79.11, 113.85, 127.03, 127.49, 130.39, 132.78, 158.69; HRMS (ESI+): *m/z* calcd for $C_{17}H_{22}NO$ $[M+H]^+$ 256.1701; found 256.1706; HPLC purity, 97.7%.

REFERENCE EXAMPLE 11

**Synthesis of 4-(4-methoxyphenethyl)-1-(prop-2-yn-1-yl)piperidine**

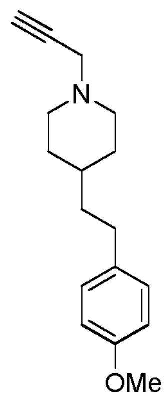

*Step 1:* **Tert-butyl 4-(4-methoxyphenethyl)piperidine-1-carboxylate**

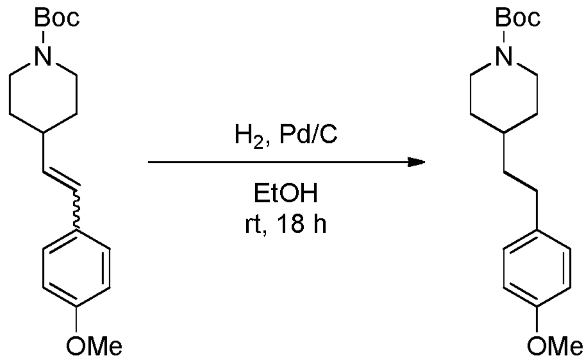


Synthesized from *tert*-butyl (E/Z)-4-(4-methoxystyryl)piperidine-1-carboxylate (0.25 g, 0.79 mmol, 1.0 equiv.) via general procedure C. The product was used without further purification. Yield: 97 % (0.245 g); Rf = 0.75 (EtOAc/n-hex = 1/1, v/v); colorless oil; $^1$H NMR (400 MHz, $CDCl_3$): δ 1.12 (ddd, *J* = 16.3, 12.5, 4.3 Hz, 2H), 1.35-1.43 (m, 1H), 1.45 (s, 9H), 1.50-1.56 (m, 2H), 1.64-1.71 (m, 2H), 2.55-2.59 (m, 2H), 2.66 (t, *J* = 11.8 Hz, 2H), 3.79 (s, 3H), 4.07 (bs, 2H), 6.81-6.84 (m, 2H), 7.07-7.10 (m, 2H); MS (ESI+): *m/z* $[M+Na]^+$ 342.20; found 342.59.

*Step 2:* **4-(4-Methoxyphenethyl)-1-(prop-2-yn-1-yl)piperidine**

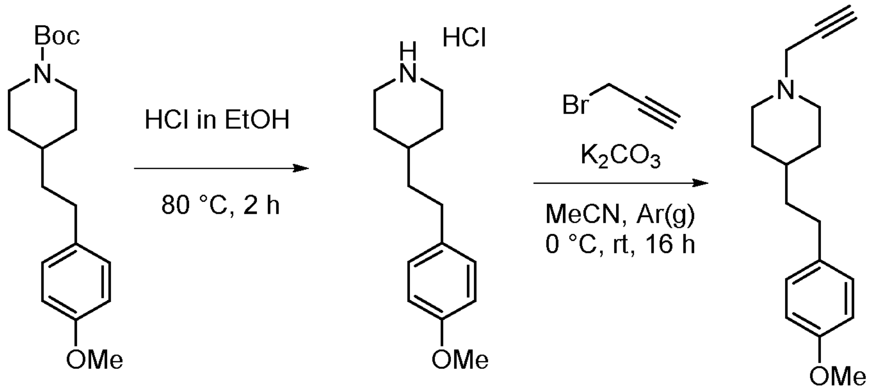


Synthesized from *tert*-butyl 4-(4-methoxyphenethyl)piperidine-1-carboxylate (0.230 g, 0.720 mmol, 1.0 equiv.) via general procedures D and E. The compound was purified by flash column chromatography using EtOAc/n-hex = 1/1 (v/v) as eluent. Yield: 57% (103 mg); Rf = 0.26 (EtOAc/n-hex = 1/1, v/v); white solid, mp 31-32 °C; $^1$H NMR (400 MHz, $CDCl_3$): δ 1.22-1.36 (m, 3H), 1.50-1.56 (m, 2H), 1.72-1.77 (m, 2H), 2.13-2.19 (m, 2H), 2.23 (t, *J* = 2.5 Hz, 1H), 2.54-2.58 (m, 2H), 2.85-2.89 (m, 2H), 3.28 (d, *J* = 2.5 Hz, 2H), 3.77 (s, 3H), 6.80-6.84 (m, 2H), 7.07-7.10 (m, 2H); $^{13}$C NMR (100 MHz, $CDCl_3$): *δ* 32.00, 32.14, 34.57, 38.45, 47.12, 52.46, 55.10, 72.77, 79.15, 113.60, 129.04, 134.60, 157.51; HRMS (ESI+): *m/z* calcd for $C_{17}H_{24}NO$ $[M+H]^+$ 258.1858; found 258.1857; HPLC purity, 100%.

REFERENCE EXAMPLE 12

**Synthesis of 4-(4-hydroxyphenethyl)-1-(prop-2-yn-1-yl)piperidine**

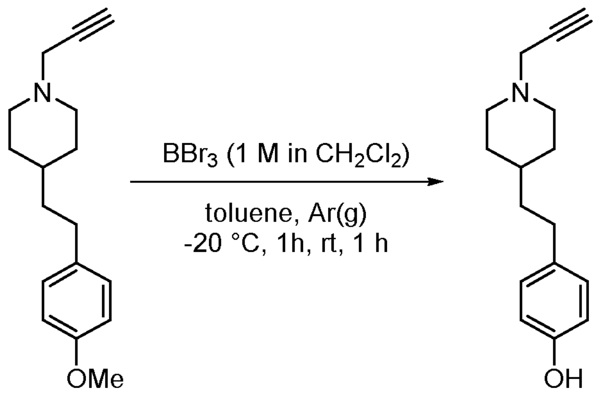


The methoxy substituted starting compound (50 mg, 0.194 mmol, 1.0 equiv.) was dissolved in anhydrous toluene (6 mL), purged under a stream of argon for 15 min, and cooled to -20 °C. $BBr_3$ (1M solution in $CH_2Cl_2$, 582 $\mu$L, 0.582 mmol, 3.0 equiv.) was added drop-wise and the reaction mixture was stirred for another hour at -20 °C. The mixture was allowed to warm-up to room temperature (1 h). Saturated aqueous $NaHCO_3$ (10 mL) was added, and the resulting emulsion was stirred vigorously for 15 min before EtOAc (30 mL) was added. The phases were separated, and the organic layer was washed with saturated brine (50 mL), dried over anhydrous $Na_2SO_4$, and evaporated under reduced pressure. The crude product was purified by flash column chromatography using $CH_2Cl_2$/MeOH = 20/1 (v/v) as eluent. Yield: 57% (27 mg); Rf = 0.19 ($CH_2Cl_2$/MeOH = 20/1, v/v); white solid, mp 104-106 °C; $^1$H NMR (400 MHz, $CDCl_3$): $\delta$ 1.22-1.39 (m, 3H), 1.48-1.54 (m, 2H), 1.72-1.78 (m, 2H), 2.21-2.27 (m, 2H), 2.25 (t, J = 2.4 Hz, 1H), 2.51-2.55 (m, 2H), 2.89-2.95 (m, 2H), 3.32 (d, J = 2.5 Hz, 2H), 6.71-6.74 (m, 2H), 6.99-7.02 (m, 2H), resonance for OH missing; $^{13}$C NMR (100 MHz, $CDCl_3$): $\delta$ 31.89, 32.09, 34.55, 38.41, 47.07, 52.41, 73.33, 78.68, 115.29, 129.27, 134.35, 153.92; HRMS (ESI+): m/z calcd for $C_{16}H_{22}NO$ [M+H]+ 244.1701; found 244.1697; HPLC purity, 96.1%.

EXAMPLE 12

**Synthesis of (*E*)-4-(4-cyanostyryl)-1-(prop-2-yn-1-yl)piperidine**

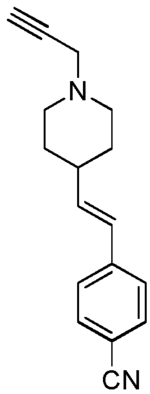

*Step 1:* ***Tert*-butyl (*E*)-4-(4-cyanostyryl)piperidine-1-carboxylate**

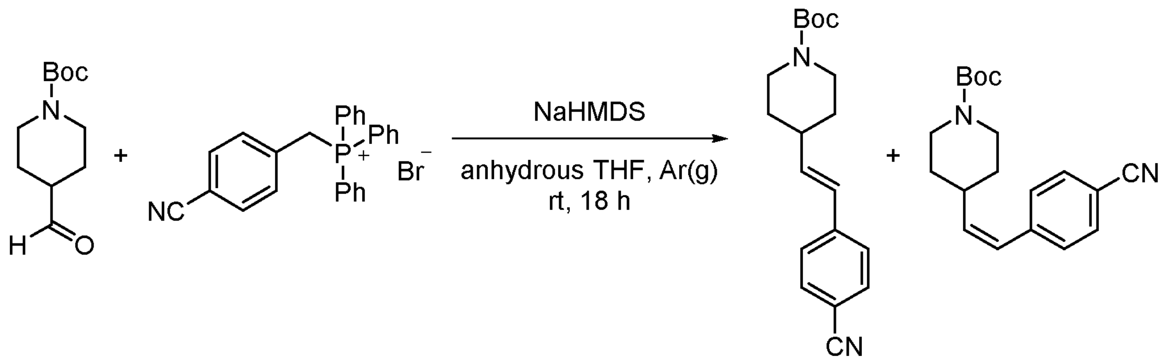


Synthesized from *tert*-butyl 4-formylpiperidine-1-carboxylate (2.35 g, 11.02 mmol, 1.0 equiv.) and 4-cyanobenzyltriphenylphosphonium bromide (5.56 g, 12.12 mmol, 1.1 equiv., prepared via general procedure A) via general procedure B. The compound was purified by flash column chromatography using petroleum ether/$Et_2O$ = 3/1 (v/v) as eluent. Yield: 9,3 % (320 mg); Rf = 0.14 (petroleum ether/$Et_2O$ = 3/1, v/v); white crystals, mp 88-90 °C; $^1H$ NMR (400 MHz, $CDCl_3$): δ 1.30-1.40 (m, 2H), 1.43 (s, 9H), 1.70-1.75 (m, 2H), 2.25-2.34 (m, 1H), 2.75 (t, *J* = 10.7 Hz, 2H), 4.11 (bs, 2H), 6.26 (dd, *J* = 16.0, 6.6 Hz, 1H), 6.36 (d, *J* = 16.0 Hz, 1H), 7.37-7.39 (m, 2H), 7.52-7.55 (m, 2H); MS (ESI+): *m/z* $[M-H]^-$ 311.17; found 311.37.

*Step 2:* ***(E)*-4-(4-Cyanostyryl)-1-(prop-2-yn-1-yl)piperidine**

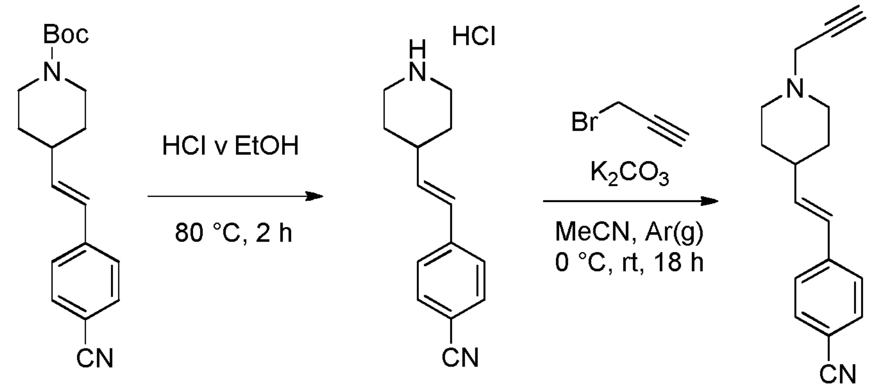


Synthesized from *tert*-butyl (E)-4-(4-cyanostyryl)piperidine-1-carboxylate (0.300 g, 0.96 mmol, 1.0 equiv.) via general procedures D and E. The compound was purified by flash column chromatography using $CH_2Cl_2$/MeOH = 50/1 (v/v) as eluent. Yield: 54% (130 mg); Rf = 0.16 ($CH_2Cl_2$/MeOH = 50/1, v/v); white solid, mp 59-62 °C; $^1H$ NMR (400 MHz, $CDCl_3$): δ 1.47-1.58 (m, 2H), 1.76-1.79 (m, 2H), 2.10-2.17 (m, 1H), 2.21-2.27 (m, 2H), 2.23 (t, *J* = 2.4 Hz, 1H), 2.88-2.91 (m, 2H), 3.28 (d, *J* = 2.5 Hz, 2H), 6.27 (dd, *J* = 16.0, 6.5 Hz, 1H), 6.35 (d, *J* = 16.1 Hz, 1H), 7.36-7.39 (m, 2H), 7.51-7.54 (m, 2H); $^{13}C$ NMR (100 MHz, $CDCl_3$): δ 31.58, 38.78, 47.06, 51.94, 72.96, 78.83, 109.94, 118.93, 126.35, 126.77, 132.16, 138.94, 142.00; HRMS (ESI+): *m/z* calcd for $C_{17}H_{19}N_2$ $[M+H]^+$ 251.1548; found 251.1549; HPLC purity, 99.3%.

REFERENCE EXAMPLE 13

**Synthesis of 4-(2-(1-(prop-2-yn-1-yl)piperidine-4-yl)ethyl)benzonitrile**

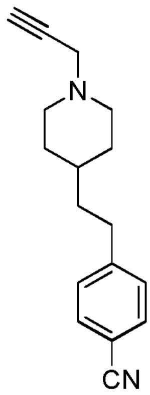

*Step 1:* **Tert-butyl 4-(4-cyanophenethyl)piperidine-1-carboxylate**

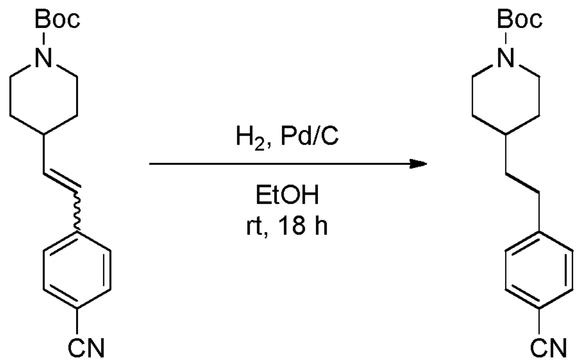


Synthesized from *tert*-butyl (E/Z)-4-(4-cyanostyryl)piperidine-1-carboxylate (0.658 g, 2.11 mmol, 1.0 equiv.) via general procedure C. The crude product was purified by flash column chromatography using petroleum ether/$Et_2O$ = 3/1 (v/v) as eluent. Yield: 95% (0.632 g); $R_f$ = 0.15 (petroleum ether/$Et_2O$ = 3/1, v/v); colorless oil; $^1H$ NMR (400 MHz, $CDCl_3$): δ 1.09 (dd, *J* = 12.4, 4.2 Hz, 1H), 1.12 (dd, *J* = 12.5, 4.3 Hz, 1H), 1.34-1.40 (m, 1H), 1.42 (s, 9H), 1.51-1.58 (m, 2H), 1.63-1.68 (m, 2H), 2.61-2.68 (m, 4H), 4.06 (bs, 2H), 7.23-7.25 (m, 2H), 7.51-7.54 (m, 2H); MS (ESI+): *m*/*z* $[M+Na]^+$ 337.19; found 337.07.

*Step 2:* **4-(2-(1-(Prop-2-yn-1-yl)piperidine-4-yl)ethyl)benzonitrile**

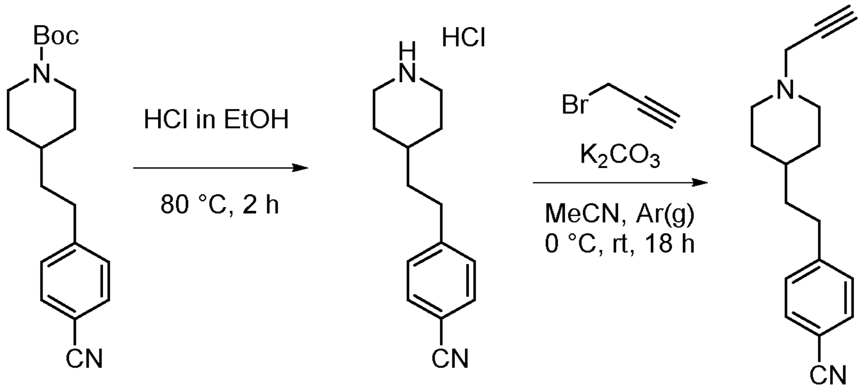


Synthesized from *tert*-butyl 4-(4-cyanophenethyl)piperidine-1-carboxylate (0.075 g, 0.239 mmol, 1.0 equiv.) via general procedures D and E. The compound was purified by flash column chromatography using EtOAc/n-hex = 1/1

(v/v) as eluent. Yield: 59% (106 mg); Rf = 0.14 (EtOAc/n-hex = 1/1, v/v); white solid, mp 40-42 °C; $^1$H NMR (400 MHz, $CDCl_3$): δ 1.21-1.36 (m, 3H), 1.53-1.58 (m, 2H), 1.72-1.77 (m, 2H), 2.13-2.19 (m, 2H), 2.22 (t, *J* = 2.4 Hz, 1H), 2.65-2.69 (m, 2H), 2.86-2.89 (m, 2H), 3.28 (d, *J* = 2.5 Hz, 2H), 7.25-7.28 (m, 2H), 7.54-7.57 (m, 2H); $^{13}$C NMR (100 MHz, $CDCl_3$): *δ* 31.99, 33.13, 34.62, 37.60, 47.03, 52.28, 72.82, 78.97, 109.39, 118.96, 128.96, 132.01, 148.23; HRMS (ESI+): *m/z* calcd for $C_{17}H_{21}N_2$ $[M+H]^+$ 253.1705; found 253.1706; HPLC purity, 96.0%.

EXAMPLE 13

**Synthesis of (*E*)-4-(2-(1-(Prop-2-yn-1-yl)piperidine-4-yl)vinyl)benzamide**

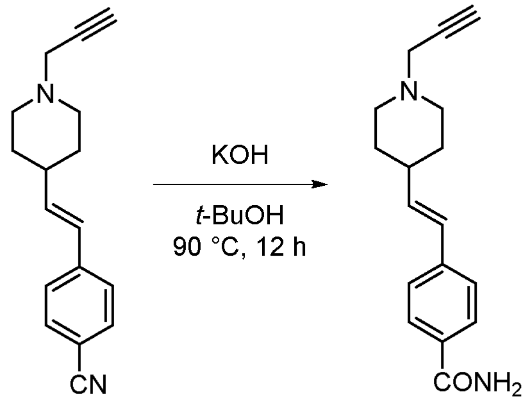


The starting (*E*)-4-(2-(1-(prop-2-yn-1-yl)piperidin-4-yl)vinyl)benzonitrile (0.080 g, 0.320 mmol, 1.0 equiv.) was dissolved in tert-butanol (40 mL), followed by the addition of powdered KOH (0.054 g, 0.960 mmol, 3.0 equiv.). The resulting suspension was stirred at 90 °C for 12 h, and then the solvent was evaporated under reduced pressure. The residue was dissolved in a mixture of $CH_2Cl_2$ (70 mL) and saturated aqueous $NaHCO_3$ (50 mL), and transferred into a separating funnel. Water layer was additionally extracted with $CH_2Cl_2$ (30 mL). Combined organic layers were washed with saturated brine (50 mL), dried over anhydrous $Na_2SO_4$, and evaporated under reduced pressure. The crude product was purified by flash column chromatography using $CH_2Cl_2$/MeOH = 20/1 (v/v) as eluent. Yield: 63% (54 mg); Rf = 0.08 ($CH_2Cl_2$/MeOH = 20/1, v/v); white crystals, mp 181-184; $^1$H NMR (400 MHz, DMSO-$d_6$): δ 1.42 (dq, *J* = 12.7, 3.9 Hz, 2H), 1.71-1.75 (m, 2H), 2.06-2.12 (m, 1H), 2.17 (td, *J* = 11.5, 2.2 Hz, 2H), 2.79-2.83 (m, 2H), 3.14 (t, *J* = 2.4 Hz, 1H), 3.26 (d, *J* = 2.4 Hz, 2H), 6.36-6.46 (m, 2H), 7.30 (bs, 1H), 7.44-7.47 (m, 2H), 7.79-7.82 (m, 2H), 7.93 (bs, 1H); 13C NMR (100 MHz, DMSO-$d_6$): δ 31.34, 38.32, 46.38, 51.42, 75.53, 79.53, 125.53, 126.97, 127.76, 132.41, 137.05, 140.00, 167.45; HRMS (ESI+): m/z calcd for $C_{17}H_{21}N_2O$ $[M+H]^+$ 269.1654; found 269.1656; HPLC purity, 99.4%.

REFERENCE EXAMPLE 14

**Synthesis of 4-(2-(1-(Prop-2-yn-1-yl)piperidine-4-yl)ethyl)benzamide**

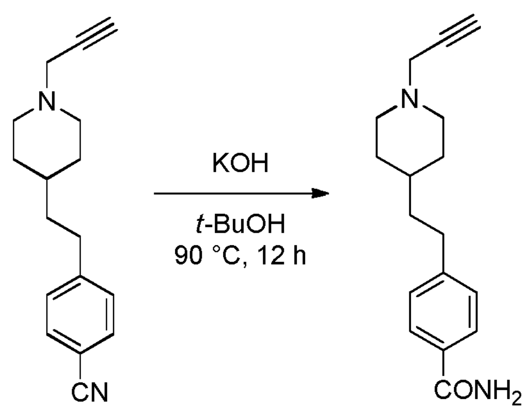

The starting 4-(2-(1-(prop-2-yn-1-yl)piperidin-4-yl)ethyl)benzonitrile (0.075 g, 0.297 mmol, 1.0 equiv.) was dissolved in tert-butanol (20 mL), followed by the addition of powdered KOH (0.050 g, 0.892 mmol, 3.0 equiv.). The resulting suspension was stirred at 90 °C for 12 h, and then the solvent was evaporated under reduced pressure. The residue was dissolved in a mixture of $CH_2Cl_2$ (50 mL) and saturated aqueous $NaHCO_3$ (50 mL), and transferred into a separating funnel. Water layer was additionally extracted with $CH_2Cl_2$ (30 mL). Combined organic layers were washed with saturated brine (50 mL), dried over anhydrous $Na_2SO_4$, and evaporated under reduced pressure. The crude product was purified by flash column chromatography using $CH_2Cl_2$/MeOH = 9/1 (v/v) as eluent. Yield: 42% (34 mg); Rf = 0.25 ($CH_2Cl_2$/MeOH = 9/1, v/v); white solid, mp 155-158 °C; $^1$H NMR (400 MHz, MeOD): $\delta$ 1.28-1.34 (m, 3H), 1.57-1.63 (m, 2H), 1.80-1.83 (m, 2H), 2.21 (t, *J* = 11.3 Hz, 2H), 2.69 (t, *J* = 2.5 Hz, 1H), 2.70-2.74 (m, 2H), 2.94-2.98 (m, 2H), 3.29 (d, *J* = 2.5 Hz, 2H), 7.29-7.32 (m, 2H), 7.79-7.82 (m, 2H), resonance for $CONH_2$ missing; $^{13}$C NMR (100 MHz, MeOD): $\delta$ 32.79, 33.93, 35.97, 39.16, 47.69, 53.60, 75.15, 79.24, 128.88, 129.55, 132.45, 148.46, 172.44; HRMS (ESI+): *m/z* calcd for $C_{17}H_{23}N_2O$ $[M+H]^+$ 269.1810; found 269.1816; HPLC purity, 99.6%.

EXAMPLE 14

**Synthesis of (*E*)-4-(4-(trifluoromethyl)styryl)-1-(prop-2-yn-1-yl)piperidine**

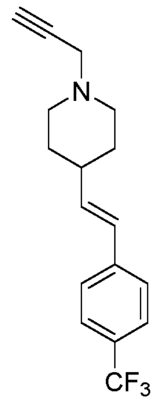

*Step 1:* **Tert-butyl (*E*)-4-(4-(trifluoromethyl)styryl)piperidine-1-carboxylate**

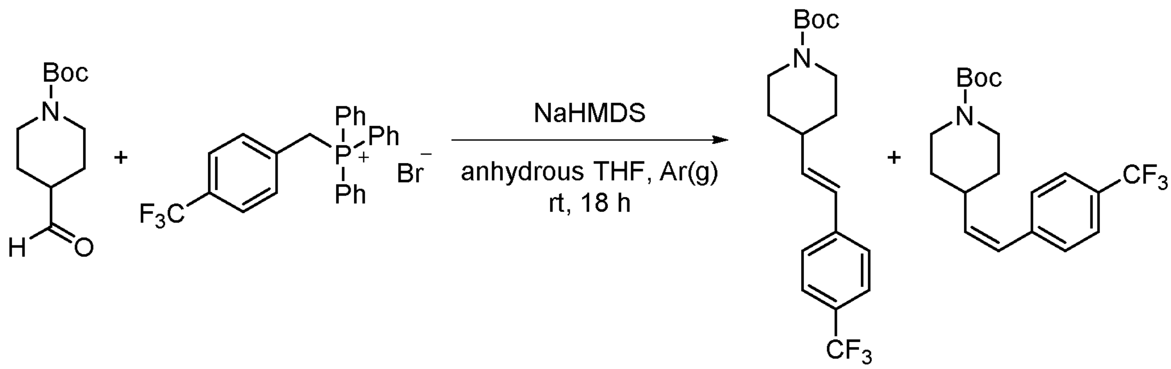


Synthesized from tert-butyl 4-formylpiperidine-1-carboxylate (2.00 g, 9.38 mmol, 1.0 equiv.) and 4-(trifluoromethyl)benzyltriphenylphosphonium bromide (5.56 g, 10.32 mmol, 1.0 equiv., prepared via general procedure A) via general procedure B. The compound was purified by flash column chromatography using petroleum ether/$Et_2O$ = 9/1 (v/v) as eluent. Yield: 23 % (780 mg); Rf = 0.18 (petroleum ether/$Et_2O$ = 9/1, v/v); white crystals, mp 46-48 °C; $^1$H NMR (400 MHz, $CDCl_3$): $\delta$ 1.35 (dd, *J* = 12.2, 4.1 Hz, 1H), 1.39 (dd, *J* = 12.5, 4.1 Hz, 1H), 1.46 (s, 9H), 1.71-1.77 (m, 2H), 2.24-2.33 (m, 1H), 2.76 (t, *J* = 11.4 Hz, 2H), 4.14 (bs, 2H), 6.22 (dd, *J* = 16.0, 6.8 Hz, 1H), 6.38 (d, *J* = 16.1 Hz, 1H), 7.40 (d, *J* = 8.4 Hz, 2H), 7.51 (d, *J* = 8.2 Hz, 2H).

*Step 2:* **(*E*)-4-(4-(Trifluoromethyl)styryl)-1-(prop-2-yn-1-yl)piperidine**

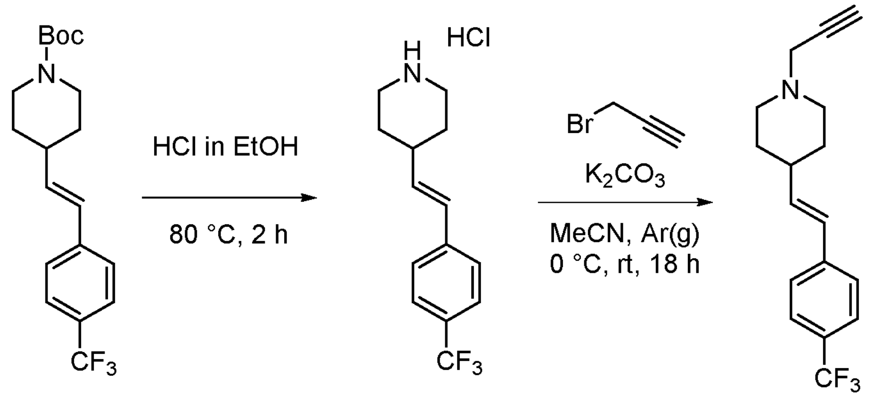


Synthesized from *tert*-butyl (*E*)-4-(4-(trifluoromethyl)styryl)piperidine-1-carboxylate (580 mg, 1.632 mmol, 1.0 ekviv.) via general procedures D and E. For the alkylation (procedure E) 440 mg of (E)-4-(4-(trifluoromethyl)styryl)piperidinium chloride was used. The compound was purified by flash column chromatography using EtOAc/n-hex = 2/1 (v/v) as eluent. Yield: 85% (376 mg); Rf = 0.50 (EtOAc/n-hex = 2/1, v/v); white solid, mp 72-74 °C; $^1$H NMR (400 MHz, $CDCl_3$): δ 1.52-1.62 (m, 2H), 1.80-1.84 (m, 2H), 2.12-2.19 (m, 1H), 2.26 (t, *J* = 2.4 Hz, 1H), 2.25-2.32 (m, 2H), 2.92-2.96 (m, 2H), 3.33 (d, *J* = 2.5 Hz, 2H), 6.27 (dd, *J* = 16.0, 6.9 Hz, 1H), 6.41 (d, *J* = 16.0 Hz, 1H), 7.43 (d, *J* = 8.1 Hz, 2H), 7.54 (d, *J* = 8.2 Hz, 2H); $^{13}$C NMR (100 MHz, $CDCl_3$): δ 31.78, 38.84, 47.17, 52.10, 72.97, 78.95, 121.50 (d, $J_{C,F}$ = 271.6 Hz), 125.35 (q, $J_{C,F}$ = 3.7 Hz), 126.07, 127.03, 128.69 (q, $J_{C,F}$ = 32.4 Hz), 137.62, 140.07 (q, $J_{C,F}$ = 1.3 Hz); HRMS (ESI+): *m*/*z* calcd for $C_{17}H_{19}F_3N$ $[M+H]^+$ 294.1470; found 294.1464; HPLC purity, 99.9%.

REFERENCE EXAMPLE 15

**Synthesis of 1-(prop-2-yn-1-yl)-4-(4-(trifluoromethyl)phenethyl)piperidine**

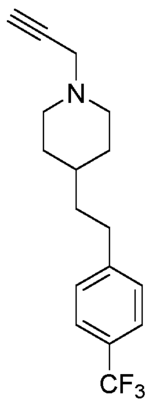

*Step 1:* **Tert-butyl 4-(4-(trifluoromethyl)phenethyl)piperidine-1-carboxylate**

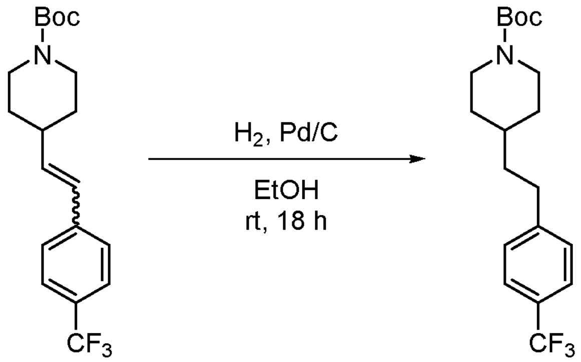


Synthesized from *tert*-butyl (*E*/*Z*)-4-(4-(trifluoromethyl)styryl)piperidine-1-carboxylate (0.24 g, 0.68 mmol, 1.0 equiv.) via general procedure C. The product was used without further purification. Yield: quantitative (241 mg); Rf = 0.56 (EtOAc/n-hex = 1/2, v/v); yellow transparent oil; $^1$H NMR (400 MHz, $CDCl_3$): δ 1.12 (dt, *J* = 12.4, 4.2 Hz, 1H), 1.15 (dt, *J* = 12.3, 4.3 Hz, 1H), 1.36-1.44 (m, 1H), 1.45 (s, 9H), 1.53-1.61 (m, 2H), 1.65-1.73 (m, 2H), 2.60-2.70 (m, 4H), 4.08 (bs, 2H), 7.25-7.28 (m, 2H), 7.50-7.53 (m, 2H).

*Step 2:* **4-(4-(Trifluoromethyl)phenethyl)-1-(prop-2-yn-1-yl)piperidine**

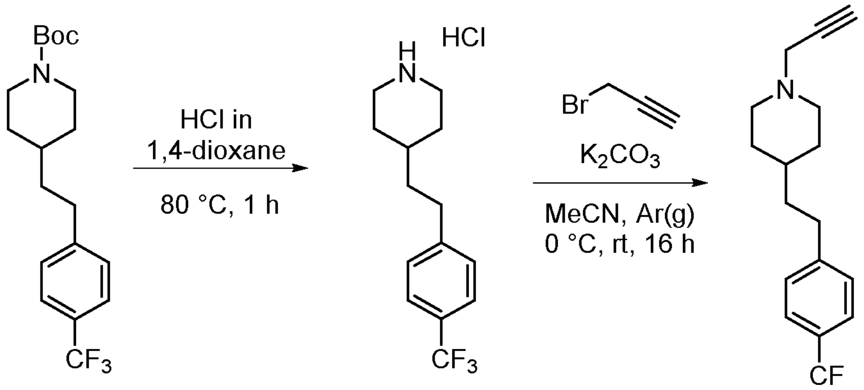


Synthesized from *tert*-butyl 4-(4-(trifluoromethyl)phenethyl)piperidine-1-carboxylate (0.230 g, 0.64 mmol, 1.0 equiv.) via general procedures D and E. The compound was purified by flash column chromatography using EtOAc/n-hex = 1/2 (v/v) as eluent. Yield: 7% (14 mg); $R_f$ = 0.12 (EtOAc/n-hex = 1/2, v/v); brown amorphous solid, mp 31-33 °C; $^1$H NMR (400 MHz, $CDCl_3$): δ 1.23-1.37 (m, 3H), 1.54-1.60 (m, 2H), 1.74-1.78 (m, 2H), 2.14-2.20 (m, 2H), 2.23 (t, *J* = 2.4 Hz, 1H), 2.65-2.70 (m, 2H), 2.86-2.90 (m, 2H), 3.29 (d, *J* = 2.4 Hz, 2H), 7.26-7.29 (m, 2H), 7.50-7.53 (m, 2H); $^{13}$C NMR (100 MHz, $CDCl_3$): δ 32.14, 32.91, 34.71, 37.97, 47.17, 52.45, 72.89, 79.11, 124.35 (q, $J_{C,F}$ = 271.9 Hz), 125.20 (q, $J_{C,F}$ = 7.9 Hz), 128.02 (q, $J_{C,F}$ = 32,3 Hz) 128.56, 146.76; HRMS (ESI+): *m*/*z* calcd for $C_{17}H_{21}F_3N$ [M+H]$^+$ 296.1626; found 296.1625 HPLC purity, 100%.

EXAMPLE 15

**Synthesis of (*E*)-4-(3-(trifluoromethyl)styryl)-1-(prop-2-yn-1-yl)piperidine**

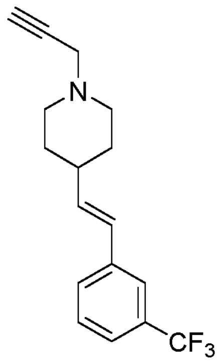

*Step 1:* **Tert-butyl (*E*)-4-(3-(trifluoromethyl)styryl)piperidine-1-carboxylate**

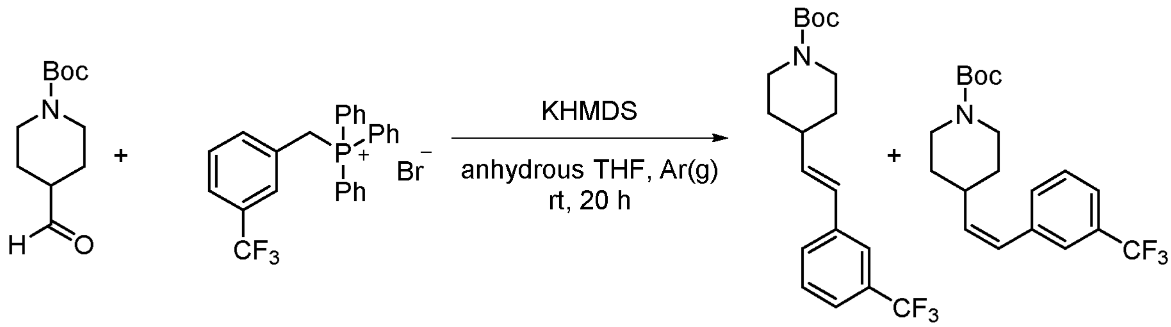


Synthesized from *tert*-butyl 4-formylpiperidine-1-carboxylate (1.49 g, 7.00 mmol, 1.0 equiv.) and 3-(trifluoromethyl)benzyltriphenylphosphonium bromide (3.51 g, 7.00 mmol, 1.0 equiv., prepared via general procedure A) via general procedure B. The compound was purified by flash column chromatography using petroleum ether/$Et_2O$ = 9/1 (v/v) as eluent. Mass: 404 mg; Rf = 0.16 (petroleum ether/$Et_2O$ = 8/1, v/v); colorless transparent oil; $^1H$ NMR (400 MHz, $CDCl_3$): δ 1.37 (dt, *J* = 12.4, 4.3 Hz, 1H), 1.40 (dt, *J* =12.2, 4.1 Hz, 1H), 1.47 (s, 9H), 1.73-1.80 (m, 2H), 2.26-2.36 (m, 1H), 2.78 (t, *J* = 12.0 Hz, 2H), 4.14 (bs, 2H), 6.22 (dd, *J* = 16.0, 6.9 Hz, 1H), 6.41 (d, *J* = 16.0 Hz, 1H), 7.38-7.46 (m, 2H), 7.48-7.51 (m, 1H), 7.57-7.59 (m, 1H); MS (ESI+): *m/z* $[M+Na]^+$ 378.17; found 378.69.

*Step 2:* **(*E*)-4-(3-(Trifluoromethyl)styryl)-1-(prop-2-yn-1-yl)piperidine**

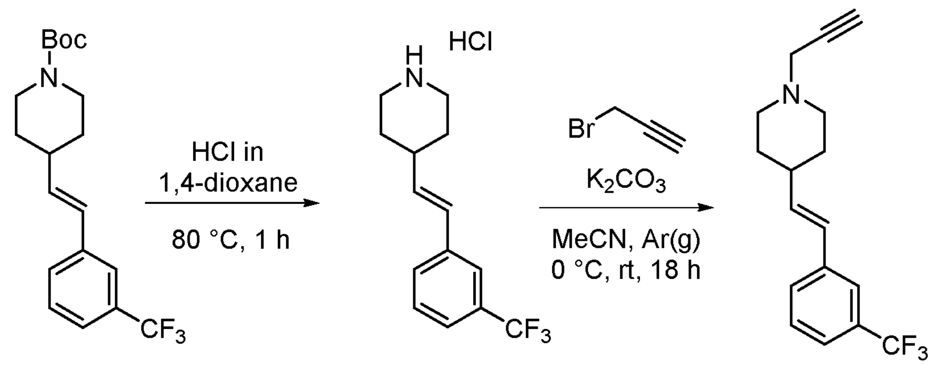


Synthesized from *tert*-butyl (*E*)-4-(3-(trifluoromethyl)styryl)piperidine-1-carboxylate (190 mg, 0.53 mmol, 1.0 ekviv.) via general procedures D and E.. The compound was purified by flash column chromatography using EtOAc/n-hex = 1/3 (v/v) as eluent. Yield: 70% (109 mg); Rf = 0.18 (EtOAc/n-hex = 1/3, v/v); green oil; $^1H$ NMR (400 MHz, $CDCl_3$): δ 1.56 (dt, *J* = 12.1, 3.7 Hz, 1H), 1.59 (dt, *J* = 12.1, 3.7 Hz, 1H), 1.79-1.85 (m, 2H), 2.11-2.20 (m, 1H), 2.26 (t, *J* = 2.4

Hz, 1H), 2.29 (dt, *J* = 11.8, 2.3 Hz, 2H), 2.92-2.97 (m, 2H), 3.33 (d, *J* = 2.4 Hz, 2H), 6.24 (dd, *J* = 16.0, 6.9 Hz, 1H), 6.41 (d, *J* = 16.0 Hz, 1H), 7.38-7.45 (m, 2H), 7.51 (d, *J* = 7.5 Hz, 1H), 7.58 (s, 1H); $^{13}$C NMR (100 MHz, $CDCl_3$): δ 31.87, 38.86, 47.24, 52.19, 73.01, 79.02, 122.64 (q, $J_{C,F}$ = 3.9 Hz), 123.49 (q, $J_{C,F}$ = 3.9 Hz), 124.16 (q, $J_{C,F}$ = 272.3 Hz), 127.03, 128.88, 129.18, 130.85 (q, $J_{C,F}$ = 32.1 Hz), 136.90, 138.38; HRMS (ESI+): *m/z* calcd for $C_{17}H_{19}NF_3$ $[M+H]^+$ 294.1470; found 294.1465; HPLC purity, 99.8%.

REFERENCE EXAMPLE 16

**Synthesis of 1-(prop-2-yn-1-yl)-4-(3-(trifluoromethyl)phenethyl)piperidine**

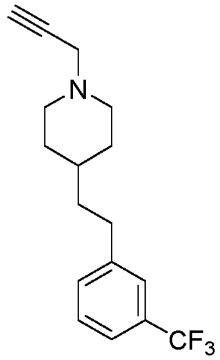

*Step 1:* **Tert-butyl 4-(3-(trifluoromethyl)phenethyl)piperidine-1-carboxylate**

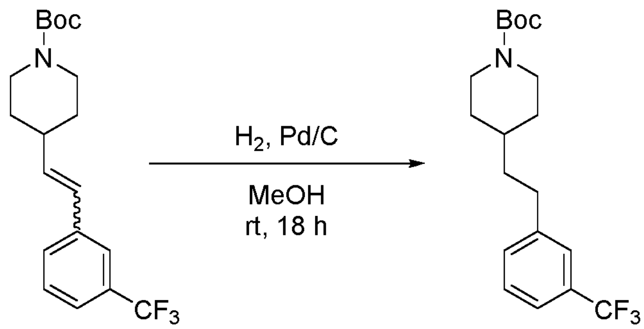


Synthesized from *tert*-butyl (*E*/*Z*)-4-(3-(trifluoromethyl)styryl)piperidine-1-carboxylate (0.27 g, 0.77 mmol, 1.0 equiv.) via general procedure C. The product was used without further purification. Yield: quantitative (274 mg); Rf = 0.58 (EtOAc/n-hex = 1/2, v/v); colorless oil; $^{1}$H NMR (400 MHz, $CDCl_3$): δ 1.13 (dt, *J* = 12.2, 4.2 Hz, 1H), 1.16 (dt, *J* = 12.4, 4.2 Hz, 1H), 1.37-1.45 (m, 1H), 1.46 (s, 9H), 1.55-1.61 (m, 2H), 1.67-1.74 (m, 2H), 2.63-2.72 (m, 4H), 4.09 (bs, 2H), 7.34-7.45 (m, 4H); MS (ESI+): *m/z* $[M+Na]^+$ 380.18; found 380.

*Step 2:* **1-(Prop-2-yn-1-yl)-4-(3-(trifluoromethyl)phenethyl)piperidine**

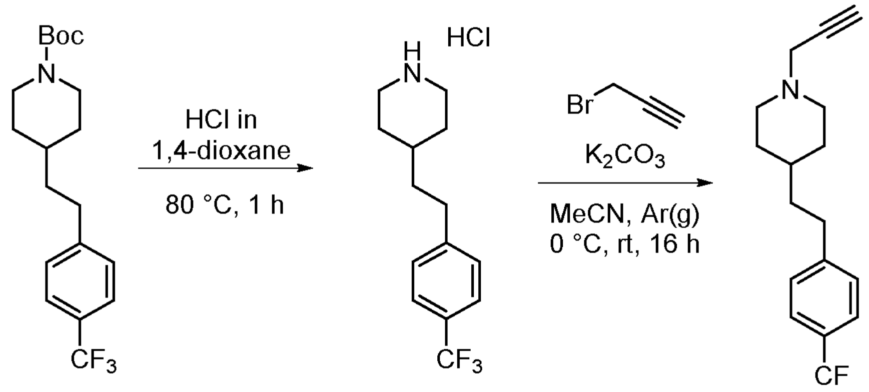


Synthesized from *tert*-butyl 4-(3-(trifluoromethyl)phenethyl)piperidine-1-carboxylate (0.270 g, 0.76 mmol, 1.0 equiv.) via general procedures D and E. The compound was purified by flash column chromatography using EtOAc/n-hex = 1/3 (v/v) as eluent. Yield: 29% (65 mg); Rf = 0.20 (EtOAc/n-hex = 1/3, v/v); yellow-orange transparent oil; $^1H$ NMR (400 MHz, $CDCl_3$): δ 1.21-1.26 (m, 2H), 1.34 (dt, $J$ = 11.1, 3.7 Hz, 1H), 1.54-1.60 (m, 2H), 1.73-1.80 (m, 2H), 2.14-2.21 (m, 2H), 2.22 (t, $J$ = 2.4 Hz, 1H), 2.66-2.70 (m, 2H), 2.86-2.91 (m, 2H), 3.28 (d, $J$ = 2.46 Hz, 2H), 7.33-7.44 (m, 4H); $^{13}C$ NMR (100 MHz, $CDCl_3$): δ 32.10, 32.89, 34.82, 38.03, 47.13, 52.43, 72.86, 79.07, 122.51 (q, $J_{C,F}$ = 3.8 Hz), 124.21 (q, $J_{C,F}$ = 272.2 Hz), 124.88 (q, $J_{C,F}$ = 3.7 Hz), 128.63, 130.51 (q, $J_{C,F}$ = 31.8 Hz), 131.64, 143.48; HRMS (ESI+): *m/z* calcd for $C_{17}H_{21}NF_3$ $[M+H]^+$ 296.1626; found 296.1629; HPLC purity, 98.7%.

EXAMPLE 16

**Synthesis of (*E*)-4-(2-chloro-4-fluorostyryl)-1-(prop-2-yn-1-yl)piperidine**

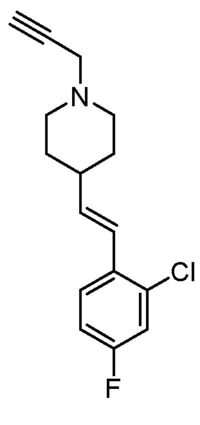

*Step 1:* ***Tert*-butyl (*E*)-4-(2-chloro-4-fluorostyryl)piperidine-1-carboxylate**

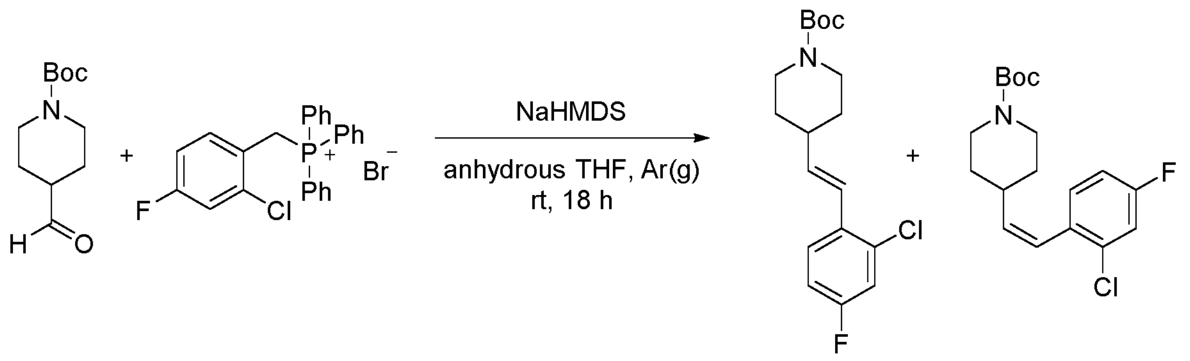


Synthesized from *tert*-butyl 4-formylpiperidine-1-carboxylate (1.50 g, 7.03 mmol, 1.0 equiv.) and 2-chloro-4-fluorobenzyltriphenylphosphonium bromide (3.76 g, 7.74 mmol, 1.1 equiv., prepared via general procedure A) via general procedure B. The compound was purified by flash column chromatography using petroleum ether/$Et_2O$ = 10/1 (v/v) as eluent. Mass: 285 mg; Rf = 0.68 (EtOAc/n-hex = 1/2, v/v); white solid, mp 90-92 °C; $^1H$ NMR (400 MHz, $CDCl_3$): δ 1.36 (dt, *J* = 12.4, 4.1 Hz, 1H), 1.39 (dt, *J* = 12.3, 4.2 Hz, 1H), 1.46 (s, 9H), 1.72-1.79 (m, 2H), 2.27-2.36 (m, 1H), 2.77 (t, *J* = 11.4 Hz, 2H), 4.13 (bs, 2H), 6.04 (dd, *J* = 15.9, 6.9 Hz, 1H), 6.67 (d, *J* = 15.9 Hz, 1H), 6.92 (dt, *J* = 8.4, 2.6 Hz, 1H), 7.07 (dd, *J* = 8.5, 2.6 Hz, 1H), 7.44 (dd, *J* = 8.8, 6.1 Hz, 1H); MS (ESI+): *m*/*z* $[M+Na]^+$ 362.13; found 362.40.

*Step 2:* **(*E*)-4-(2-chloro-4-fluorostyryl)-1-(prop-2-yn-1-yl)piperidine**

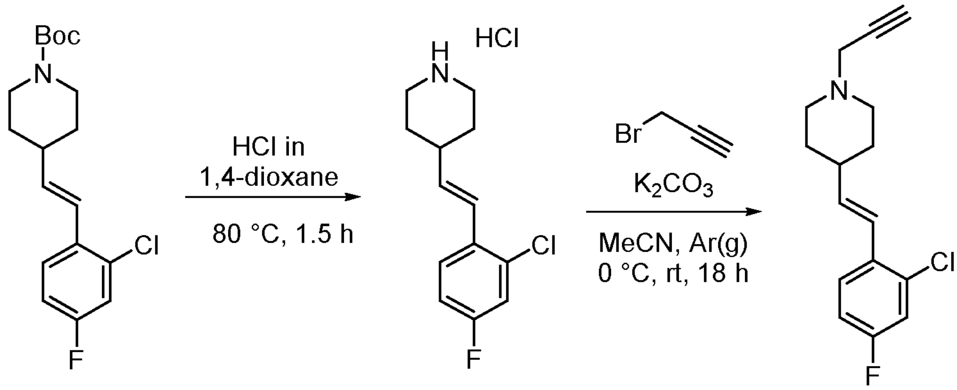


Synthesized from *tert*-butyl (E)-4-(2-chloro-4-fluorostyryl)piperidine-1-carboxylate (263 mg, 0.774 mmol, 1.0 ekviv.) via general procedures D and E. The compound was purified by flash column chromatography using EtOAc/n-hex = 1/2 (v/v) as eluent. Yield: 58% (125 mg); $R_f$ = 0.18 (EtOAc/n-hex = 1/2, v/v); pale yellow transparent oil; $^1H$ NMR (400 MHz, $CDCl_3$): δ 1.56 (dt, *J* = 12.0, 3.9 Hz, 1H), 1.59 (dt, *J* = 11.9, 3.9 Hz, 1H), 1.76-1.85 (m, 2H), 2.12-2.23 (m, 1H), 2.25 (t, *J* = 2.4 Hz, 1H), 2.28 (dt, *J* = 11.7, 2.5 Hz, 2H), 2.90-2.96 (m, 2H), 3.32 (d, *J* = 2.4 Hz, 2H), 6.07 (dd, *J* = 15.9, 7.1 Hz, 1H), 6.68 (d, *J* = 15.9 Hz, 1H), 6.90-6.95 (m, 1H), 7.08 (dd, *J* = 8.5, 2.6 Hz, 1H), 7.47 (dt, *J* = 8.8, 6.1 Hz, 1H); $^{13}C$ NMR (100 MHz, $CDCl_3$): *δ* 31.87, 38.93, 47.18, 52.10, 72.95, 78.99, 114.11 (d, $J_{C,F}$ = 21.3 Hz), 116.57 (d, $J_{C,F}$ = 24.7 Hz), 123.61, 127.49 (d, $J_{C,F}$ = 8.8 Hz), 131.98 (d, $J_{C,F}$ = 3.7 Hz), 133.00 (d, $J_{C,F}$ = 10.2 Hz), 137.51 (d, $J_{C,F}$ = 1.7 Hz), 161.33 (d, $J_{C,F}$ = 249.4 Hz); HRMS (ESI+): *m*/*z* calcd for $C_{16}H_{18}FClN$ $[M+H]^+$ 278.1112; found 278.1120; HPLC purity, 100%.

EXAMPLE 17

**Synthesis of (*E*)-2-(2-(1-(prop-2-yn-1-yl)piperidine-4-yl)vinyl)pyridine**

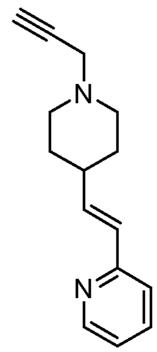

*Step 1:* **Tert-butyl (*E*)-4-(2-(pyridine-2-yl)vinyl)piperidine-1-carboxylate**

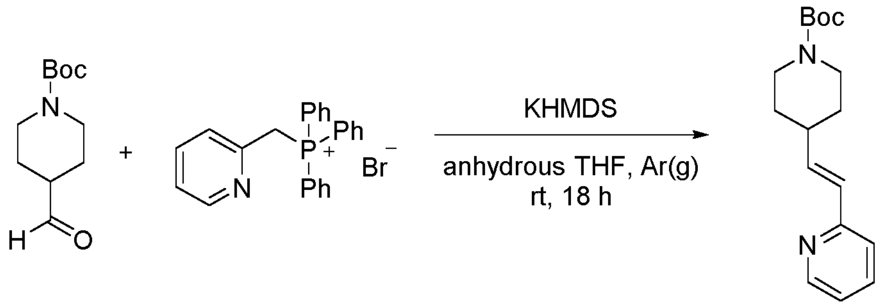


Synthesized from *tert*-butyl 4-formylpiperidine-1-carboxylate (0.46 g, 2.16 mmol, 1.0 equiv.) and triphenyl(pyridine-2-ilmethyl)phosphonium bromide (0.94 g, 2.16 mmol, 1.0 equiv., prepared via general procedure A) via general procedure B. The compound was purified by flash column chromatography using EtOAc/n-hex = 1/2 (v/v) as eluent. Yield: 26% (0.16 g); Rf = 0.19 (EtOAc/n-hex = 1/2, v/v); brown oil; $^1$H NMR (400 MHz, $CDCl_3$): δ 1.36 (dt, *J* = 12.3, 4.1 Hz, 1H), 1.40-1.44 (m, 1H), 1.44 (s, 9H), 1.73-1.80 (m, 2H), 2.27-2.36 (m, 1H), 2.76 (t, *J* = 11.5 Hz, 2H), 4.10 (bs, 2H), 6.45 (dd, *J* = 15.8, 1.2 Hz, 1H), 6.66 (dd, *J* = 15.8, 6.8 Hz, 1H), 7.08 (ddd, *J* = 7.5, 4.8, 1.1 Hz, 1H), 7.21 (td, *J* = 7.9, 0.9 Hz, 1H), 7.58 (dt, *J* = 7.7, 1.8 Hz, 1H), 8.50 (ddd, *J* = 4.8, 1.7, 0.8 Hz, 1H); MS (ESI+): *m/z* $[M+Na]^+$ 311.17; found 311.55.

*Step 2:* **(*E*)-2-(2-(1-(Prop-2-yn-1-yl)piperidine-4-yl)vinyl)pyridine**

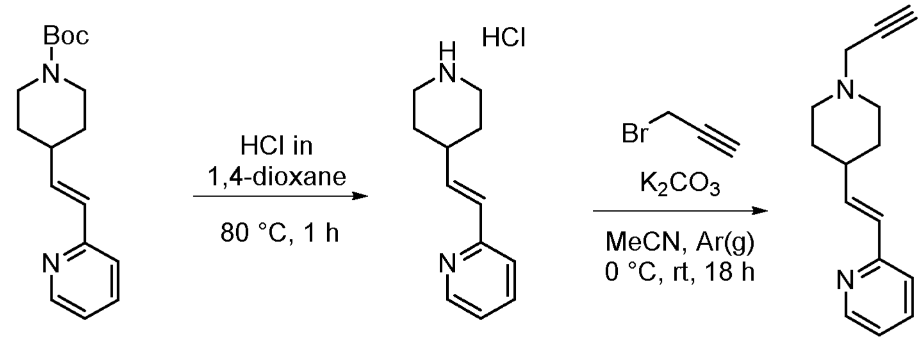


Synthesized from *tert*-butyl (*E*)-4-(2-(pyridine-2-yl)vinyl)piperidine-1-carboxylate (30 mg, 0.10 mmol, 1.0 ekviv.) via general procedures D and E. The compound was purified by flash column chromatography using EtOAc/n-hex = 10/0 (v/v) as eluent. Yield: 48% (11 mg); Rf = 0.07 (EtOAc/n-hex = 2/1, v/v); brown oil; $^1$H NMR (400 MHz, $CDCl_3$): δ 1.56-1.66 (m, 2H), 1.80-1.88 (m, 2H), 2.15-2.24 (m, 1H), 2.25 (t, *J* = 2.4 Hz, 1H), 2.29 (dt, *J* = 11.8, 2.6 Hz, 2H), 2.92-2.96 (m, 2H), 3.33 (d, *J* = 2.4 Hz, 2H), 6.48 (dd, *J* = 15.8, 1.2 Hz, 1H), 6.70 (dd, *J* = 15.8, 7.0 Hz, 1H), 7.10 (ddd, *J* = 7.5, 4.8, 1.1 Hz, 1H), 7.25 (td, *J* = 7.9, 1.0 Hz, 1H), 7.60 (dt, *J* = 7.7, 1.8 Hz, 1H), 8.53 (ddd, *J* = 4.8, 1.8, 0.9 Hz, 1H); $^{13}$C NMR

(100 MHz, $CDCl_3$): $\delta$ 31.69, 38.69, 47.24, 52.22, 73.02, 79.01, 121.27, 121.72, 128.33, 136.45, 139.45, 149.42, 155.90; HRMS (ESI+): *m/z* calcd for $C_{15}H_{19}N_2$ $[M+H]^+$ 227.1548; found 227.1543; HPLC purity, 95.4%.

EXAMPLE 18

**Synthesis of 4-(4-fluorobenzylidene)-1-(prop-2-yn-1-yl)-piperidine**

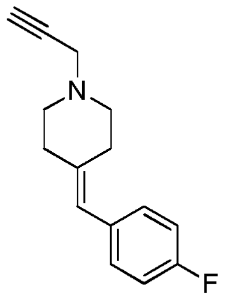

*Step 1:* **Tert-butyl 4-(4-fluorobenzylidene)piperidine-1-carboxylate**

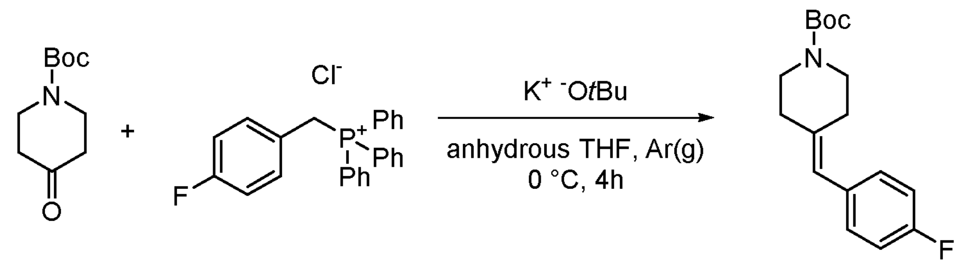


Synthesized from *tert*-butyl 4-oxopiperidine-1-carboxylate (1.00 g, 5.02 mmol, 1.0 equiv.) and 4-(fluorobenzyltriphenyl)phosphonium chloride (0.94 g, 5.02 mmol, 1.0 equiv., prepared via general procedure A) via general procedure B (potassium *tert*-butoxide was used instead of NaHMDS). The compound was purified by flash column chromatography using petroleum ether/$Et_2O$ = 10/1 (v/v) as eluent. Yield: 39% (0.57 g); $R_f$ = 0.12 (petroleum ether/$Et_2O$ = 10/1, v/v); white amorphous solid, mp 50-52 °C; $^1H$ NMR (400 MHz, $CDCl_3$): $\delta$ 1.47 (s, 9H), 2.32 (t, *J* = 5.6 Hz, 2H), 2.41 (t, *J* = 5.6 Hz, 2H), 3.40 (t, *J* = 5.8 Hz, 2H), 3.50 (t, *J* = 5.6 Hz, 2H), 6.31 (s, 1H), 6.97-7.03 (m, 2H), 7.12-7.17 (m, 2H).

*Step 2:* **(4-(4-Fluorobenzylidene)-1-(prop-2-yn-1-yl)-piperidine**

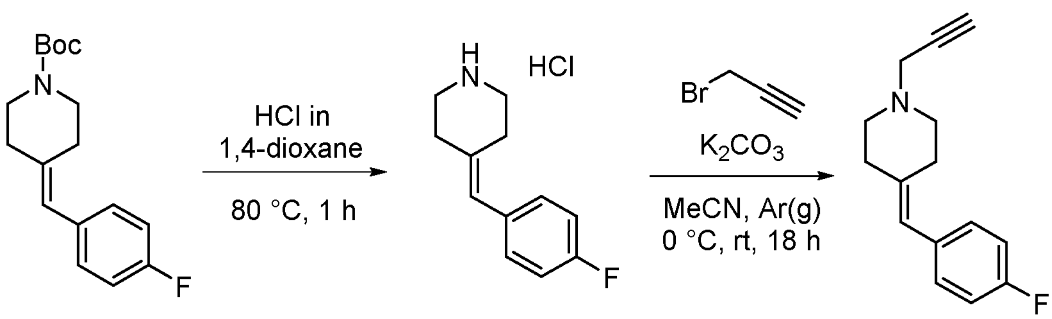


Synthesized from *tert*-butyl 4-(4-fluorobenzylidene)piperidine-1-carboxylate (380 mg, 1.30 mmol, 1.0 ekviv.) via general procedures D and E. The compound was purified by flash column chromatography using EtOAc/n-hex = 1/2

(v/v) as eluent. Yield: 5% (14 mg); Rf = 0.12 (EtOAc/n-hex = 1/2, v/v); yellow-orange transparent oil; $^1$H NMR (400 MHz, $CDCl_3$): δ 2.23 (t, *J* = 2.4 Hz, 1H), 2.40-2.43 (m, 2H), 2.49-2.56 (m, 4H), 2.63-2.66 (m, 2H), 3.33 (d, *J* = 2.4 Hz, 2H), 6.25 (s, 1H), 6.96-7.02 (m, 2H), 7.13-7.16 (m, 2H); $^{13}$C NMR (100 MHz, $CDCl_3$): δ 28.83, 36.17, 46.93, 53.07, 53.80, 73.05, 78.86, 114.91 (d, $J_{C,F}$ = 21.2 Hz), 122.51, 130.37 (d, $J_{C,F}$ = 7.4 Hz), 133.56, 138.62, 161.23 (d, $J_{C,F}$ = 245.2 Hz); HRMS (ESI+): *m/z* calcd for $C_{15}H_{17}FN$ $[M+H]^+$ 230.1345; found 230.1343; HPLC purity, 99.4%.

REFERENCE EXAMPLE 17

**Synthesis of 4-(4-fluorobenzyl)-1-(prop-2-yn-1-yl)-piperidine**

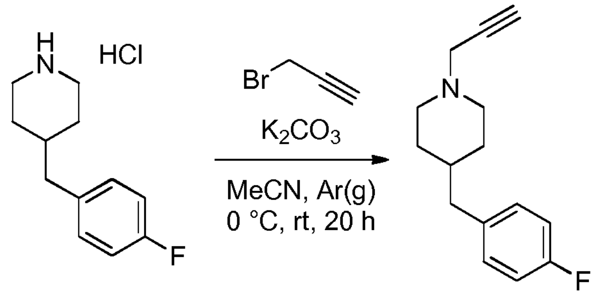


Synthesized from *tert*-butyl 4-(4-fluorobenzyl)piperidinium chloride (380 mg, 1.30 mmol, 1.0 ekviv.) via general procedure E. The compound was purified by flash column chromatography using EtOAc/n-hex = 1/2 (v/v) as eluent. Yield: 4% (10 mg); $R_f$ = 0.10 (EtOAc/n-hex = 1/2, v/v); brown oil; $^1$H NMR (400 MHz, $CDCl_3$): δ 1.28 (dt, J = 11.8, 3.8 Hz, 1H), 1.31 (dt, *J* = 12.3, 3.8 Hz, 1H), 1.41-1.52 (m, 1H), 1.61-1.65 (m, 2H), 2.13 (dt, *J* = 11.6, 2.6 Hz, 2H), 2.20 (t, *J* = 2.4 Hz, 1H), 2.49 (d, *J* = 7.1 Hz, 2H), 2.82-2.87 (m, 2H), 3.26 (d, *J* = 2.5 Hz, 2H), 6.91-6.96 (m, 2H), 7.04-7.09 (m, 2H); $^{13}$C NMR (100 MHz, $CDCl_3$): δ 31.90, 37.36, 42.14, 47.07, 52.37, 72.82, 79.04, 114.80 (d, $J_{C,F}$ = 21.2 20.5 Hz), 130.27 (d, $J_{C,F}$ = 8.1 Hz), 136.05 (d, $J_{C,F}$ = 3,6 Hz), 161.18 (d, $J_{C,F}$ = 243.6 Hz); HRMS (ESI+): *m/z* calcd for $C_{15}H_{19}NF$ $[M+H]^+$ 232.1502; found 232.1506; HPLC purity, 97.5%.

EXAMPLE 19

**Synthesis of (*E*)-4-(3-(4-fluorophenyl)prop-1-en-1-yl)-1-(prop-2-yn-1-yl)piperidine**

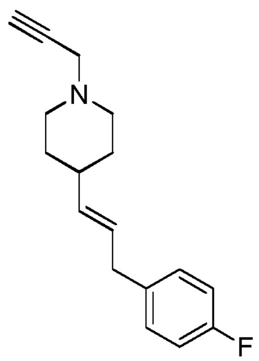

*Step 1:* **Tert-butyl (E)-4-(3-(4-fluorophenyl)prop-1-en-1-yl)piperidine-1-carboxylate**

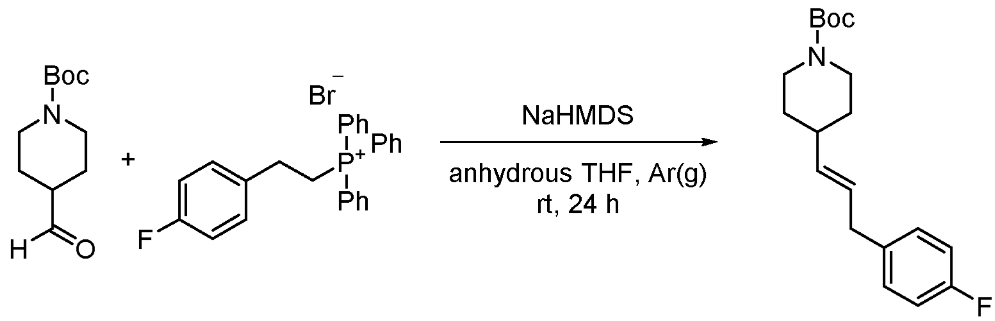


Synthesized from *tert*-butyl 4-formylpiperidine-1-carboxylate (0.50 g, 2.34 mmol, 1.0 equiv.) and (4-fluorophenethyl)triphenylphosphonium bromide (1.31 g, 2.81 mmol, 1.1 equiv., prepared via general procedure A) via general procedure B. The compound was purified by flash column chromatography using petroleum ether/$Et_2O$ = 10/1 (v/v) as eluent. Yield: 47% (0.35 g); $R_f$ = 0.18 (petroleum ether/$Et_2O$ = 10/1, v/v); colorless oil; $^1H$ NMR (400 MHz, $CDCl_3$): δ 1.29 (dt, J= 12.3, 4.1 Hz, 1H), 1.32 (dt, J= 12.3, 4.2 Hz, 1H), 1.44 (s, 9H), 1.55-1.62 (m, 2H), 2.44-2.54 (m, 1H), 2.73 (t, *J* = 11.7 Hz, 2H), 3.35 (d, *J* = 7.5 Hz, 2H), 4.07 (bs, 2H), 5.31 (tdd, *J* = 10.8, 9.3, 1.5 Hz, 1H), 5.45 (dtd, *J* = 10.8, 7.4, 0.9 Hz, 1H), 6.89-6.95 (m, 2H), 7.06-7.11 (m, 2H).

*Step 2:* **(*E*)-4-(3-(4-fluorophenyl)prop-1-en-1-yl)-1-(prop-2-yn-1-yl)piperidine**

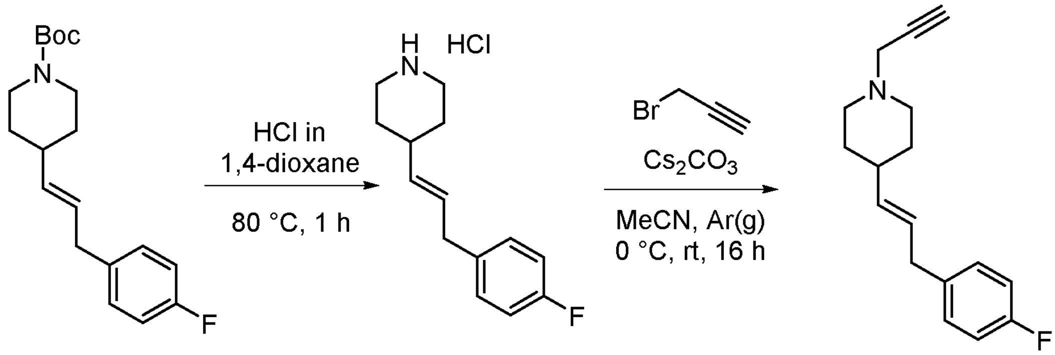


Synthesized from *tert*-butyl (E)-4-(3-(4-fluorophenyl)prop-1-en-1-yl)piperidine-1-carboxylate (220 mg, 0.69 mmol, 1.0 ekviv.) via general procedures D and E. The compound was purified by flash column chromatography using EtOAc/n-hex = 1/2 (v/v) as eluent. Yield: 38% (68 mg); Rf = 0.21 (EtOAc/n-hex = 1/2, v/v); yellow transparent oil; $^1H$ NMR (400 MHz, $CDCl_3$): δ 1.45-1.55 (m, 2H), 1.61-1.70 (m, 2H), 2.22-2.30 (m, 3H), 2.32-2.42 (m, 1H), 2.86-2.91 (m, 2H), 3.31 (d, *J* = 2.4 Hz, 2H), 3.38 (br d, *J* = 7.3 Hz, 2H), 5.34-5.40 (m, 1H), 5.44-5.51 (m, 1H), 6.94-7.00 (m, 2H), 7.09-7.15 (m, 2H); $^{13}C$ NMR (100 MHz, $CDCl_3$): δ 32.28, 32.80, 33.82, 47.28, 52.09, 72.97, 79.02, 115.11 (d, $J_{C,F}$ = 21.1 Hz), 127.05, 129.54 (d, $J_{C,F}$= 7.3 Hz), 135.54, 136.47 (d, $J_{C,F}$ = 2.9 Hz), 161.27 (d, $J_{C,F}$ = 243.6 Hz); HRMS (ESI+): *m*/*z* calcd for $C_{17}H_{21}FN$ $[M+H]^+$ 258.1658; found 258.1652;; HPLC purity, 95.2%.

EXAMPLE 20

**Synthesis of (*E*)-3-(4-fluorostyryl)-1-(prop-2-yn-1-yl)pyrrolidine**

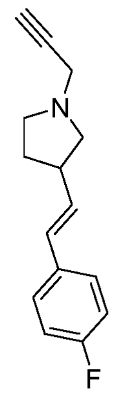

*Step 1:* ***Tert*-butyl (*E*)-3-(4-fluorostyryl)pyrrolidine-1-carboxylate**

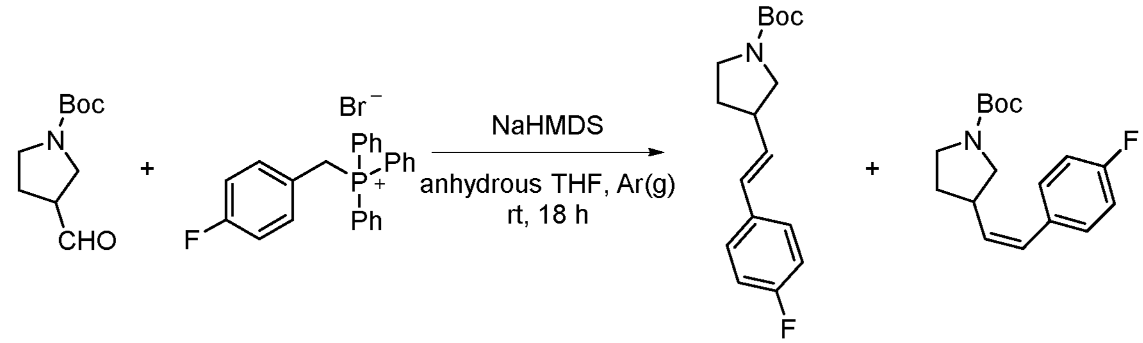


Synthesized from *tert*-butyl 4-formylpyrrolidine-1-carboxylate (1.58 g, 7.93 mmol, 1.0 equiv.) and (4-fluorobenzyl)triphenylphosphonium bromide (3.94g, 8.72 mmol, 1.1 equiv., prepared via general procedure A) via general procedure B. The compound was purified by flash column chromatography using petroleum ether/$Et_2O$ = 9/1 (v/v) as eluent. Mass: 210 mg; Rf = 0.68 (EtOAc/n-hex = 1/2, v/v); white solid, mp 70-72 °C; $^1H$ NMR (400 MHz, $CDCl_3$): δ 1.45 (s, 9H), 1.67-1.80 (m, 1H), 2.04 (dtd, *J* = 9.6, 6.6, 3.2 Hz, 1H), 2.84-2.96 (m, 1H), 3.05-3.16 (m, 1H), 3.25-3.36 (m, 1H), 3.42-3.64 (m, 2H), 6.01 (dd, *J* = 15.9, 7.8 Hz, 1H), 6.39 (d, *J* = 15.9 Hz, 1H), 6.93-6.99 (m, 2H), 7.25-7.30 (m, 2H); MS (ESI+): *m/z* $[M+Na]^+$ 314.15; found 314.37.

*Step 2:* **(*E*)-3-(4-Fluorostyryl)-1-(prop-2-yn-1-yl)pyrrolidine**

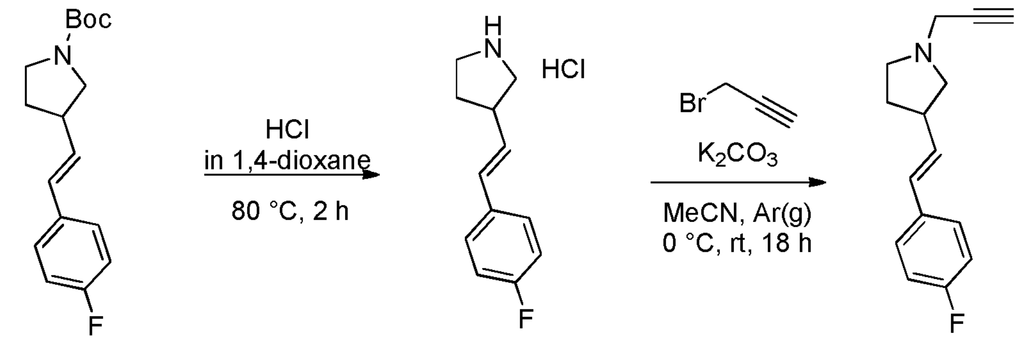


Synthesized from *tert*-butyl ((E)-3-(4-fluorostyryl)pyrrolidine-1-carboxylate (0.15 g, 0.515 mmol, 1.0 equiv.) via general procedures D and E. The compound was purified by flash column chromatography using EtOAc/n-hex = 2/1 (v/v) as eluent. Yield: 65% (77 mg); Rf = 0.18 (EtOAc/n-hex = 1/2, v/v); yellow transparent oil; $^1H$ NMR (400 MHz, $CDCl_3$): δ 1.66-1.74 (m, 1H), 2.11-2.19 (m, 1H), 2.23 (t, *J* = 2.4 Hz, 1H), 2.50 (dd, *J* = 8.6, 6.7 Hz, 1H), 2.72 (dt, *J* = 8.8, 5.5 Hz,

1H), 2.77-2.83 (m, 1H), 2.92-3.02 (m, 2H), 3.44 (d, *J* = 2.4 Hz, 2H), 6.12 (dd, *J* = 15.8, 8.1 Hz, 1H), 6.34 (d, *J* = 15.8 Hz, 1H), 6.94-7.00 (m, 2H), 7.26-7.32 (m, 2H); $^{13}C$ NMR (100 MHz, $CDCl_3$): $\delta$ 31.68, 41.46, 42.79, 52.13, 58.40, 72.55, 79.21, 115.32 (d, $J_{C,F}$ = 21.1 Hz), 127.44 (d, $J_{C,F}$ = 8.1 Hz), 128.02, 133.11 (d, $J_{C,F}$ = 2.2 Hz), 133.48, 161.96 (d, $J_{C,F}$ = 245.7 Hz); HRMS (ESI+): *m*/*z* calcd for $C_{15}H_{17}FN$ $[M+H]^+$ 230.1345; found 230.1340; HPLC purity, 95.3%.

EXAMPLE 21

**Synthesis of (*E*)-4-(2-(4-fluorophenyl)ethylidene)-1-(prop-2-yn-1-yl)azepane and (*Z*)-4-(2-(4-fluorophenyl)ethylidene)-1-(prop-2-yn-1-yl)azepane**

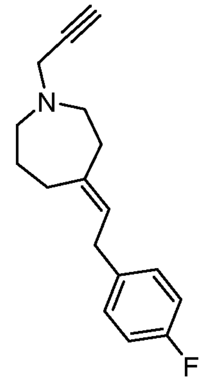

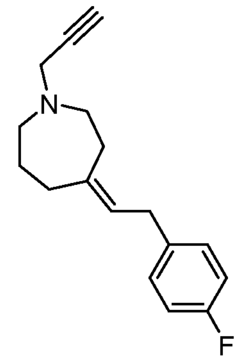

*Step 1:* **Tert-butyl (E/Z)-4-(2-(4-fluorophenyl)ethylidene)azepan-1-carboxylate**

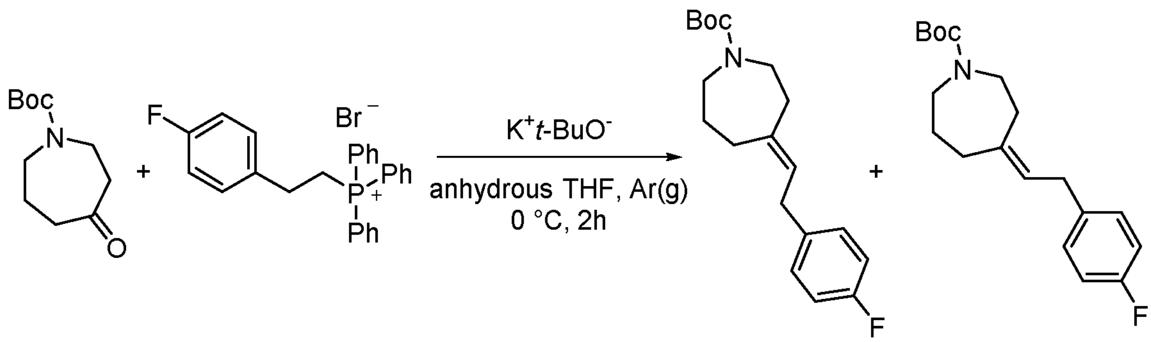


The solution of (4-fluorophenethyl)triphenylphosphonium bromide (2.80 g, 6.00 mmol, 1.0 equiv.) in anhydrous THF (15 mL) was purged under a stream of argon for 5 min and cooled to 0 °C. To the resulting solution $t\text{-}BuO^-K^+$ (0.70 g, 6.30 mmol, 1.05 equiv.) was added, and stirred for 30 min before the solution of *tert*-butyl 4-oxoazepane-1-carboxylate (1.28 g, 6.00 mmol, 1.0 equiv.) in THF (10 mL) was added. The reaction mixture was stirred for 2 h at 0 °C, and then the solvent was evaporated under reduced pressure. The crude product was purified by flash column chromatography using EtOAc/n-hex = 1/9 (v/v) as eluent to obtain a mixture of *cis*/*trans* (ratio ~1/1, estimared from $^1H$ NMR) isomers. Overall yield (both isomers): 90% (1.728 g). colorless oil; Rf = 0.24 (EtOAc/n-hex = 1/9, v/v); $^1H$ NMR (400 MHz, $CDCl_3$): $\delta$ 1.40 (s, 9H), 1.41 (s, 9H), 1.63 (bs, 4H, *cis* + *trans),* 2.19-2.22 (m, 2H, *cis* + *trans),* 2.24-2.28 (m, 2H, *cis* + *trans),* 2.35 (t, J = 6.2 Hz, 2H, *cis* + *trans),* 2.46 (bs, 2H, *cis* + *trans),* 3.25-3.29 (m, 4H, *cis* + *trans),* 3.30-3.41 (m, 8H, *cis* + *trans),* 5.27-5.32 (m, 1H, *trans*), 5.37 (t, J = 6.7 Hz, 1H, *cis),* 6.87-6.93 (m, 4H, *cis* + *trans),* 7.04-7.09 (m, 4H, *cis* + *trans*); MS (ESI+): m/z [M+H]+ 342.18; found 342.01.

*Step 2:* **(*E*)-4-(2-(4-Fluorophenyl)ethylidene)-1-(prop-2-yn-1-yl)azepane and (*Z*)-4-(2-(4-fluorophenyl)ethylidene)-1-(prop-2-yn-1-yl)azepane**

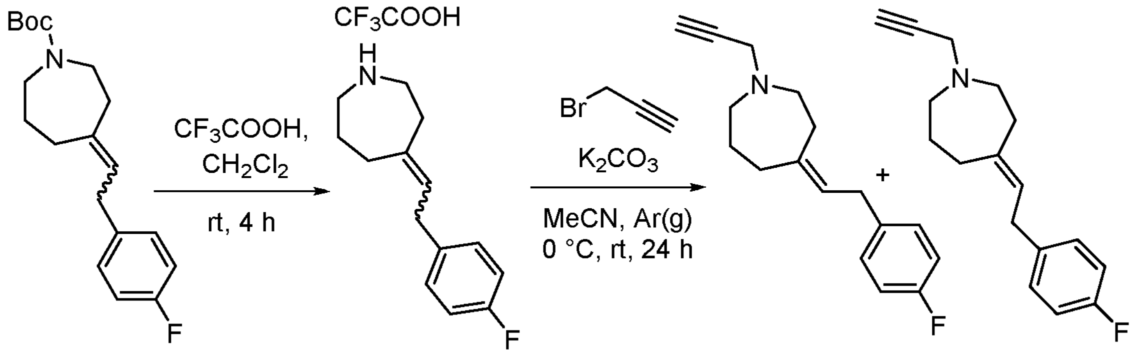


Synthesized from *tert*-butyl (E/Z)-4-(2-(4-fluorophenyl)ethylidene)azepan-1-carboxylate (1.20 g, 3.757 mmol, 1.0 equiv.) via general procedures D and E. The compounds were purified by flash column chromatography using $CH_2Cl_2$/acetone = 20/1 (v/v) as eluent to obtain both isomers.

*(Z)-4-(2-(4-Fluorophenyl)ethylidene)-1-(prop-2-yn-1-yl)azepane*: 82 mg, $R_f$ = 0.38 ($CH_2Cl_2$/acetone = 9/1, v/v); pale yellow transparent oil; $^1$H NMR (400 MHz, $CDCl_3$): δ 1.70-1.76 (m, 2H), 2.22 (t, *J* = 2.4 Hz, 1H), 2.31-2.34 (m, 2H), 2.48-2.51 (m, 2H), 2.67-2.69 (m, 2H), 2.73-2.76 (m, 2H), 3.29 (d, *J* = 7.2 Hz, 2H), 3.40 (d, *J* = 2.4 Hz, 2H), 5.35 (tt, *J* = 7.2, 1.2 Hz, 1H), 6.92-6.97 (m, 2H), 7.11-7.14 (m, 2H); $^{13}$C NMR (100 MHz, $CDCl_3$): δ 28.71, 30.54, 32.96, 36.49, 48.10, 53.58, 56.38, 72.36, 79.59, 114.96 (d, $J_{C,F}$ = 21.1 Hz), 123.73, 129.55 (d, $J_{C,F}$ = 7.9 Hz), 136.97 (d, $J_{C,F}$ = 3.3 Hz); 140.24, 161.13 (d, $J_{C,F}$ = 243.4 Hz); HRMS (ESI+): m/z calcd for $C_{17}H_{21}FN$ $[M+H]^+$ 258.1658; found 258.1653; .HPLC purity, 95.5%.

*(E)-4-(2-(4-Fluorophenyl)ethylidene)-1-(prop-2-yn-1-yl)azepane:* 29 mg, $R_f$ = 0.32 ($CH_2Cl_2$/acetone = 9/1, v/v); pale yellow transparent oil; $^1$H NMR (400 MHz, $CDCl_3$): δ 1.78-1.84 (m, 2H), 2.23 (t, *J* = 2.4 Hz, 1H), 2.40-2.45 (m, 4H), 2.67-2.72 (m, 4H), 3.29 (d, *J* = 7.3 Hz, 2H), 3.39 (d, *J* = 2.4 Hz, 2H), 5.35 (tt, *J* = 7.2, 1.4 Hz, 1H), 6.92-6.98 (m, 2H), 7.10-7.15 (m, 2H); $^{13}$C NMR (100 MHz, $CDCl_3$): *δ*26.54, 29.26, 32.99, 38.03, 48.00, 56.17, 56.78, 72.62, 79.43, 115.00 (d, $J_{C,F}$ = 21.3 Hz), 124.05, 129.55 (d, $J_{C,F}$ = 7.4 Hz), 136.96 (d, $J_{C,F}$ = 2.9 Hz); 140.45, 161.16 (d, $J_{C,F}$ = 243.5 Hz); HRMS (ESI+): *m/z* calcd for $C_{17}H_{21}FN$ $[M+H]^+$ 258.1658; found 258.1651; HPLC purity, 97.4%.

REFERENCE EXAMPLE 18

**Synthesis of 4-(4-fluorophenethyl)-1-(prop-2-yn-1-yl)azepane**

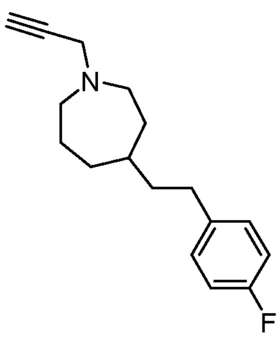

*Step 1:* **Tert-butyl 4-(4-fluorophenethyl)azepane-1-carboxylate**

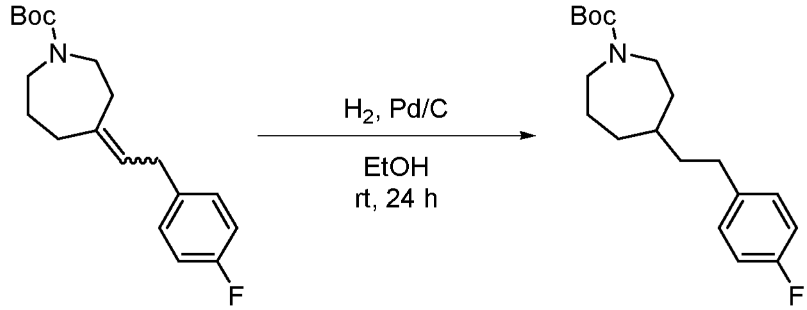


Synthesized from *tert*-butyl (*E*/*Z*)-4-(2-(4-fluorophenyl)ethylidene)azepan-1-carboxylate (0.41 g, 1.284 mmol, 1.0 equiv.) via general procedure C. The product was used without further purification. Yield: quantitative (0.414 g); $R_f$ = 0.82 (EtOAc/n-hex = 1/1, v/v); colorless oil; $^1$H NMR (400 MHz, DMSO-$d_6$, 80 °C): δ 1.16-1.35 (m, 3H), 1.40 (s, 9H), 1.44-1.55 (m, 3H), 1.68-1.85 (m, 3H), 2.57-2.61 (m, 2H), 3.14-3.27 (m, 2H), 3.34-3.48 (m, 2H), 7.01-7.07 (m, 2H), 7.18-7.23 (m, 2H); MS (ESI+): *m*/*z* $[M+H]^+$ 344.20; found 344.05.

*Step 2:* **4-(4-Fluorophenethyl)-1-(prop-2-yn-1-yl)azepane**

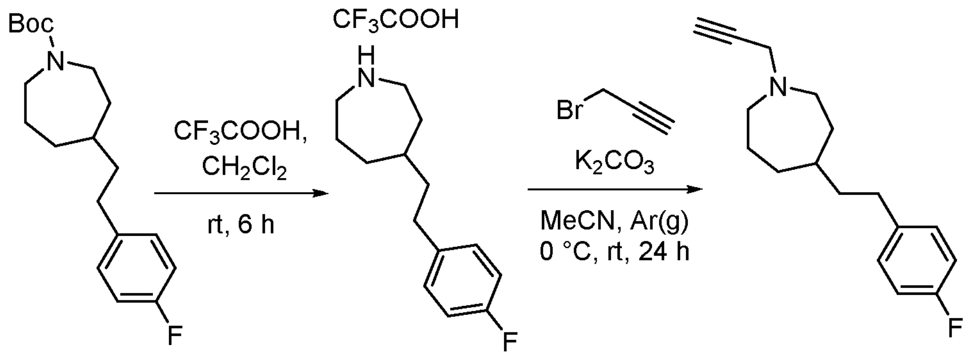


Synthesized from *tert*-butyl 4-(4-fluorophenethyl)azepane-1-carboxylate (0.370 g, 1.151 mmol, 1.0 equiv.) via general procedures D and E. The compound was purified by flash column chromatography using EtOAc/n-hex = 1/1 (v/v) as eluent. Yield: 32% (84 mg); $R_f$ = 0.26 (EtOAc/n-hex = 1/1, v/v); pale yellow transparent oil; $^1$H NMR (400 MHz, $CDCl_3$): δ 1.30-1.63 (m, 6H), 1.75-1.84 (m, 3H), 2.19 (t, *J* = 2.4 Hz, 1H), 2.54-2.59 (m, 2H), 2.61 (dd, *J* = 9.6, 3.0 Hz, 1H), 2.67-2.70 (m, 2H), 2.75 (ddd, *J* = 12.9, 6.9, 3.2 Hz, 1H), 3.36 (t, *J* = 2.3 Hz, 2H), 6.91-6.97 (m, 2H), 7.07-7.12 (m, 2H); $^{13}$C NMR (100 MHz, $CDCl_3$): *δ* 26.60, 32.74, 32.82, 34.36, 37.45, 39.79, 47.95, 53.13, 55.35, 72.08, 79.97, 114.90 (d, $J_{C,F}$ = 21.3 Hz), 129.50 (d, $J_{C,F}$ = 8.0 Hz), 138.31 (d, $J_{C,F}$ = 3.7 Hz); 161.03 (d, $J_{C,F}$ = 243.5 Hz); HRMS (ESI+): *m*/*z* calcd for $C_{17}H_{23}FN$ $[M+H]^+$ 260.1815; found 260.1809; HPLC purity, 98.2%.

EXAMPLE 22

**Synthesis of (*E*)-4-(2-(4-fluorobenzylidene)-1-(prop-2-yn-1-yl)azepane and (*Z*)-4-(2-(4-fluorobenzylidene)-1-(prop-2-yn-1-yl)azepane**

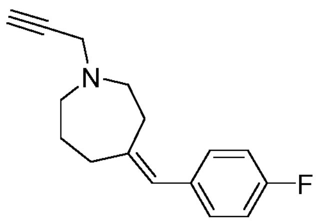

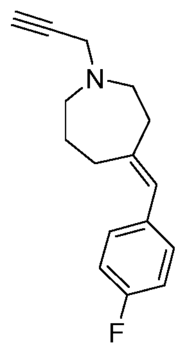

*Step 1:* **Tert-butyl (*E*/*Z*)-4-(2-(4-fluorobenzylidene)azepan-1-carboxylate**

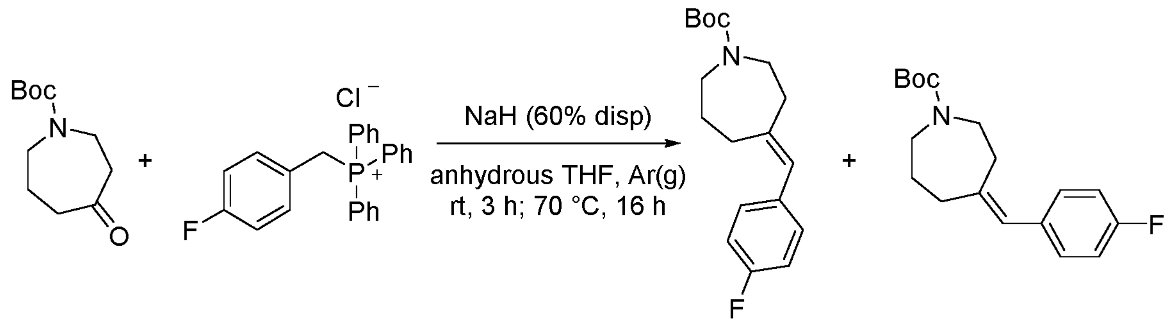


The solution of (4-fluorobenzyl)triphenylphosphonium chloride (2.71 g, 6.66 mmol, 1.1 equiv.) in anhydrous THF (20 mL) was purged under a stream of argon for 5 min before NaH (60% dispersion on mineral oil, 0.27 g, 6.66 mmol, 1.1 equiv.) was added. The resulting suspension was stirred for 3 h at room temperature, and then the solution of *tert*-butyl 4-oxoazepane-1-carboxylate (1.29 g, 6.05 mmol, 1.0 equiv.) in THF (15 mL) was added. The reaction mixture was stirred under reflux for 16 h, and then allowed to cool down to room temperature. The precipitate formed was filtered off, and the mother liquor was evaporated under reduced pressure. The resulting residue was purified by flash column chromatography using petroleum ether/$Et_2O$ = 9/1 (v/v) as eluent to obtain a mixture of cis/trans isomers (ratio ~ 42/58, estimated from $^1H$ NMR). Overall yield (both isomers): 35% (649 mg), colorless oil; $R_f$ = 0.12 (petroleum ether/$Et_2O$ = 9/1, v/v); $^1H$ NMR (400 MHz, $CDCl_3$): δ 1.29 (s, 5H, *cis),* 1.43 (s, 4H, *cis),* 1.45 (s, 12.5H, *trans),* 1.63 (bs, 4.8H, *cis* + *trans),* 2.34-2.37 (m, 4.8H, *cis* + *trans),* 2.50 (t, *J* = 6.1 Hz, 2.8H, *trans),* 2.58 (td, *J* = 6.3, 1.3 Hz, 2H, *cis*), 3.40-3.48 (m, 9.8H, *cis* + *trans),* 6.28 (d, *J* = 6.9 Hz, 1.4H, *trans),* 6.34 (bs, 1H, *cis*), 6.96-7.01 (m, 4.8H, *cis* + *trans),* 7.13-7.16 (m, 4.8H, *cis* + *trans*); MS (ESI+): *m/z* $[M+Na]^+$ 328.17; found 327.77.

*Step 2:* **(*E*)-4-(2-(4-Fluorobenzylidene)-1-(prop-2-yn-1-yl)azepane and (*Z*)-4-(2-(4-fluorobenzylidene)-1-(prop-2-yn-1-yl)azepane**

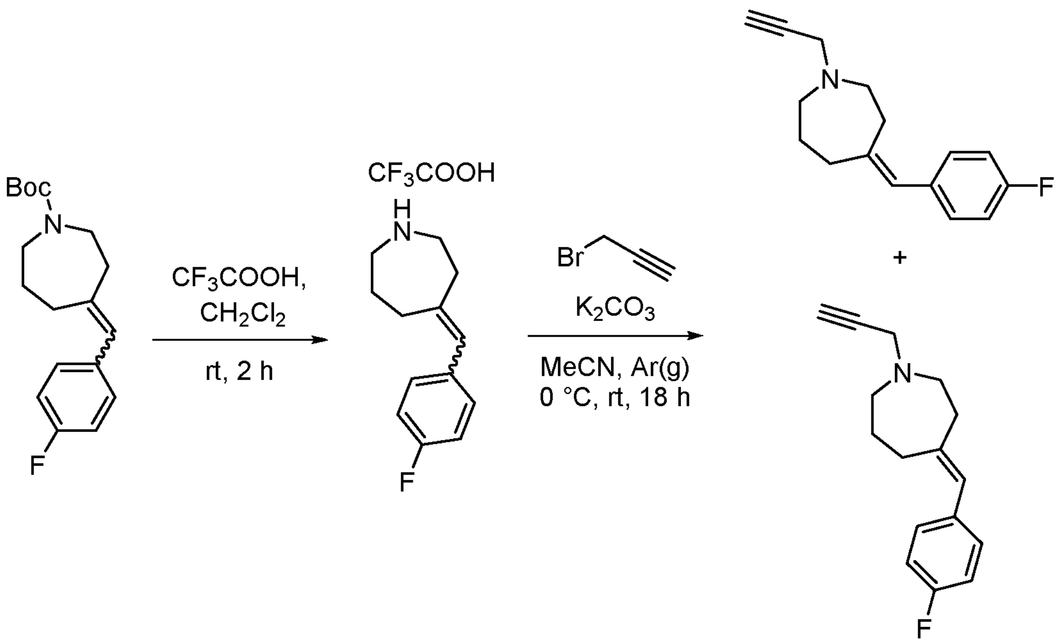


Synthesized from *tert*-butyl (E/Z)-4-(2-(4-fluorobenzylidene)azepan-1-carboxylate (270 mg, 0.884 mmol, 1.0 equiv.) via general procedures D and E. The compounds were purified by flash column chromatography using $CH_2Cl_2$/acetone = 20/1 (v/v) as eluent to obtain both isomers.

*(Z)-4-(4-fluorobenzylidene)-1-(prop-2-yn-1-yl)azepane:* 9 mg, $R_f$ = 0.52 ($CH_2Cl_2$/acetone = 9/1, v/v); colorless oil; $^1H$ NMR (400 MHz, $CDCl_3$): δ 1.80-1.86 (m, 2H), 2.23 (t, $J$ = 2.4 Hz, 1H), 2.47-2.50 (m, 2H), 2.63-2.66 (m, 2H), 2.74-2.76 (m, 2H), 2.78-2.80 (m, 2H), 3.41 (d, $J$ = 2.4 Hz, 2H), 6.27 (s, 1H), 6.96-7.02 (m, 2H), 7.14-7.19 (m, 2H); $^{13}C$ NMR (100 MHz, $CDCl_3$): $\delta$ 28.24, 31.65, 37.14, 48.07, 53.82, 56.67, 72.88, 79.09, 114.86 (d, $J_{C,F}$ = 21.2 Hz), 124.65, 130.18 (d, $J_{C,F}$ = 7.9 Hz), 134.14 (d, $J_{C,F}$ = 3.1 Hz); 142.44, 161.10 (d, $J_{C,F}$ = 245.0 Hz); HRMS (ESI+): *m/z* calcd for $C_{16}H_{19}FN$ $[M+H]^+$ 244.1502; found 244.1507; HPLC purity, 97.7%.

*(E)-4-(4-fluorobenzylidene)-1-(prop-2-yn-1-yl)azepane:* 16 mg, $R_f$ = 0.35 ($CH_2Cl_2$/acetone = 9/1, v/v); pale yellow transparent oil; $^1H$ NMR (400 MHz, $CDCl_3$): δ 1.82-1.88 (m, 2H), 2.24 (t, $J$ = 2.4 Hz, 1H), 2.54-2.60 (m, 4H), 2.70-2.72 (m, 2H), 2.78-2.81 (m, 2H), 3.42 (d, $J$ = 2.4 Hz, 2H), 6.25 (s, 1H), 6.96-7.02 (m, 2H), 7.16-7.21 (m, 2H); $^{13}C$ NMR (100 MHz, $CDCl_3$): $\delta$ 26.70, 30.62, 38.68, 47.94, 55.81, 56.45, 72.91, 79.16, 114.85 (d, $J_{C,F}$ = 21.2 Hz), 124.90, 130.16 (d, $J_{C,F}$ = 7.8 Hz), 134.18 (d, $J_{C,F}$ = 3.5 Hz); 142.68, 161.08 (d, $J_{C,F}$ = 245.5 Hz); HRMS (ESI+): *m/z* calcd for $C_{16}H_{19}FN$ $[M+H]^+$ 244.1502; found 244.1505; HPLC purity, 96.9%.

REFERENCE EXAMPLE 19

**Synthesis of 4-(4-fluorobenzyl)-1-(prop-2-yn-1-yl)azepane**

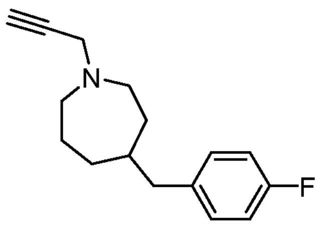

*Step 1:* **Tert-butyl 4-(4-fluorobenzyl)azepane-1-carboxylate**

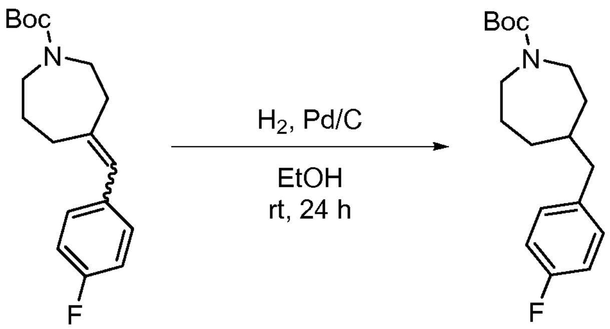


Synthesized from *tert*-butyl (E/Z)-4-(2-(4-fluorobenzylidene)azepan-1-carboxylate (0.315 g, 1.031 mmol, 1.0 equiv.) via general procedure C. The product was used without further purification. Yield: quantitative (0.317 g); $R_f$ = 0.74 (EtOAc/n-hex = 1/1, v/v); colorless oil; $^1H$ NMR (400 MHz, $CDCl_3$): δ 1.10-1.20 (m, 1H), 1.25-1.34 (m, 1H), 1.45 (s, 9H), 1.47-1.56 (m, 1H), 1.62-1.85 (m, 4H), 2.50 (d, *J* = 7.3 Hz, 2H), 3.09 (ddd, *J* = 13.9, 10.2, 3.8 Hz, 1H), 3.30-3.43 (m, 2H), 3.56 (d, *J* = 13.6 Hz, 1H), 6.92-6.98 (m, 2H), 7.05-7.11 (m, 2H); MS (ESI+): *m/z* $[M+Na]^+$ 330.18; found 329.81.

*Step 2:* **4-(4-Fluorobenzyl)-1-(prop-2-yn-1-yl)azepane**

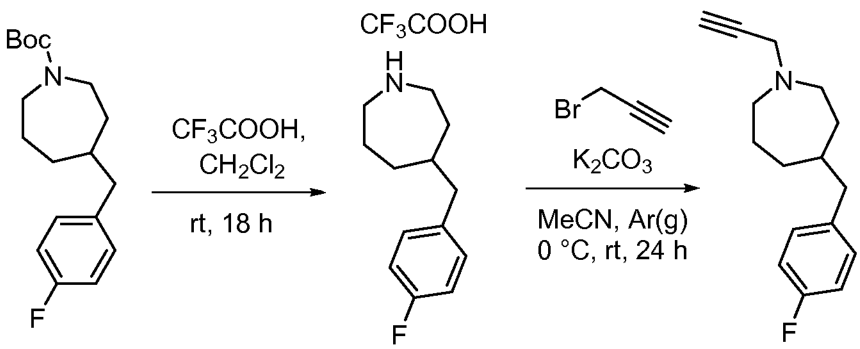


Synthesized from *tert*-butyl 4-(4-fluorobenzyl)azepane-1-carboxylate (0.290 g, 0.943 mmol, 1.0 equiv.) via general procedures D and E. The compound was purified by flash column chromatography using EtOAc/n-hex = 1/1 (v/v) as eluent. Yield: 56% (110 mg); Rf = 0.27 (EtOAc/n-hex = 1/1, v/v); colorless oil; $^1H$ NMR (400 MHz, $CDCl_3$): δ 1.25-1.45 (m, 2H), 1.53-1.59 (m, 1H), 1.61-1.87 (m, 4H), 2.18 (t, *J* = 2.4 Hz, 1H), 2.49 (d, *J* = 7.4 Hz, 2H), 2.57 (ddd, *J* = 12.9, 9.8, 3.1 Hz, 1H), 2.67-2.70 (m, 2H), 2.72 (ddd, *J* = 12.9, 6.5, 3.4 Hz, 1H), 3.34 (dd, *J* = 3.8, 2.4 Hz, 2H), 6.90-6.96 (m, 2H), 7.05-7.10 (m, 2H); $^{13}C$ NMR (100 MHz, $CDCl_3$): *δ* 26.55, 32.60, 34.05, 40.08, 43.15, 47.91, 52.94, 55.10, 72.10, 79.89, 114.78 (d, $J_{C,F}$ = 20.8 Hz), 130.31 (d, $J_{C,F}$ = 8.0 Hz), 136.76 (d, $J_{C,F}$ = 3.0 Hz); 161.11 (d, $J_{C,F}$ = 243.4 Hz); HRMS (ESI+): *m/z* calcd for $C_{16}H_{21}FN$ $[M+H]^+$ 246.1658; found 246.1660; IR (ATR): 3303, 2917, 2848, 1601, 1509, 1468, 1449, 1326, 1221, 1157, 1102, 835, 761, 663, 636 $cm^{-1}$; HPLC purity, 97.4%.

EXAMPLE 23

**Synthesis of (*E*)-1-(but-3-yn-1-yl)-4-(4-fluorostyryl)piperidine**

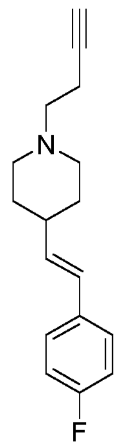

*Step 1:* **But-3-yn-1-yl methanesulfonate**

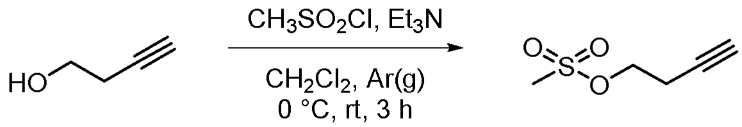


The activated primary alcohol intermediate (but-3-yn-1-yl methanesulfonate) was synthesized from but-3-yn-1-ol (0.227 mL, 3.00 mmol, 1.0 equiv.) via general procedure H. The product was used without further purification. Yield: 89% (395 mg); Rf = 0.15 (EtOAc/n-hex = 1/4, v/v); pale yellow transparent oil.

*Step 2:* **(*E*)-1-(But-3-yn-1-yl)-4-(4-fluorostyryl)piperidine**

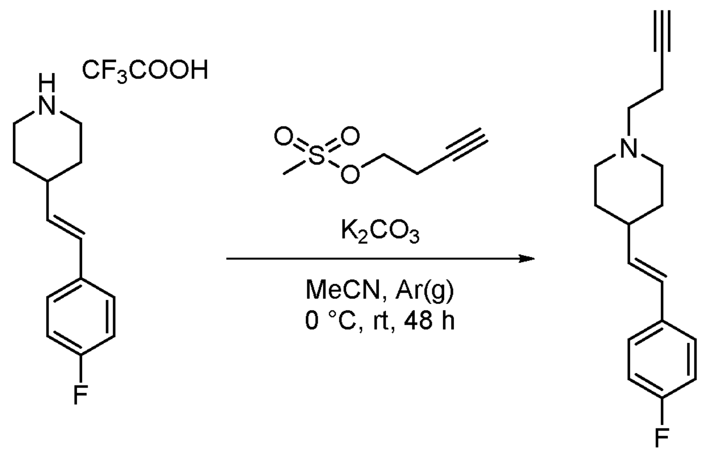


The titled compound synthesized from (*E*)-4-(4-fluorostyryl)piperidine 2,2,2-trifluoroacetate (0.122 g, 0.382 mmol, 1.0 equiv.; prepared as described under the general procedure E) via general procedure G using previously prepared but-3-yn-1-yl methanesulfonate instead of propargyl bromide. The crude product was purified by flash column chromatography using EtOAc/n-hex = 2/1 (v/v) as eluent. Yield: 43% (42 mg); $R_f$ = 0.34 (EtOAc/n-hex = 2/1, v/v); pale orange solid, mp 44-46 °C; $^1$H NMR (400 MHz, $CDCl_3$): δ 1.52 (dt, *J* = 12.5, 3.2 Hz, 1H), 1.55 (dt, *J* = 12.0, 3.6 Hz, 1H), 1.74-1.79 (m, 2H), 1.99 (t, *J* = 2.7 Hz, 1H), 2.06-2.15 (m, 3H), 2.39-2.43 (m, 2H), 2.60-2.64 (m, 2H), 2.97 (dt, *J* = 11.6, 3.6 Hz, 2H), 6.06 (dd, *J* = 16.0, 7.0 Hz, 1H), 6.33 (d, *J* = 16.0 Hz, 1H), 6.94-7.00 (m, 2H), 7.27-7.32 (m, 2H); $^{13}$C NMR (100 MHz, $CDCl_3$): δ 16.77, 31.92, 39.11, 53.24, 57.35, 69.02, 82.81, 115.30 (d, $J_{C,F}$ = 21.4 Hz), 127.09, 127.40 (d, $J_{C,F}$ = 7.9 Hz), 133.79 (d, $J_{C,F}$ = 3.2 Hz), 134.66, 161.97 (d, $J_{C,F}$ = 245.8 Hz); HRMS (ESI+): *m/z* calcd for $C_{17}H_{21}FN$ [M+H]

+ 258.1658; found 258.1654; HPLC purity, 98.9%.

REFERENCE EXAMPLE 20

**Synthesis of (*E*)-1-(but-3-yn-1-yl)-4-(4-fluorophenethyl)piperidine**

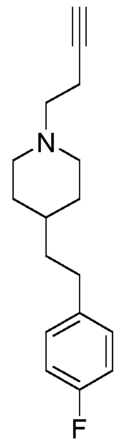

*Step 1:* **But-3-yn-1-yl methanesulfonate**

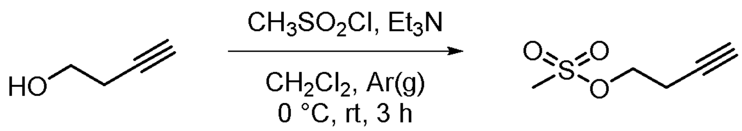


The activated primary alcohol intermediate (but-3-yn-1-yl methanesulfonate) was synthesized from but-3-yn-1-ol (0.227 mL, 3.00 mmol, 1.0 equiv.) via general procedure H. The product was used without further purification. Yield: 89% (395 mg); Rf = 0.15 (EtOAc/n-hex = 1/4, v/v); pale yellow transparent oil.

*Step 2:* **(*E*)-1-(but-3-yn-1-yl)-4-(4-fluorostyryl)piperidine**

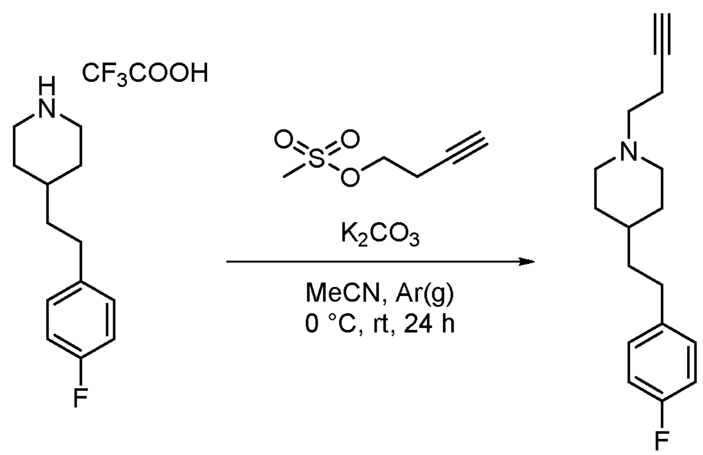


The titled compound synthesized from 4-(4-fluorophenethyl)piperidine 2,2,2-trifluoroacetate (0.160 g, 0.498

mmol, 1.0 equiv.; prepared as described under the general procedure E) via general procedure G using previously prepared but-3-yn-1-yl methanesulfonate. The crude product was purified by flash column chromatography using EtOAc/n-hex = 1/1 (v/v) as eluent. Yield: 33% (42 mg); $R_f$ = 0.20 (EtOAc/n-hex = 1/1, v/v); orange transparent oil; $^1$H NMR (400 MHz, $CDCl_3$): δ 1.22-1.34 (m, 3H), 1.50-1.55 (m, 2H), 1.70-1.73 (m, 2H), 1.97 (t, $J$ = 2.7 Hz, 1H), 1.95-2.01 (m, 2H), 2.36-2.41 (m, 2H), 2.56-2.61 (m, 4H), 2.90-2.93 (m, 2H), 6.91-6.97 (m, 2H), 7.08-7.13 (m, 2H); $^{13}$C NMR (100 MHz, $CDCl_3$): $\delta$ 16.67, 32.05, 32.18, 35.02, 38.38, 53.51, 57.33, 68.96, 82.81, 114.95 (d, $J_{C,F}$ = 20.6 Hz), 129.51 (d, $J_{C,F}$ = 8.1 Hz), 138.16 (d, $J_{C,F}$ = 3.6 Hz), 161.08 (d, $J_{C,F}$ = 243.1 Hz); HRMS (ESI+): *m/z* calcd for $C_{17}H_{23}FN$ $[M+H]^+$ 260.1815; found 260.1808; IR (ATR): 3311, 2928, 2849, 1509, 1448, 1309, 1219, 1157, 1087, 892, 823, 640 $cm^{-1}$; HPLC purity, 100%.

EXAMPLE 24

**Synthesis of (*E*)-1-(pent-4-yn-1-yl)-4-(4-fluorophenethyl)piperidine**

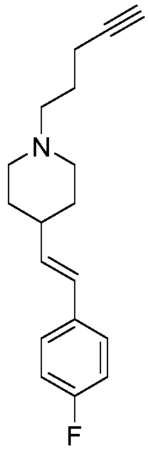

*Step 1:* **Pent-4-yn-1-yl methanesulfonate**

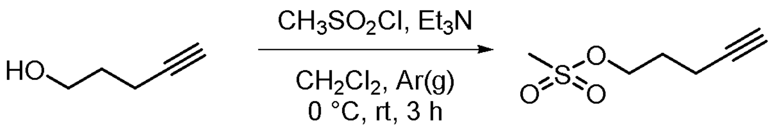


The activated primary alcohol intermediate (pent-4-yn-1-yl methanesulfonate) was synthesized from pent-4-yn-1-ol (0.464 mL, 5.00 mmol, 1.0 equiv.) via general procedure H. The product was used without further purification. Yield: 46% (376 mg); Rf = 0.17 (EtOAc/n-hex = 1/4, v/v); colorless oil.

*Step 2:* **(*E*)-1-(Pent-4-yn-1-yl)-4-(4-fluorostyryl)piperidine**

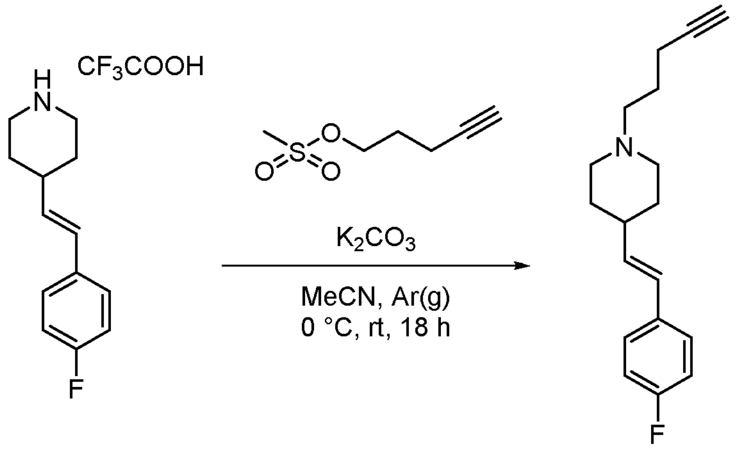


The titled compound synthesized from (E)-4-(4-fluorostyryl)piperidine 2,2,2-trifluoroacetate (0.150 g, 0.470 mmol, 1.0 equiv.; prepared as described under the general procedure E) via general procedure G using previously prepared pent-4-yn-1-yl methanesulfonate instead of propargyl bromide. The crude product was purified by flash column chromatography using $CH_2Cl_2$/MeOH = 20/1 (v/v) as eluent. Yield: 43% (55 mg); $R_f$ = 0.20 ($CH_2Cl_2$/MeOH = 20/1, v/v); orange transparent oil; $^1H$ NMR (400 MHz, $CDCl_3$): δ 1.47-1.58 (m, 2H), 1.70-1.77 (m, 4H), 1.95 (t, *J* = 2.7 Hz, 1H), 2.01 (td, *J* = 11.7, 2.4 Hz, 2H), 2.07-2.16 (m, 1H), 2.23 (td, *J* = 7.1, 2.7 Hz, 2H), 2.42-2.45 (m, 2H), 2.95 (dt, *J* = 11.7, 2.6 Hz, 2H), 6.07 (dd, *J* = 16.0, 7.0 Hz, 1H), 6.33 (d, *J* = 16.0 Hz, 1H), 6.94-7.00 (m, 2H), 7.27-7.32 (m, 2H); $^{13}C$ NMR (100 MHz, $CDCl_3$): δ 16.50, 25.83, 32.00, 39.30, 53.60, 57.73, 68.40, 84.17, 115.28 (d, $J_{C,F}$ = 21.3 Hz), 126.92, 127.36 (d, $J_{C,F}$ = 8.1 Hz), 133.80, 134.84 (d, $J_{C,F}$ = 2.1 Hz), 161.90 (d, $J_{C,F}$ = 245.7 Hz); HRMS (ESI+): *m*/*z* calcd for $C_{18}H_{23}FN$ $[M+H]^+$ 272.1815; found 272.1818; HPLC purity, 99.8%.

**Biological procedures**

**Inhibition assay using human recombinant MAO-B**

The effects of the compounds on hMAO-B (hMAO-B, Sigma Aldrich, M7441) were investigated using a previously described fluorimetric assay (Zhou M, Anal Biochem, 1997, 253, 169-174; Bautista-Aguilera OM, J Med Chem, 2014, 57, 10455-10463). The inhibitory activity of the compounds was evaluated by their effects on the production of hydrogen peroxide ($H_2O_2$) from p-tyramine, a nonspecific substrate for both hMAO isoforms. The production of the $H_2O_2$ was detected using Amplex Red reagent in the presence of horseradish peroxidase, where a highly sensitive fluorescent product, resorufin, is produced at stoichiometric amounts. Recombinant human microsomal hMAO enzymes expressed in baculovirus infected insect cells (BTI-TN-5B1-4), horse-radish peroxidase (type II, lyophilized powder), and *p*-tyramine hydrochloride were purchased from Sigma Aldrich. 10-Acetyl-3,7-dihydroxyphenoxazine (Amplex Red reagent) was synthesized as described in the literature (von der Eltz H, USP Patent No. 4900822, Feb. 13, 1990).

Briefly, 100 μL 50 mM sodium phosphate buffer (pH 7.4, 0.05% [v/v] Triton X-114) containing the compounds or the reference inhibitors and hMAO-A or hMAO-B required to oxidize (in the control group) approximately 15 pmol of *p*-tyramine/min (hMAO-A, 0.25 μg protein; hMAO-B, 1.2 μg protein) were incubated for 15 min at 37 °C in a flat-bottomed black 96-well microplate (μCLEAR® microplate; Greiner Bio One International GmbH, Germany), and placed in a dark microplate reader chamber. After the pre-incubation, the reaction was started by adding the final concentrations of 250 μM Amplex Red reagent, 4 U/mL horseradish peroxidase, and 1 mM *p*-tyramine (final volume, 200 μL). The production of resorufin was quantified on the basis of the fluorescence generated ($\lambda_{ex}$= 530 nm, $\lambda_{em}$ = 590 nm) at 37 °C over a period of 20 min, during which time the fluorescence increase linearly. For control experiments, DMSO was used instead of the appropriate dilutions of the compounds in DMSO. To determine the blank value (*b*), phosphate-buffered solution replaced the enzyme solution. The initial velocities were calculated from the trends obtained, with each measurement carried out in duplicate. The inhibitory potencies are expressed as the residual activities (RA = ($v_i$-*b*) / ($v_o$-*b*), where $v_i$ is the velocity in the presence of the test compounds, and $v_0$ the control velocity in the presence of DMSO. The $IC_{50}$ values were obtained by plotting residual enzyme activities against applied inhibitor concentration, with the experimental data fitted to a Hill four parameter equation (Equation (1)) using in-house python script and Gnuplot software.

$$Y = Min + (Max - Min) / (1+10\hat{}((LogIC_{50} - X) \times Hill\ coefficient)) \qquad (1),$$

where X represent logarithm of inhibitor concentration, and Y the residual activity.

The capacity of the test compounds to react directly with Amplex Red was determined by adding these compounds to a solution containing all of the components except the MAO isoenzyme. No significant interference was detected for the test compounds.

**Reversibility assay**

For the reversibility assay, hMAO-B at 100-fold final concentration was incubated with the inhibitors at a concentration 10-fold the $IC_{50}$ at 37 °C (volume, 50 μL). After 15 min, the mixture was diluted 100-fold into the reaction buffer containing Amplex Red reagent, horseradish peroxidase, and p-tyramine hydrochloride. The final concentrations of all of the reagents and MAO-B were the same as in the assay described above. The reaction was monitored for 30 min. Control experiments were carried out in the same manner, where the inhibitor solution was replaced by DMSO.

**Results of biological assays**

**Table 1**: Inhibitory potencies of the compounds with general Formula (I) and reference compounds

| Structure | hMAO-B $IC_{50}$ [nM] ±SEM | Structure | hMAO-B $IC_{50}$ [nM] ±SEM |
|---|---|---|---|
| 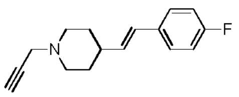 | 342.2 ±22.4 | 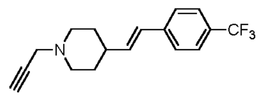 | 18.6 ±3.3 |
| 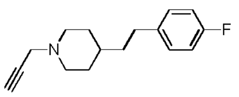 | 370.1 ±37.3 | 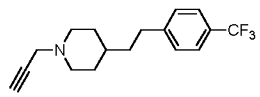 | 10.9 ±3.0 |
| 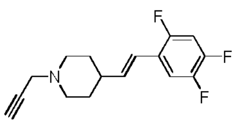 | 436.2 ±11.7 | 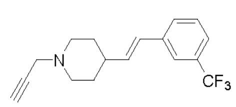 | 549.7 ±127.5 |
| 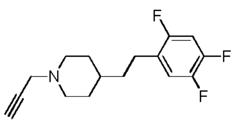 | 216.4 ±14.2 | 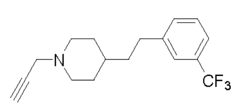 | 501.8 ±107.1 |
| 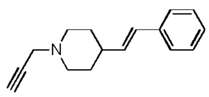 | 3540.2 ±611.8 | 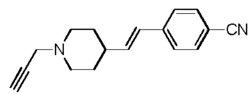 | 57.0 ±7.1 |
| 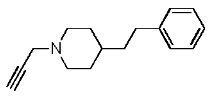 | 4603.4 ±371.1 | 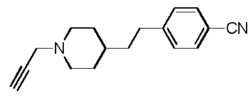 | 56.6 ±8.7 |
| 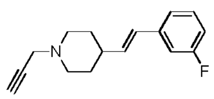 | 5048.7 ±1224.6 | 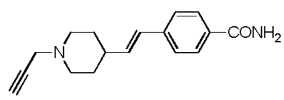 | 1737.0 ±691.0 |
| 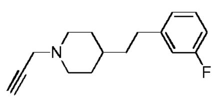 | 3363.5±310.5 | 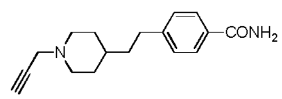 | 5554.5 ±1778.9 |
| 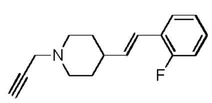 | 11830.4 ±766.5 | 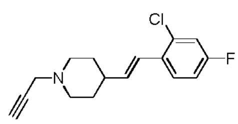 | 417.6 ±46.9 |

(continued)

| Structure | hMAO-B $IC_{50}$ [nM] ±SEM | Structure | hMAO-B $IC_{50}$ [nM] ±SEM |
|---|---|---|---|
| 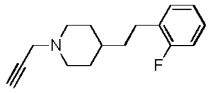 | 14159.5 ±708.9 | 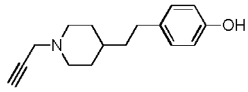 | 7440.7 ±1291.3 |
| 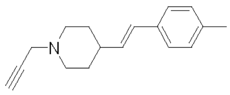 | 147.0 ±13.8 | 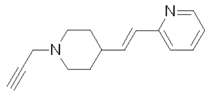 | 730.1 ±85.6 |
| 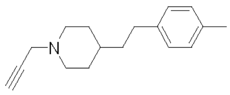 | 159.0 ±13.3 | 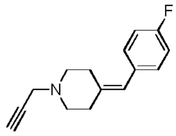 | 48836.5 ±3243.2 |
| 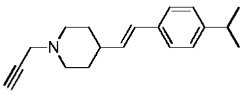 | 44.4 ±8.2 | 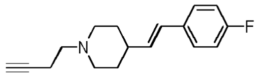 | 475.4 ±28.6 |
| 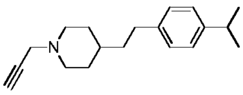 | 7.0 ±2.2 | 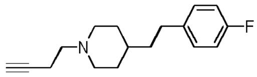 | 1698.9 ±291.5 |
| 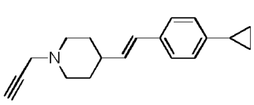 | 43.7 ±6.4 | 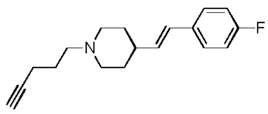 | 163.0 ±17.0 |
| 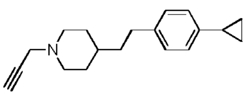 | 36.1 ±4.1 | 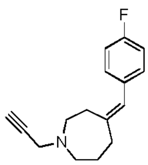 | 24581.0 ±2301.7 |
| 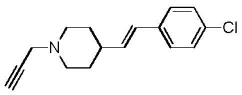 | 50.4 ±4.7 | 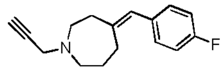 | 254.4 ±25.7 |
| 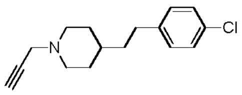 | 38.1 ±6.8 | 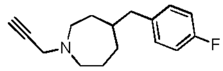 | 59.2 ±6.0 |
| 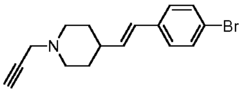 | 21.2 ±4.2 | 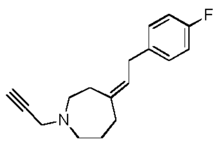 | 3185.7 ±385.1 |
| 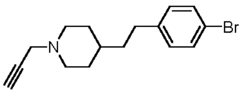 | 1439.0 ±140.1 | 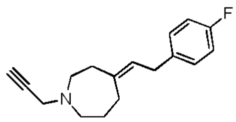 | 94.4 ±15.3 |
| 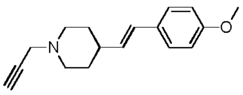 | 68.1 ±11.1 | 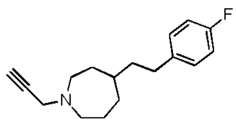 | 250.3 ±42.4 |

(continued)

| Structure | hMAO-B $IC_{50}$ [nM] ±SEM | Structure | hMAO-B $IC_{50}$ [nM] ±SEM |
|---|---|---|---|
| 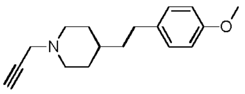 | **67.0 ±11.4** | 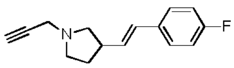 | **17402.0 ±2039.1** |
| hMAO-B: human monoamine oxidase B<br>RA: residual activity at 100 μM of the test compound expressed as percentage | | | |

**Table 2:** Reversibility assay of inhibitors with general Formula (I) and reference compounds

| Structure | Activity of the enzyme after 100-fold dilution (in percentage ±SD) |
|---|---|
| 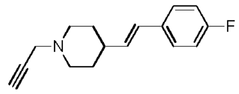 | **21.47 ±2.35** |
| 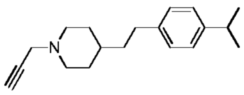 | **6.20 ±0.71** |
| 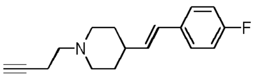 | **94.91 ±2.52** |
| 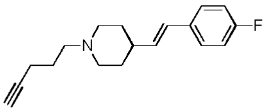 | **74.01 ±2.76** |
| 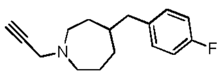 | **5.55 ±0.25** |

**Claims**

1. Compound of general Formula (I):

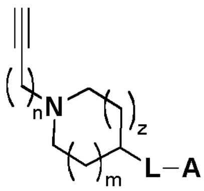

**(I)**

wherein:

**n:** 1-5
**m:** 0, 1, or 2
**z:** 0 or 1
**L:** $=CH_2$-, $=CH_2CH_2$- or

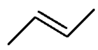

trans ;

**A:** is

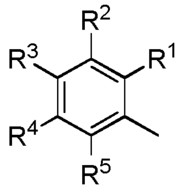

,

wherein

**$R^1$**: H, Me, Et, i-Pr, t-Bu, i-Bu, CN, F, Cl, Br, $CF_3$, $CH_2CF_2$, cyclopropyl, cyclobutyl, cyclopentyl, $SR^6$, $OR^6$ or $NR^6R^7$

**$R^6$:** H, Me, Et, n-Pr, i-Pr, t-Bu or i-Bu,
**$R^7$:** H, Me, Et, n-Pr, i-Pr, t-Bu or i-Bu,

**$R^2$:** H, Me, Et, i-Pr, t-Bu, i-Bu, CN, F, Cl, Br, $CF_3$, $CH_2CF_2$, cyclopropyl, cyclobutyl, cyclopentyl, $SR^6$, $OR^6$ or $NR^6R^7$

**$R^6$:** H, Me, Et, n-Pr, i-Pr, t-Bu, or i-Bu,
**$R^7$:** H, Me, Et, n-Pr, i-Pr, t-Bu, or i-Bu,

**$R^3$:** H, Me, Et, i-Pr, t-Bu, i-Bu, CN, F, Cl, Br, $CF_3$, $CH_2CF_2$, cyclopropyl, cyclobutyl, cyclopentyl, $SR^6$, $OR^6$ or $NR^6R^7$

**$R^6$:** H, Me, Et, n-Pr, i-Pr, t-Bu, or i-Bu,
**$R^7$:** H, Me, Et, n-Pr, i-Pr, t-Bu, or i-Bu,

**$R^4$:** H, Me, Et, i-Pr, t-Bu, i-Bu, CN, F, Cl, Br, $CF_3$, $CH_2CF_2$, cyclopropyl, cyclobutyl, cyclopentyl, $SR^6$, $OR^6$ or $NR^6R^7$

**$R^6$:** H, Me, Et, n-Pr, i-Pr, t-Bu or i-Bu,
**$R^7$:** H, Me, Et, n-Pr, i-Pr, t-Bu or i-Bu,

**$R^5$:** H, Me, Et, i-Pr, t-Bu, i-Bu, CN, F, Cl, Br, $CF_3$, $CH_2CF_2$, cyclopropyl, cyclobutyl, cyclopentyl, $SR^6$, $OR^6$ or $NR^6R^7$

**$R^6$:** H, Me, Et, n-Pr, i-Pr, t-Bu or i-Bu,
**$R^7$:** H, Me, Et, n-Pr, i-Pr, t-Bu or i-Bu,

or a pharmaceutically acceptable salt, hydrate or solvate thereof; in the form of a pure diastereoisomer or as a mixture of diastereomers, in the form of a pure enantiomer or as a mixture of enantiomers.

**2.** The compound according to claim 1, wherein $R^1$ is H, Me, i-Pr, CN, F, Cl, Br or $CF_3$.

**3.** The compound according to claim 1 or 2, wherein L is

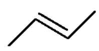

trans .

**4.** The compound according to any one of claims 1 to 3, wherein m is 1 or 2.

**5.** The compound according to any one of claims 1 to 4, wherein n is 1.

**6.** A compound according to claim 1 selected from the group consisting of:

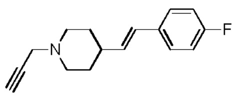, 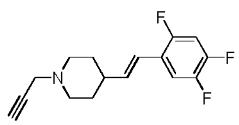, 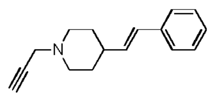, 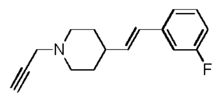,

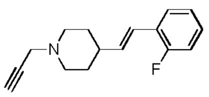, 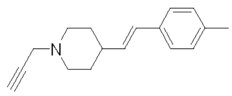, 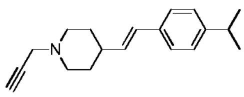, 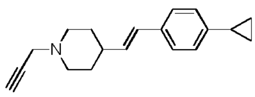,

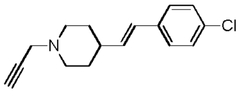, 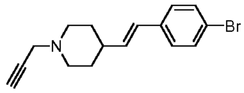, 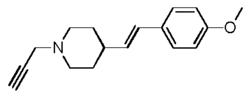, 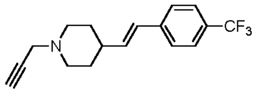,

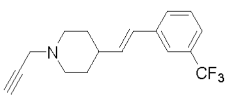, 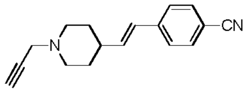, 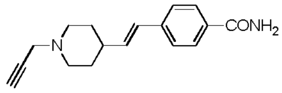, 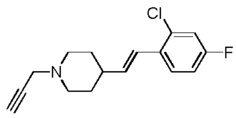,

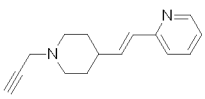, 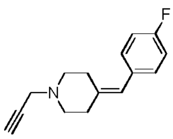, 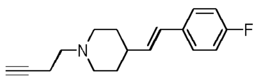, 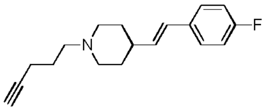,

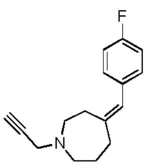, 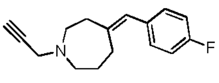, 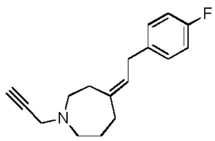, 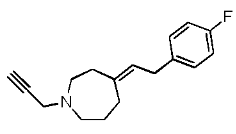

and

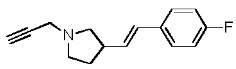.

**7.** The compound according to claim 1 or 6, wherein said compound is selected from the group consisting of:

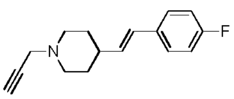, 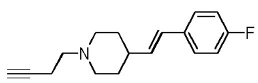, 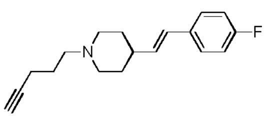, 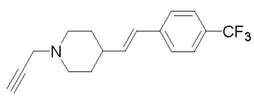,

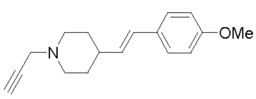, and 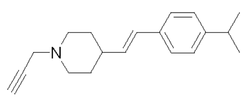.

**8.** The compound according to claim 1 or 6, wherein said compound is

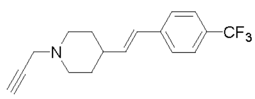.

**9.** The compound according to claim 1 or 6, wherein said compound is

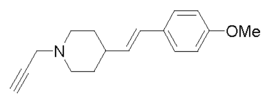
.

**10.** The compound according to claim 1 or 6, wherein said compound is

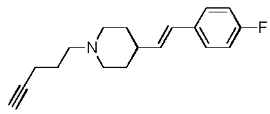
.

**11.** The compound according to any one of claims 1 to 10 for use as a medicament.

**12.** The compound according to any one of claims 1 to 10 for use in the prevention, alleviation of symptoms or treatment of a medical condition selected from the group consisting of acute and chronic neurological disorders, cognitive and neurodegenerative diseases.

**13.** The compound for use according to claim 12, which is for use in the prevention, alleviation of symptoms or treatment of a neurodegenerative disease, such as Parkinson's disease, Alzheimer's disease, Huntington's disease or dementia.

**14.** The compound for use according to claim 12, which is for use in the prevention, alleviation of symptoms or treatment of Parkinson's disease, Alzheimer's disease, Huntington's disease or dementia.

**15.** A pharmaceutical composition comprising a therapeutically effective amount of at least one compound according to any one of claims 1 to 10 and a pharmaceutical acceptable excipient.

## Patentansprüche

**1.** Verbindung der allgemeinen Formel (I):

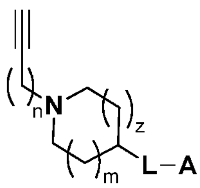

**(I)**

wobei:

**n:** 1-5
**m:** 0, 1 oder 2
**z:** 0 oder 1
**L:** $=CH_2-$, $=CH_2CH_2-$ oder

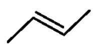

trans ;

**A:**

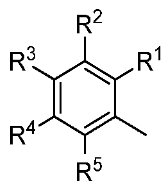 ist,

wobei

**$R^1$:** H, Me, Et, i-Pr, t-Bu, i-Bu, CN, F, Cl, Br, $CF_3$, $CH_2CF_2$, Cyclopropyl, Cyclobutyl, Cyclopentyl, $SR^6$, $OR^6$ oder $NR^6R^7$
**$R^6$:** H, Me, Et, n-Pr, i-Pr, t-Bu oder i-Bu,
**$R^7$:** H, Me, Et, n-Pr, i-Pr, t-Bu oder i-Bu,
**$R^2$:** H, Me, Et, i-Pr, t-Bu, i-Bu, CN, F, Cl, Br, $CF_3$, $CH_2CF_2$, Cyclopropyl, Cyclobutyl, Cyclopentyl, $SR^6$, $OR^6$ oder $NR^6R^7$
**$R^6$:** H, Me, Et, n-Pr, i-Pr, t-Bu, oder i-Bu,
**$R^7$:** H, Me, Et, n-Pr, i-Pr, t-Bu, oder i-Bu,
**$R^3$:** H, Me, Et, i-Pr, t-Bu, i-Bu, CN, F, Cl, Br, $CF_3$, $CH_2CF_2$, Cyclopropyl, Cyclobutyl, Cyclopentyl, $SR^6$, $OR^6$ oder $NR^6R^7$
**$R^6$:** H, Me, Et, n-Pr, i-Pr, t-Bu, oder i-Bu,
**$R^7$:** H, Me, Et, n-Pr, i-Pr, t-Bu, oder i-Bu,
**$R^4$:** H, Me, Et, i-Pr, t-Bu, i-Bu, CN, F, Cl, Br, $CF_3$, $CH_2CF_2$, Cyclopropyl, Cyclobutyl, Cyclopentyl, $SR^6$, $OR^6$ oder $NR^6R^7$
**$R^6$:** H, Me, Et, n-Pr, i-Pr, t-Bu oder i-Bu,
**$R^7$:** H, Me, Et, n-Pr, i-Pr, t-Bu oder i-Bu,
**$R^5$:** H, Me, Et, i-Pr, t-Bu, i-Bu, CN, F, Cl, Br, $CF_3$, $CH_2CF_2$, Cyclopropyl, Cyclobutyl, Cyclopentyl, $SR^6$, $OR^6$ oder $NR^6R^7$
**$R^6$:** H, Me, Et, n-Pr, i-Pr, t-Bu oder i-Bu,
**$R^7$:** H, Me, Et, n-Pr, i-Pr, t-Bu oder i-Bu,

oder ein pharmazeutisch verträgliches Salz, Hydrat oder Solvat davon; in Form eines reinen Diastereoisomers oder als eine Mischung von Diastereomeren, in Form eines reinen Enantiomers oder als eine Mischung von Enantiomeren.

2. Verbindung nach Anspruch 1, wobei $R^1$ H, Me, i-Pr, CN, F, Cl, Br oder $CF_3$ ist.

3. Verbindung nach Anspruch 1 oder 2, wobei L

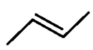

trans

ist.

4. Verbindung nach einem der Ansprüche 1 bis 3, wobei m 1 oder 2 ist.

5. Verbindung nach einem der Ansprüche 1 bis 4, wobei n 1 ist.

6. Verbindung nach Anspruch 1, ausgewählt aus der Gruppe bestehend aus:

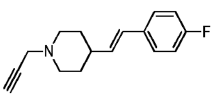 , 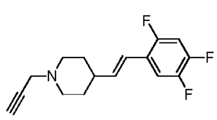 , 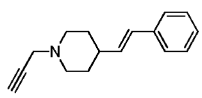 , 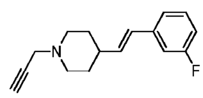 ,

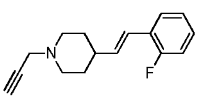 , 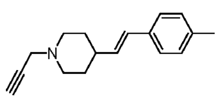 , 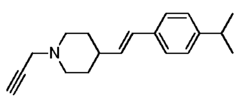 , 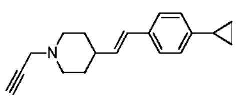 ,

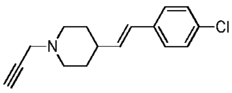 , 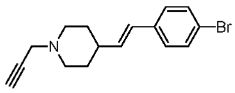 , 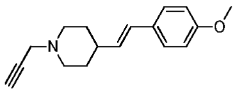 , 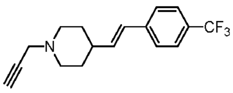 ,

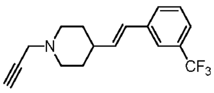 , 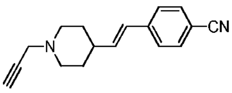 , 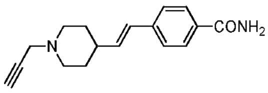 , 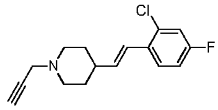 ,

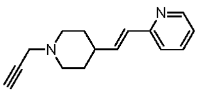 , 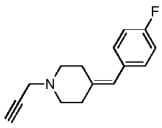 , 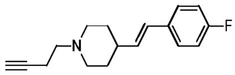 , 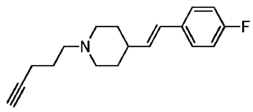 ,

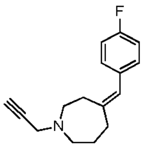 , 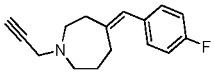 , 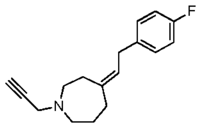 , 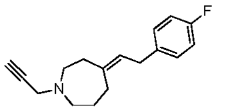

und

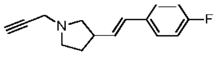 .

**7.** Verbindung nach Anspruch 1 oder 6, wobei die Verbindung ausgewählt ist aus der Gruppe bestehend aus:

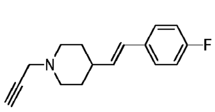 , 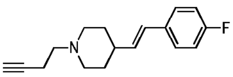 , 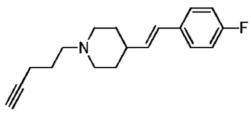 ,

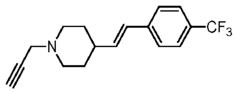 , 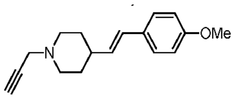 , und 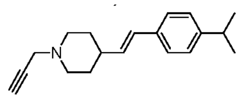 .

**8.** Verbindung nach Anspruch 1 oder 6, wobei die Verbindung

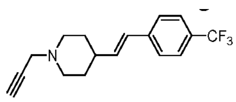

ist.

**9.** Verbindung nach Anspruch 1 oder 6, wobei die Verbindung

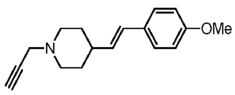

ist.

**10.** Verbindung nach Anspruch 1 oder 6, wobei die Verbindung

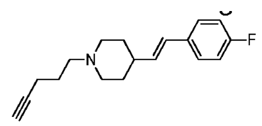

ist.

**11.** Verbindung nach einem der Ansprüche 1 bis 10 zur Verwendung als ein Arzneimittel.

**12.** Verbindung nach einem der Ansprüche 1 bis 10 zur Verwendung bei der Vorbeugung, Linderung von Symptomen oder Behandlung eines medizinischen Zustands ausgewählt aus der Gruppe bestehend aus akuten und chronischen neurologischen Störungen, kognitiven und neurodegenerativen Erkrankungen.

**13.** Verbindung zur Verwendung nach Anspruch 12, die zur Verwendung bei der Vorbeugung, Linderung von Symptomen oder Behandlung einer neurodegenerativen Erkrankung, wie Parkinson-Krankheit, Alzheimer-Krankheit, Huntington-Krankheit oder Demenz, dient.

**14.** Verbindung zur Verwendung nach Anspruch 12, die zur Verwendung bei der Vorbeugung, Linderung von Symptomen oder Behandlung von Parkinson-Krankheit, Alzheimer-Krankheit, Huntington-Krankheit oder Demenz dient.

**15.** Pharmazeutische Zusammensetzung, die eine therapeutisch wirksame Menge von mindestens einer Verbindung nach einem der Ansprüche 1 bis 10 und einen pharmazeutisch verträglichen Hilfsstoff umfasst.

## Revendications

**1.** Composé avec la formule suivante (I) :

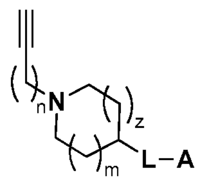

**(I)**

dans laquelle,

**n:** 1-5
**m:** 0, 1 ou 2
**z** : 0 ou 1
**L** : $=CH_2-$, $=CH_2CH_2-$ ou

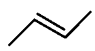

trans ;

**A:** est

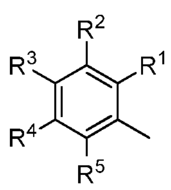

où

**$R^1$:** H, Me, Et, i-Pr, t-Bu, i-Bu, CN, F, Cl, Br, $CF_3$, $CH_2CF_2$, cyclopropyle, cyclobutyle, cyclopentyle, $SR^6$, $OR^6$ ou $NR^6R^7$
**$R^6$** : H, Me, Et, n-Pr, i-Pr, t-Bu ou i-Bu,
**$R^7$** : H, Me, Et, n-Pr, i-Pr, t-Bu ou i-Bu,
**$R^2$** : H, Me, Et, i-Pr, t-Bu, i-Bu, CN, F, Cl, Br, $CF_3$, $CH_2CF_2$, cyclopropyle, cyclobutyle, cyclopentyle, $SR^6$, $OR^6$ ou $NR^6R^7$
**$R^6$** : H, Me, Et, n-Pr, i-Pr, t-Bu ou i-Bu,
**$R^7$** : H, Me, Et, n-Pr, i-Pr, t-Bu ou i-Bu,
**$R^3$** : H, Me, Et, i-Pr, t-Bu, i-Bu, CN, F, Cl, Br, $CF_3$, $CH_2CF_2$, cyclopropyle, cyclobutyle, cyclopentyle, $SR^6$, $OR^6$ ou $NR^6R^7$
**$R^6$** : H, Me, Et, n-Pr, i-Pr, t-Bu ou i-Bu,
**$R^7$** : H, Me, Et, n-Pr, i-Pr, t-Bu ou i-Bu,
**$R^4$** : H, Me, Et, i-Pr, t-Bu, i-Bu, CN, F, Cl, Br, $CF_3$, $CH_2CF_2$, cyclopropyle, cyclobutyle, cyclopentyle, $SR^6$, $OR^6$ ou $NR^6R^7$
**$R^6$** : H, Me, Et, n-Pr, i-Pr, t-Bu ou i-Bu,
**$R^7$** : H, Me, Et, n-Pr, i-Pr, t-Bu ou i-Bu,
**$R^5$** : H, Me, Et, i-Pr, t-Bu, i-Bu, CN, F, Cl, Br, $CF_3$, $CH_2CF_2$, cyclopropyle, cyclobutyle, cyclopentyle, $SR^6$, $OR^6$ ou $NR^6R^7$
**$R^6$** : H, Me, Et, n-Pr, i-Pr, t-Bu ou i-Bu,
**$R^7$** : H, Me, Et, n-Pr, i-Pr, t-Bu ou i-Bu,

ou un sel, hydrate ou solvate pharmaceutiquement acceptable de celui-ci ; sous forme d'un diastéréoisomère pur ou en tant que mélange de diastéréoisomères, ou sous forme d'un énantiomère pur ou en tant que mélange d'énantiomères.

**2.** Composé selon la revendication 1, dans lequel $R^1$ est H, Me, i-Pr, CN, F, Cl, Br ou $CF_3$.

**3.** Composé selon la revendication 1 ou 2, dans lequel L est

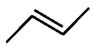

trans .

**4.** Composé selon l'une quelconque des revendications 1 à 3, dans lequel m est 1 ou 2.

**5.** Composé selon l'une quelconque des revendications 1 à 4, dans lequel n est 1.

**6.** Composé selon la revendication 1 choisis parmi le groupe constitué par:

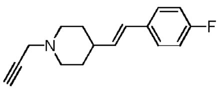 , 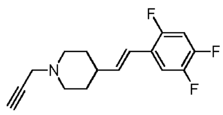 , 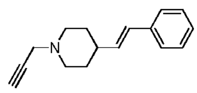 , 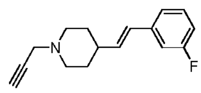 ,

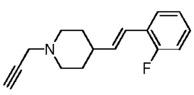 , 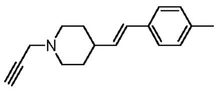 , 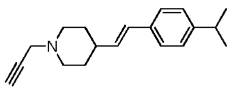 , 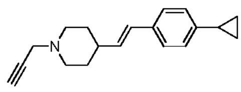 ,

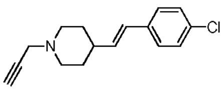 , 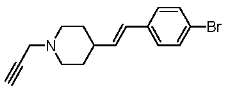 , 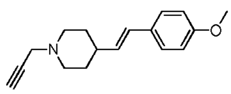 , 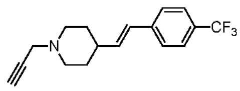 ,

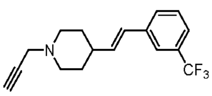 , 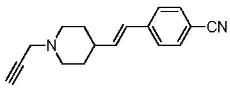 , 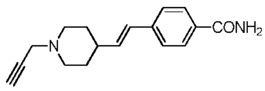 , 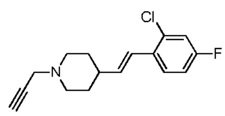 ,

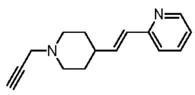 , 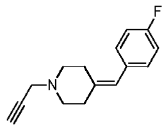 , 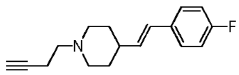 , 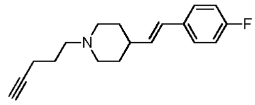 ,

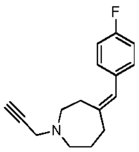 , 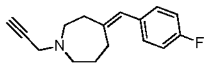 , 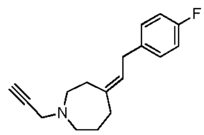 , 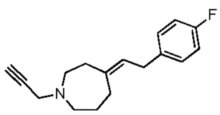

et

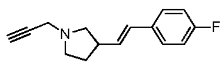 .

7. Composé selon la revendication 1 ou 6, dans lequel ledit composé est choisi parmi le groupe constitué par:

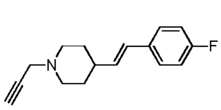 , 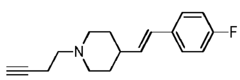 , 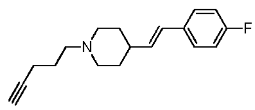 ,

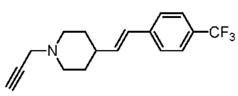 , 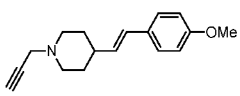 et 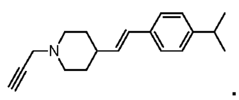 .

8. Composé selon la revendication 1 ou 6, dans lequel ledit composé est

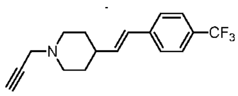 .

9. Composé selon la revendication 1 ou 6, dans lequel ledit composé est.

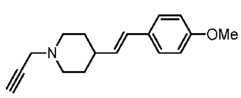 .

10. Composé selon la revendication 1 ou 6 dans lequel ledit composé est

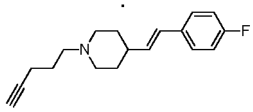 .

11. Composé selon l'une quelconque des revendications 1 à 10 pour l'utilisation en tant qu'un médicament.

12. Composé selon l'une quelconque des revendications 1 à 10, destiné à être utilisé dans la prévention, le soulagement des symptômes ou le traitement d'une affection médicale choisie dans le groupe constitué des troubles neurologiques aigus et chroniques, des maladies cognitives et neurodégénératives.

13. Composé pour l'usage selon la revendication 12, destiné à être utilisé dans la prévention, le soulagement des

symptômes ou le traitement d'une maladie neurodégénérative, telle que la maladie de Parkinson, la maladie d'Alzheimer, la maladie de Huntington ou la démence.

**14.** Composé pour l'usage selon la revendication 12, destiné à être utilisé dans la prévention, le soulagement des symptômes ou le traitement de la maladie de Parkinson, de la maladie d'Alzheimer, de la maladie de Huntington ou de la démence.

**15.** Composition pharmaceutique comprenant une quantité thérapeutiquement efficace d'au moins un composé selon l'une quelconque des revendications 1 à 10 et un excipient pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030105133 A
- WO 9943657 A, Caroon JM
- US 4900822 A


### Non-patent literature cited in the description


- **CAI Z.** *Mol Med Rep,* 2014, vol. 9, 1533-1541
- **CARRADORI S.** *J Med Chem,* 2015, vol. 58, 6717-6732
- **RIEDERER P.** *NeuroToxicology,* 2004, vol. 25, 271-277
- **SHIH JC.** *Annu Rev Neurosci,* 1999, vol. 22, 197-217
- **SINGH R.** *Chem Biol Lett,* 2014, vol. 1, 33-39
- **KUMAR B.** *RSC Adv,* 2016, vol. 6, 42660-42683
- **ASTHON A.** *Pain: New Insights for the Healthcare Professional,* 2013
- **RIEDERER Z.** *Exp Neurobiol,* 2011, vol. 20, 1-17
- **CAI Z.** *Mol Med Rep.,* 2014, vol. 9, 1533-1541
- **OOI J.** *Mol Neurobiol,* 2015, vol. 52, 1850-1861
- **KUMAR B et al.** *RSC Adv.,* 2016, vol. 6, 42660-42683
- **PISANI et al.** *Curr Med Chem.,* 2011, vol. 18 (30), 4568-87
- **HUANG YS.** *Ind End Chem Res,* 2010, vol. 49, 12164-12167
- **ZHOU M.** *Anal Biochem,* 1997, vol. 253, 169-174
- **BAUTISTA-AGUILERA OM.** *J Med Chem,* 2014, vol. 57, 10455-10463